# EUROPEAN PATENT SPECIFICATION

(11) **EP 3 072 943 B1**
(45) Date of publication and mention of the grant of the patent: **02.05.2018**
(21) Application number: 15161070.6
(22) Date of filing: 26.03.2015
(51) Int. Cl.: C09K 11/06, H01L 51/00, H01L 51/50, C07D 333/00, C07D 307/00, C07D 405/14, C07D 405/04, C07D 405/10, C07D 409/04, C07D 409/10, C07D 409/14

(54) **DIBENZOFURAN/CARBAZOLE-SUBSTITUTED BENZONITRILES**
DIBENZOFURAN/CARBAZOL-SUBSTITUIERTE BENZONITRILE
BENZONITRILES DE DIBENZOFURANE/CARBAZOLE-SUBSTITUÉ

(43) Date of publication of application: 28.09.2016
(73) Proprietor: Idemitsu Kosan Co., Ltd., Chiyoda-ku Tokyo 100-8321 (JP)
(72) Inventor: Wolleb, Heinz, 4232 Fehren (CH); Nagashima, Hideaki, 4057 Basel (CH)
(74) Representative: Hollah, Dorothee

(56) References cited:
- WO-A1-2013/145923
- WO-A1-2015/022974
- JP-A- 2013 149 880
- US-A1- 2013 234 119
- US-A1- 2013 313 536
- US-A1- 2014 001 446

## Description

The present invention relates to compounds of formula **I**, a process for their production and their use in electronic devices, especially electroluminescent devices. When used as charge transport material and/or host material for phosphorescent emitters in electroluminescent devices, the compounds of formula **I** may provide improved efficiency and reduced driving voltage of electroluminescent devices.

US20070202358 relates to an organic electroluminescent device, which comprises a compound of formula 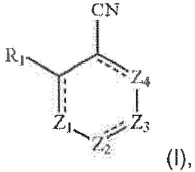 wherein wherein Z₁, Z₂, Z₃ and Z₄ each independently represents an atom selected from the group consisting of carbon, nitrogen, sulfur and oxygen and necessary for forming an unsaturated 6-membered ring skeleton, the atom may have a hydrogen atom or a substituent; a bond in the unsaturated 6-membered ring indicates a single bond or a double bond; and R₁ represents a substituent. The following compounds are explicitly disclosed: 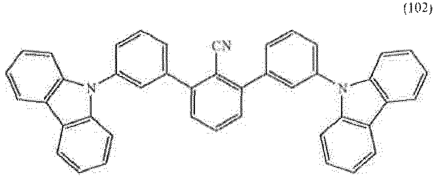 and 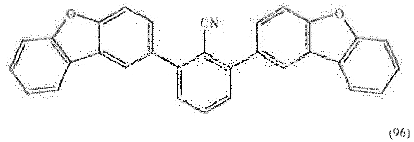 US20090026938 relates to an organic electroluminescence element comprising an organic layer comprising a light-emitting layerbetween a pair of electrodes, wherein the light-emitting layer comprises at least two light-emitting materials having different Ea (electron affinity) values and at least one host material, a concentration of a light-emitting material having a larger Ea value among the at least two light-emitting materials in the lightemitting layer gradually decreases from a cathode side toward an anode side in the light-emitting layer, and a concentration of a light-emitting material having a smaller Ea value among the at least two light-emitting materials in the light-emitting layer gradually decreases from the anode side toward the cathode side.

US20090072727 relates to an organic electroluminescent device comprising:
a pair of electrodes; and an organic layer between the pair of electrodes, which comprises a light-emitting layer and contains a compound represented by the following formula 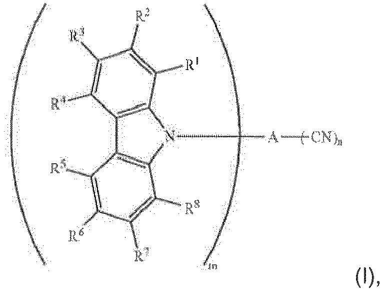 wherein each of R¹ to R⁸ independently represents a hydrogen atom or a substituent, A represents an aromatic ring which may have a substituent, m represents an integer of 2 or greater, and n represents an integer of 1 or greater.
The following compound is explicitly disclosed: 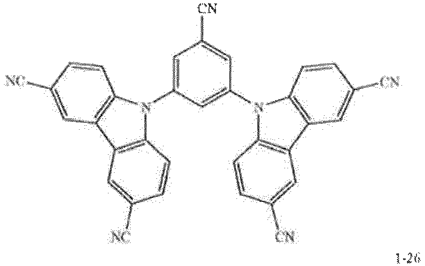 (see also WO2010140482).

Journal of Chemical Sciences (Bangalore, India) 122 (2010) 119-124 discloses a series of 2,8-bis(aryl)dibenzothiophene derivatives, such as, for example, 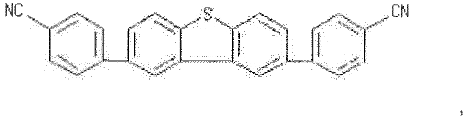 which were obtained by a coupling reaction of 2,8-dibromodibenzothiophene with arylboronic acid. These molecules have electron-withdrawing or electron-donating groups at the para-Ph position, which alters the electronic properties of these derivatives.

US2010244676 relates to organic electroluminescent device comprising:
a pair of electrodes; and at least one organic layer between the pair of electrodes, the at least one organic layer including a light emitting layer, the device comprising, in the at least one organic layer, a compound represented by formula 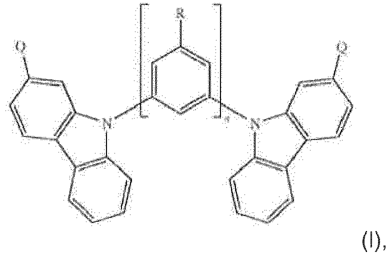 wherein Qs each independently represents a t-butyl group or a trimethylsilyl group; when the compound has a plurality of Rs, the Rs each independently represents a hydrogen atom an alkyl group, a cyano group, an aryl group, or a heteroaryl group; and n stands for 1 or 2, such as, for example,

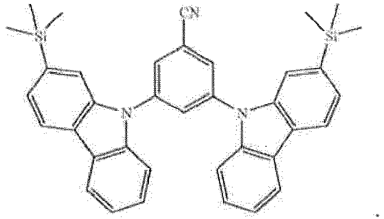

EP2461390 relates to an organic electroluminescence device, comprising on a substrate: a pair of electrodes; and at least one organic layer including a light emitting layer disposed between the electrodes on a substrate, wherein a compound represented by the following Formula (1) is contained in any one layer of organic layers, and wherein a phosphorescent emitting material represented by the following Formula (1) is contained in the light emitting layer: (Cz)p-L-(A)q (1), wherein in Formula (1), Cz is a substituted or unsubstituted aryl-carbazolyl group or a carbazolylaryl group, L is a single bond or a substituted or unsubstituted arylene group, a substituted or unsubstituted cycloalkylene group, or a substituted or unsubstituted aromatic heterocyclic ring, A is a substituted or unsubstituted nitrogen-containing six-membered aromatic heterocyclic ring, and each of p and q is independently an integer of 1 to 6.

WO2013154054 relates to an electroluminescent substance for an organic light-emitting device, comprising a cyanobenzene represented by formula 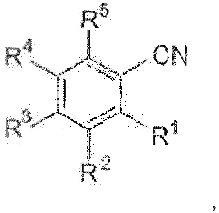 wherein at least one of R¹-R⁵ is a cyano group; at least one of R¹-R⁵ is 9-carbazolyl, 1,2,3,4-tetrahydro-9-carbazolyl, 1-indolyl, or a diarylamino group; and the residual groups R¹-R⁵ are H, or a residue.

US2013062597 relates to nitrogen-containing heteroaromatic compounds represented by a formula 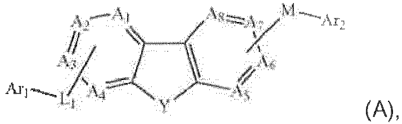 wherein wherein A₁ to A₈ are independently CR¹, Ar₁ is a hydrogen atom, a substituted or unsubstituted aromatic hydrocarbon group having 6 to 18 ring carbon atoms, or a substituted or unsubstituted heteroaromatic group having 5 to 18 ring atoms, L₁ is a single bond, a substituted or unsubstituted aromatic hydrocarbon group having 6 to 18 ring carbon atoms, or a substituted or unsubstituted heteroaromatic group having 5 to 18 ring atoms,
M is a substituted or unsubstituted nitrogen-containing heteroaromatic group, and
Ar₂ is a substituted aromatic hydrocarbon group having 6 to 18 ring carbon atoms, a substituted or unsubstituted monocyclic heteroaromatic group having 5 or 6 ring atoms, a dibenzofuran ring group that may be substituted with a substituent (excluding a 3-carbazolyl group and an N-carbazolyl group), a dibenzothiophene ring group that may be substituted with a substituent (excluding a 3-carbazolyl group and an N-carbazolyl group), or a nitrogen-containing polycyclic group among nitrogen-containing polycyclic groups respectively represented by formulas (1) to (5). The folowing compounds are explicitly disclosed:

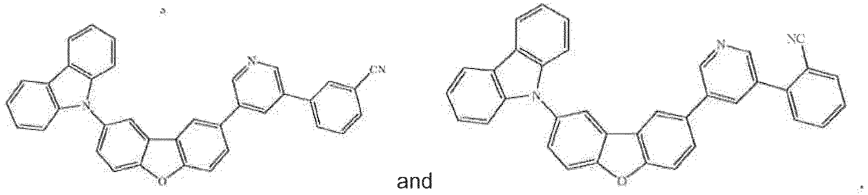

WO2014054912 relates to to organic electroluminescent compdounds of formula 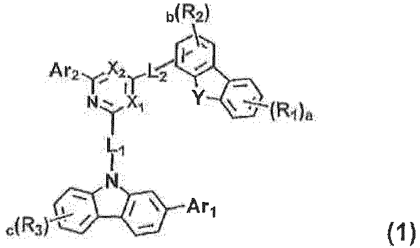 and an organic electroluminescent device comprising the same. The folowing compound is explicitly disclosed:

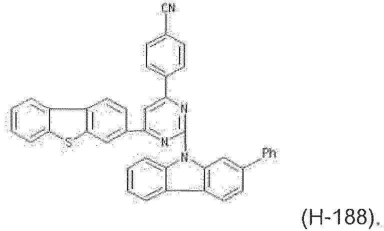

WO2014091958 relates to EL materials of formula 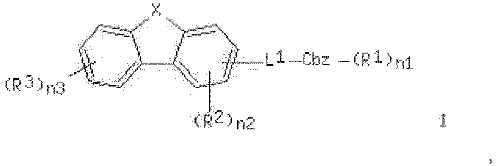 such as, for example,

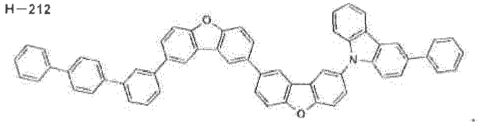

WO 2013/145923 A1 discloses an organic electroluminescent element which is characterized by: using, as a first host, a biscarbazole derivative that has a specific structure having a cyano group; and using, as a second host, a compound that has both a carbazole derivative structure and a nitrogen-containing heteroaromatic ring. The following structure is illustrated in WO 2013/145923 A1: 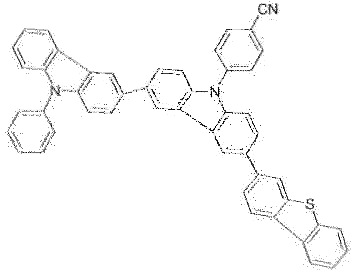 This structure is used for a host material.

US 2013/234119 A1 discloses an organic electroluminescence device employing a specific biscarbazole derivative having a cyano group as a first host and a compound having both a carbazole structure and a nitrogen-containing aromatic heteroring as a second host. The following compound is disclosed in US 2013/234119 A1:

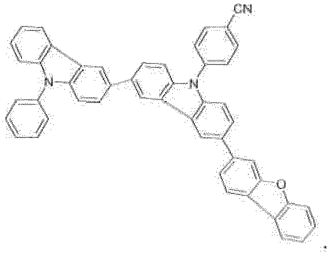

US 2013/313536 A1 discloses an organic electroluminescence device that includes: an anode, a cathode opposed to the anode and an emitting layer provided between the anode and the cathode. The following compound is disclosed in US 2013/313536 A1:

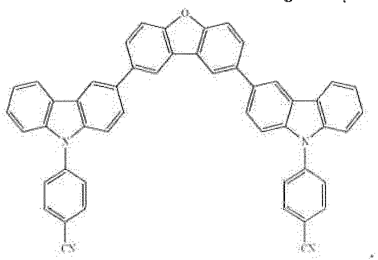

Notwithstanding these developments, there remains a need for organic light emitting devices comprising new electron transport materials to provide improved efficiency, stability, manufacturability, and/or spectral characteristics of electroluminescent devices.

Accordingly, it is an object of the present invention, with respect to the aforementioned prior art, to provide further materials suitable for use in OLEDs and further applications in organic electronics. More particularly, it should be possible to provide electron transport materials, hole/exciton blocker materials and matrix materials for use in OLEDs. The materials should be suitable especially for OLEDs which comprise at least one phosphorescence emitter, especially at least one green emitter or at least one blue emitter. Furthermore, the materials should be suitable for providing OLEDs which ensure good efficiencies, good operative lifetimes and a high stability to thermal stress, and a low use and operating voltage of the OLEDs.

Certain nitrile substituted benzonitriles bearing at the same time substituted or unsubstituted carbazole moieties and substituted or unsubstituted dibenzofurane, or dibenzothiophene moieties are found to be suitable for use in organo-electroluminescent devices. In particular, said derivatives are suitable electron transporting materials, hole blocking materials or host materials for phosphorescent emitters with good efficiency and durability.

Accordingly the present invention relates to compounds of formula 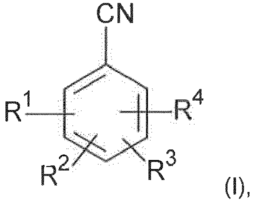 wherein
R₁ is a group of formula 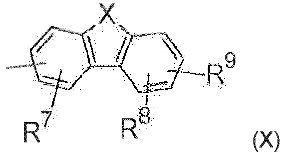 or 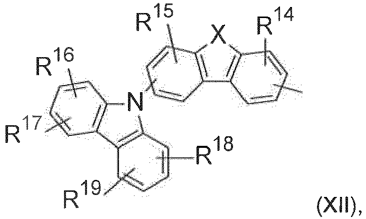
R², R³ and R⁴ are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², -SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G; a group of formula (**X**), (**XI**), (**XII**), or (**XIII**);
X is O, or S,
R⁷ to R⁹, R¹⁴ to R¹⁹ are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³²,-SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G;
R²⁴, R²⁵, R²⁶, R²⁷ and R²⁸ are independently of each other a C₁-C₂₅alkyl group, a C₆-C₂₄aryl group, which can optionally be substituted by G, or a C₂-C₃₀heteroaryl group, which can optionally be substituted by G;
R³² and R³³ are independently of each other a C₆-C₁₈aryl group; a C₆-C₁₈aryl group, which is substituted by C₁-C₁₈alkyl group, or C₁-C₁₈alkoxy group; a C₁-C₁₈alkyl group; or a C₁-C₁₈alkyl group, which is interrupted by -O-;
R³⁴ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R³⁵ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
E is -OR⁶⁹, -SR⁶⁹, -NR⁶⁵R⁶⁶, -COR⁶⁸, -COOR⁶⁷, -CONR⁶⁵R⁶⁶, -CN, or NO₂;
G is E, or a C₁-C₁₈alkyl group, a C₃-C₁₈cycloalkyl group, a C₆-C₂₄aryl group, a C₆-C₂₄aryl group, which is substituted by -CN, C₁-C₁₈alkyl, or C₁-C₁₈alkyl which is interrupted by O; a C₂-C₅₀heteroaryl group, or a C₂-C₃₀heteroaryl group, which is substituted by -CN, C₁-C₁₈alkyl, C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring;
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-;
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-;
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
with the proviso, that R² is a group of formula (**XI**), (**XII**), or (**XIII**), if R¹ is a group of formula (**X**), 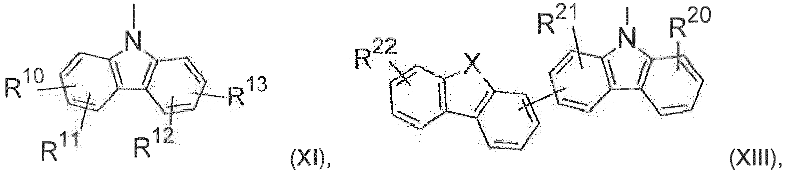 R¹⁰ to R¹³ and R²⁰ to R²³ are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³²,-SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G..

Certain compounds of the present invention show, when used as host and/or hole blocker in combination with phosphorescent emitters, excellent power efficiencies, in particular electroluminescent (EL) devices comprising the compounds of the present invention exhibit reduced driving voltage and excellent operative lifetime while maintaining excellent luminance properties. Furthermore, the colour and external quantum efficiency (EQE) can be improved and the roll-off may be reduced by use of the inventive compounds.

The compounds of the present invention may be used for electrophotographic photoreceptors, photoelectric converters, organic solar cells (organic photovoltaics), switching elements, such as organic transistors, for example, organic FETs and organic TFTs, organic light emitting field effect transistors (OLEFETs), image sensors, dye lasers and electroluminescent devices, such as, for example, organic light-emitting diodes (OLEDs).

Accordingly, a further subject of the present invention is directed to an electronic device, comprising a compound according to the present invention. The electronic device is preferably an electroluminescent device.

The compounds of formula I can in principal be used in any layer of an EL device, but are preferably used as host, electron transport and/or hole blocking material.

Hence, a further subject of the present invention is directed to an electron transport layer and/or a hole blocking layer comprising a compound of formula I according to the present invention.

A further subject of the present invention is directed to an emitting layer, comprising a compound of formula **I** according to the present invention. In said embodiment a compound of formula **I** is preferably used as host material in combination with a phosphorescent emitter.

D is preferably -CO-, -COO-, -S-, -SO-, -SO₂-, -O-, -NR⁶⁵-, wherein R⁶⁵ is C₁-C₁₈alkyl, such as methyl, ethyl, n-propyl, iso-propyl, n-butyl, isobutyl, tert-butyl, or sec-butyl, or C₆-C₁₄aryl, such as phenyl, tolyl, naphthyl, or biphenylyl.

E is preferably-OR⁶⁹; -SR⁶⁹; -NR⁶⁵R⁶⁵; -COR⁶⁸; -COOR⁶⁷; -CONR⁶⁵R⁶⁵; or -CN; wherein R⁶⁵, R⁶⁷, R⁶⁸ and R⁶⁹ are independently of each other C₁-C₁₈alkyl, such as methyl, ethyl, n-propyl, iso-propyl, n-butyl, isobutyl, tert-butyl, sec-butyl, hexyl, octyl, or 2-ethyl-hexyl, or C₆-C₁₄aryl, such as phenyl, tolyl, naphthyl, or biphenylyl.

The C₁-C₂₅alkyl groups, R² to R⁴ and R⁷ to R²², are typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl.

A C₁-C₂₅alkyl group substituted by one or more E and/or interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H; C₁-C₈alkylene-COO-R^{z}, e.g. CH₂COOR_{z,} CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CH=CH₂; CH₂CH(OH)CH₂-O-CO-C(CH₃)=CH₂.
An alkyl group substituted by E is, for example, an alkyl group where at least one of the hydrogen atoms is replaced by F. Examples are -CF₃, -CF₂CF₃, -CF₂CF₂CF₃, -CF(CF₃)₂,-(CF₂)₃CF₃, and -C(CF₃)₃.

The C₂-C₂₅alkenyl group, R² to R⁴ and R⁷ to R²², is a straight-chain, or branched alkenyl group, such as, for example, vinyl, allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, isododecenyl, n-dodec-2-enyl or n-octadec-4-enyl.

The C₂-C₂₅alkynyl group, R² to R⁴ and R⁷ to R²², is straight-chain or branched and preferably a C₂-C₈alkynyl group, such as, for example, ethynyl, 1-propyn-3-yl, 1-butyn-4-yl, 1-pentyn-5-yl, 2-methyl-3-butyn-2-yl, 1,4-pentadiyn-3-yl, 1,3-pentadiyn-5-yl, 1-hexyn-6-yl, cis-3-methyl-2-penten-4-yn-1-yl, trans-3-methyl-2-penten-4-yn-1-yl, 1,3-hexadiyn-5-yl, 1-octyn-8-yl, 1-nonyn-9-yl, 1-decyn-10-yl, or 1-tetracosyn-24-yl.

Examples of the C₃-C₁₈cycloalkyl group, R² to R⁴ and R⁷ to R²², are cyclopentyl, cyclohexyl, cycloheptyl, cyclooctyl, cyclononyl, cyclodecyl, cycloundecyl, cyclododecyl, preferably cyclopentyl, cyclohexyl, cycloheptyl and cyclooctyl, which may be unsubstituted or substituted by G.

The C₆-C₂₄aryl groups, R² to R⁴ and R⁷ to R²², which optionally can be substituted by G, are typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted by G.

The C₂-C₃₀heteroaryl group groups, R² to R⁴ and R⁷ to R²², which optionally can be substituted by G, represent a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, carbazolyl, or phenoxazinyl, which can be unsubstituted or substituted by G.

The wording "substituted by G" means that one, or more, especially one to three substituents G might be present. G has the same preferences as E, or is C₁-C₁₈alkyl, especially C₁-C₈alkyl, such as methyl, ethyl, n-propyl, iso-propyl, n-butyl, isobutyl, tert-butyl, sec-butyl, hexyl, octyl, or 2-ethyl-hexyl.

Prefered C₂-C₃₀heteroaryl groups are pyridyl, pyrimidyl, triazinyl, benzimidazo[1,2-a]benzimidazo-5-yl ( 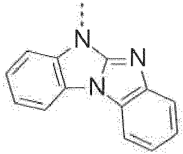 ), carbazolyl, dibenzofuranyl, which can be unsubstituted or substituted especially by C₆-C₁₀aryl, or C₆-C₁₀aryl, which is substituted by C₁-C₄alkyl; or C₂-C₁₄heteroaryl, especially

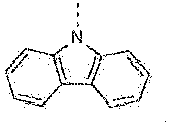

Examples of a group of formula Si(R²⁴)(R²⁵)(R²⁶) are a trimethylsilyl group, a triethylsilyl group, a tert-butyldimethylsilyl group, a propyldimethylsilyl group, a triisopropylsilyl group, a triphenylsilyl group, a phenyldimethylsilyl group, a t-butyldiphenylsilyl group, a tritolylsilyl group, a trixylylsilyl group, or a trinaphthylsilyl group.

Examples of the group P(O)(R²⁷)(R²⁸) include diphenylphosphoryl group and a dimethylphosphoryl group.

Examples of the group N(R³⁰)(R³¹) include diphenylamino and a phenylnaphthylamino group.

Preferred embodiments of the present invention are directed to compounds of formula 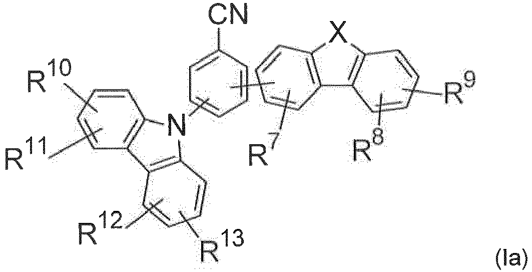 or 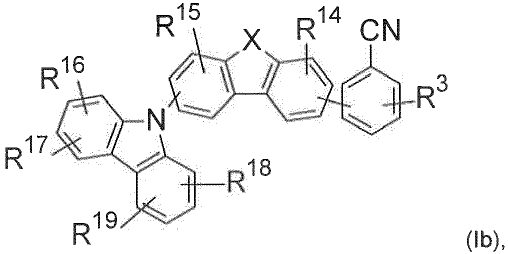 wherein X, R³ and R⁷ to R¹⁹ are defined above, or below.

In said embodiment compounds of formula 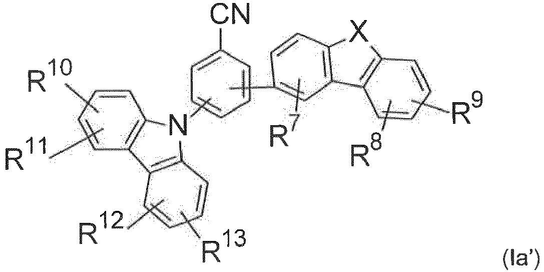 or 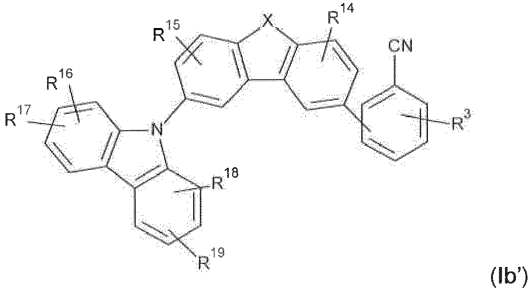 are more preferred, wherein X, R³ and R⁷ to R¹⁹ are defined above, or below.

Among compounds of formula (**Ia**) and (**Ia'**) compounds of formula 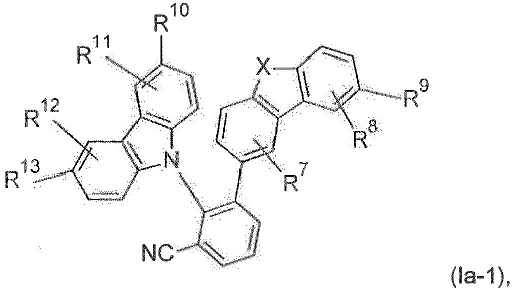 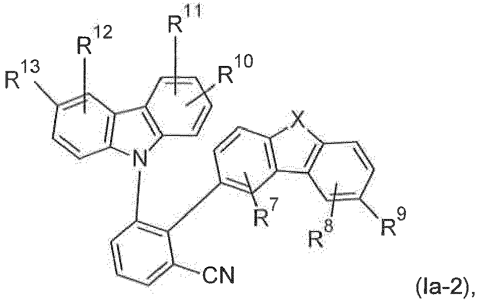 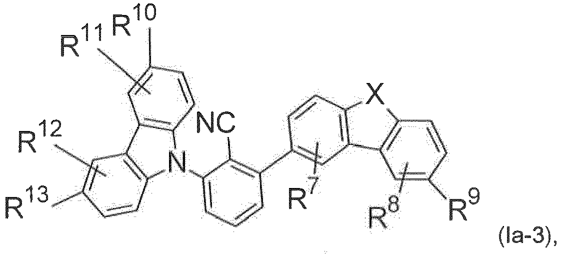 or 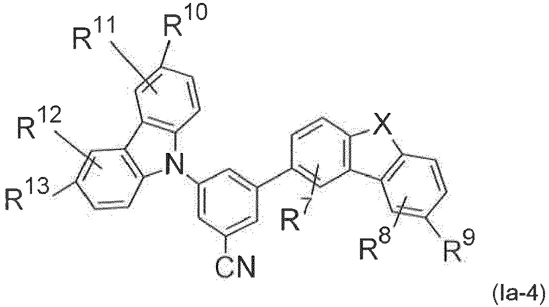 are more preferred, wherein X, R⁷ to R¹³ are as defined are defined above, or below.

Preferably, R⁷, R⁸, R¹¹ and R¹² are independently of each other H, or a C₁-C₂₅alkyl group. Preferably, R⁹, R¹⁰ and R¹³ are independently of each other H, CN, a group of formula 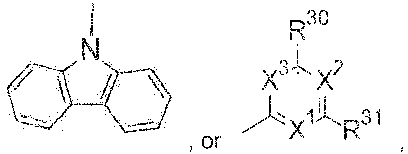 wherein
X¹, X² and X³ are independently of each other N, or CH,
R³⁰ and R³¹ are independently of each other H, or a phenyl group, with the proviso that at least one of X¹, X² and X³ is N.

Compounds of formula 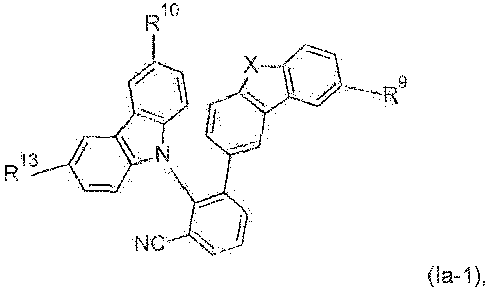 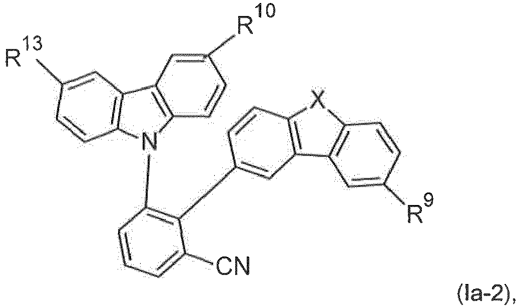 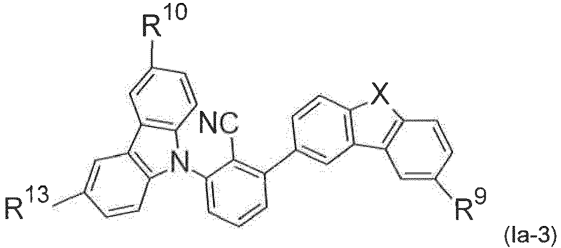 and 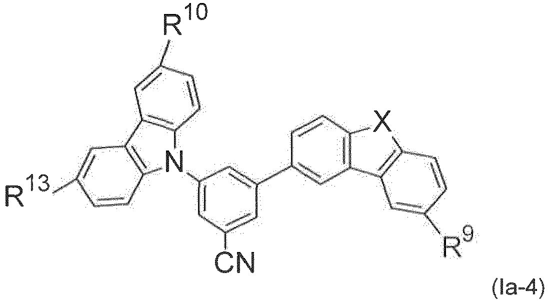 are most preferred, wherein
X is O, or S,
R⁹, R¹⁰ and R¹³ are independently of each other H, CN, a group of formula 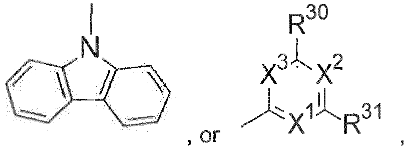
   X¹, X² and X³ are independently of each other N, or CH,
   R³⁰ and R³¹ are independently of each other H, or a phenyl group, with the proviso that at least one of X¹, X² and X³ is N.

Examples of particularly preferred compounds of formula (**Ia**) are shown below: 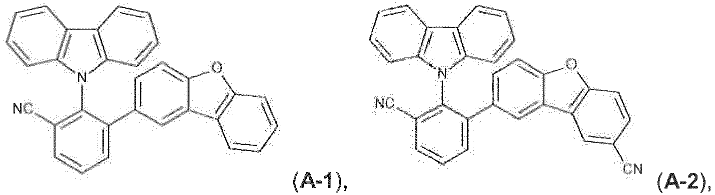 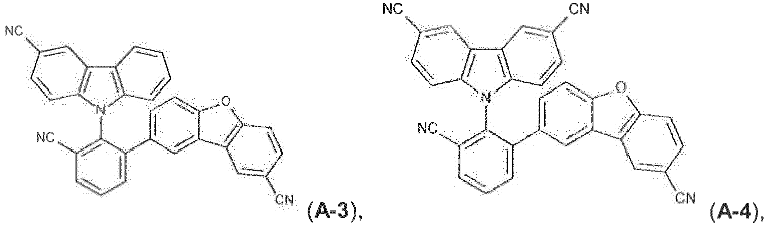 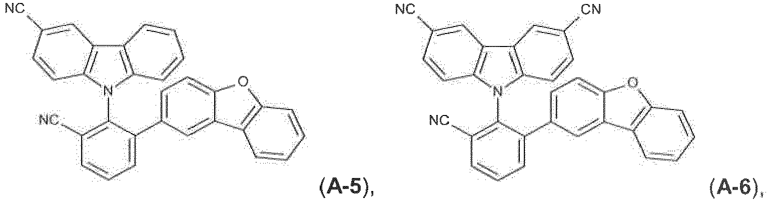 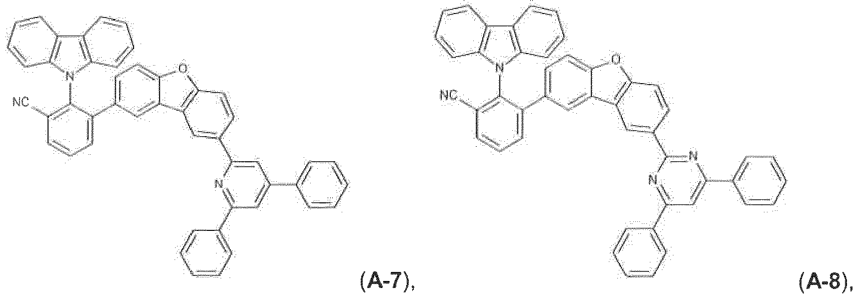 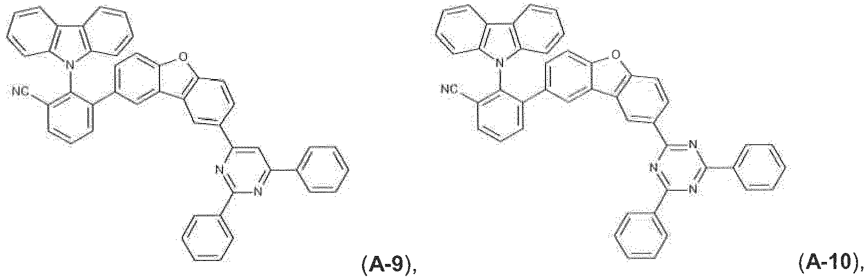 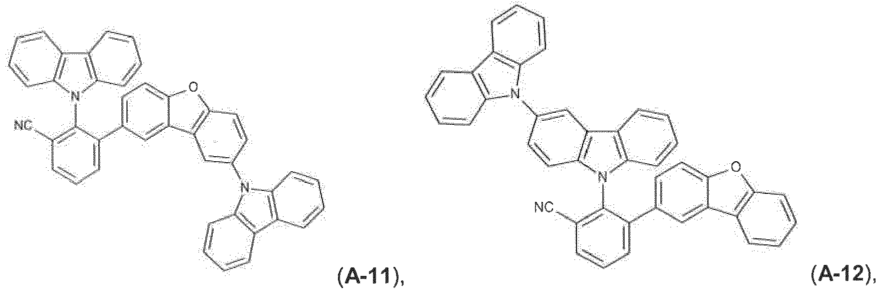 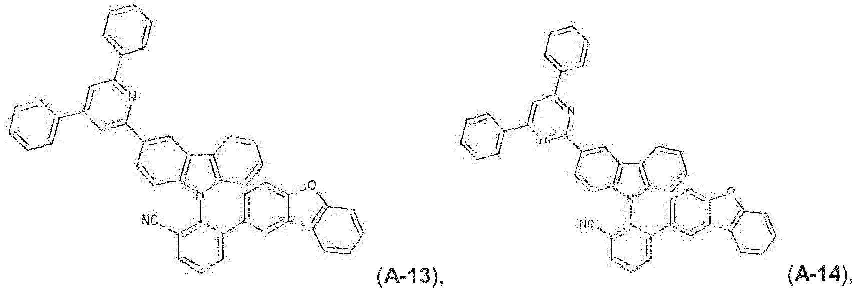 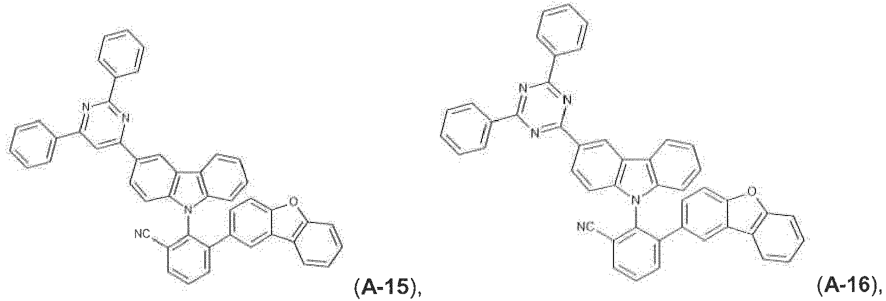 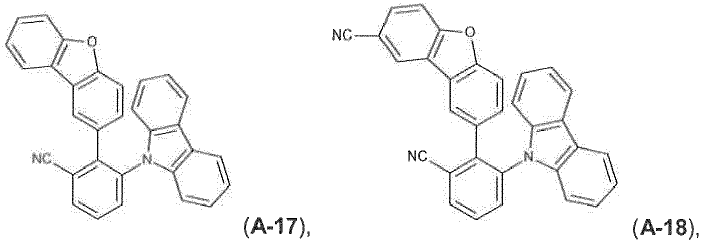 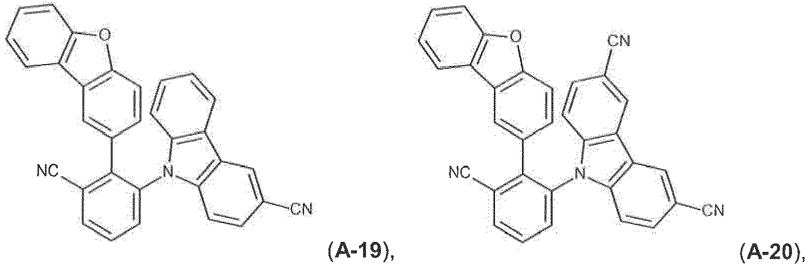 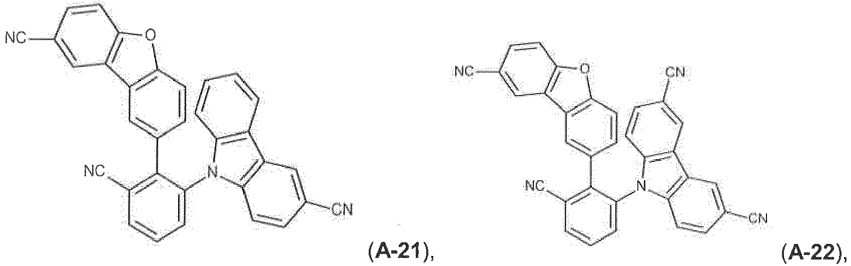 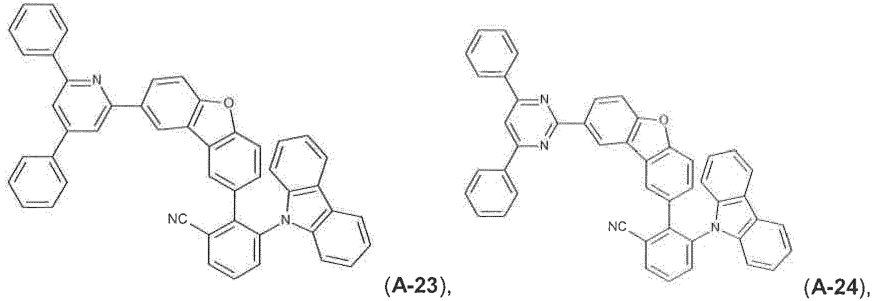 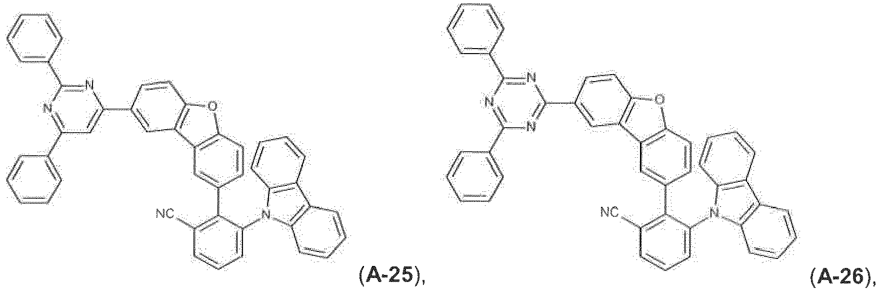 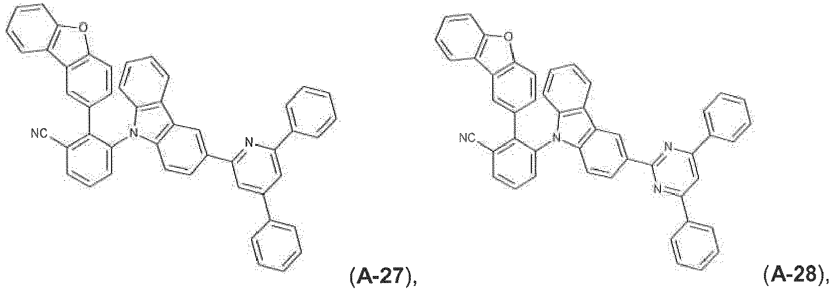 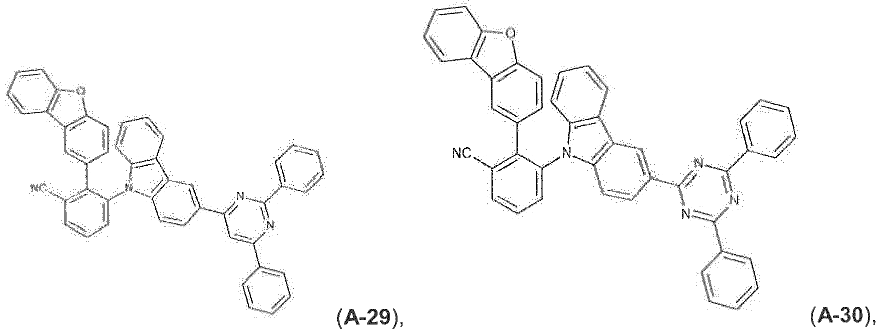 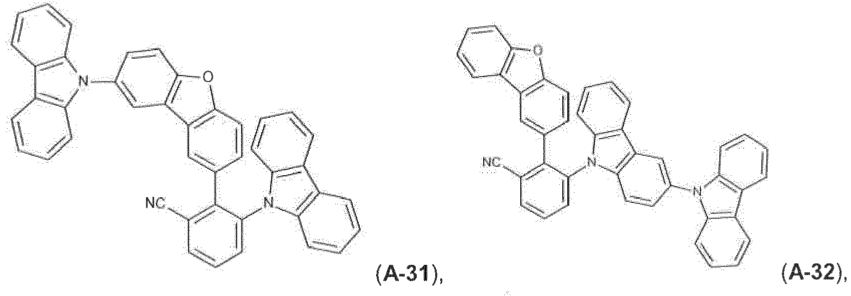 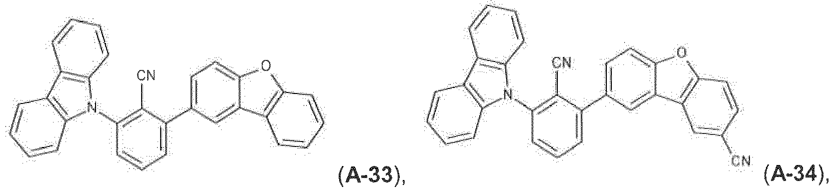 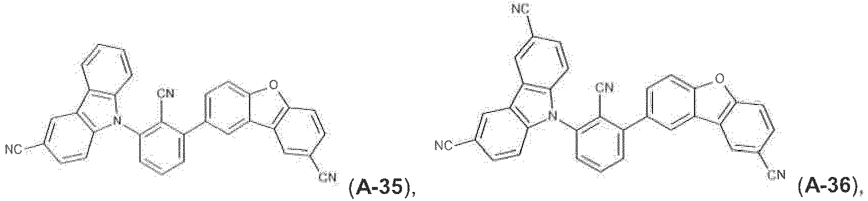 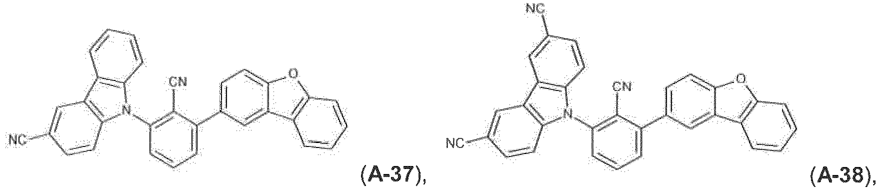 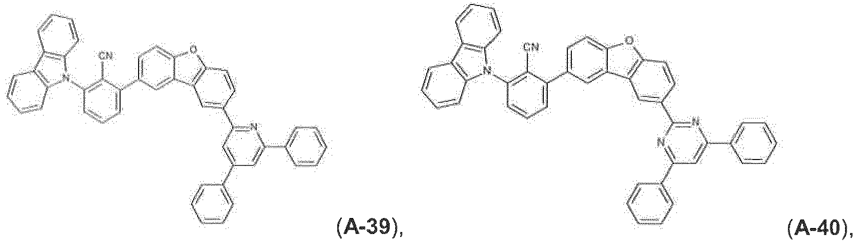 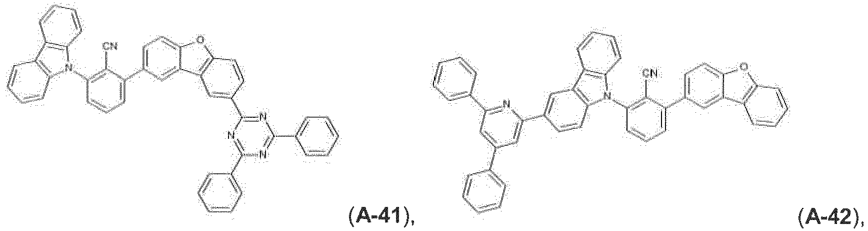 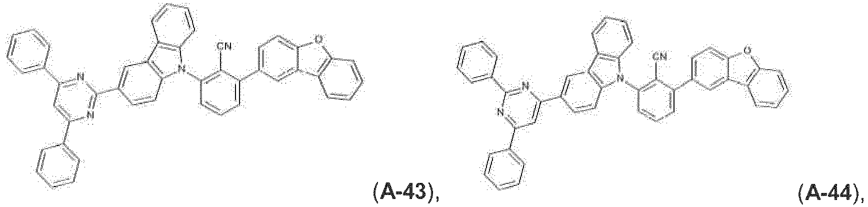 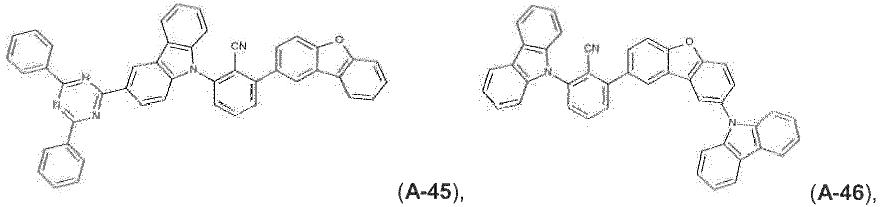 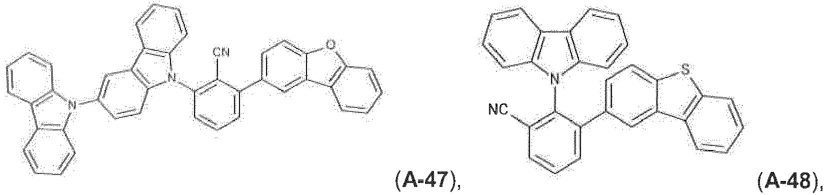 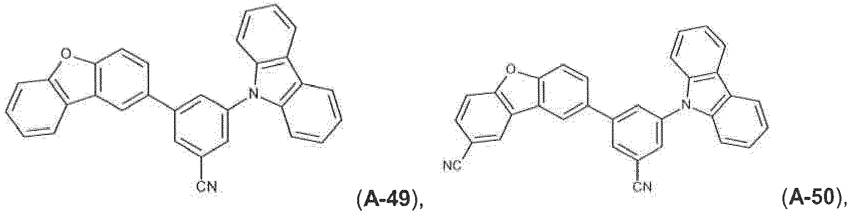 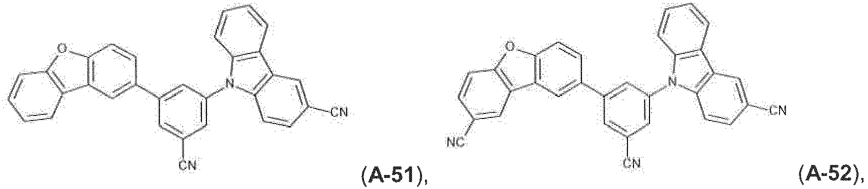 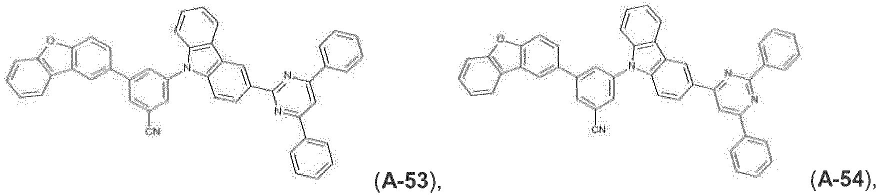 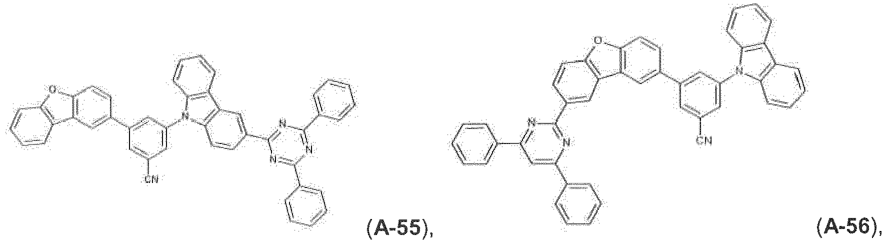 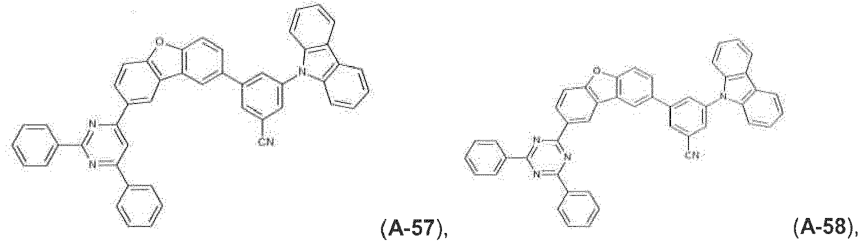 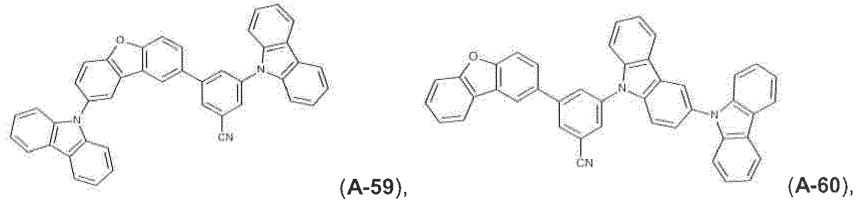 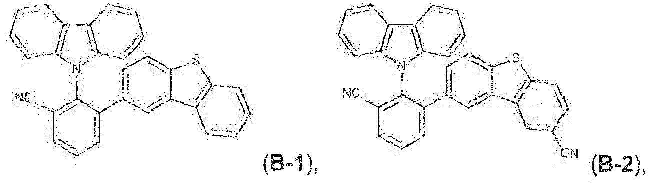 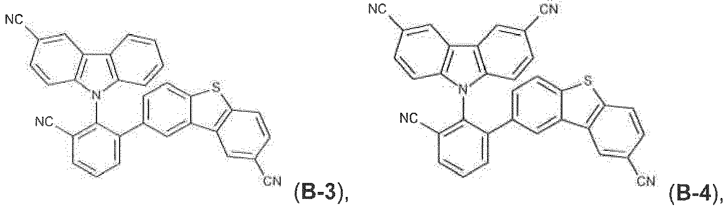 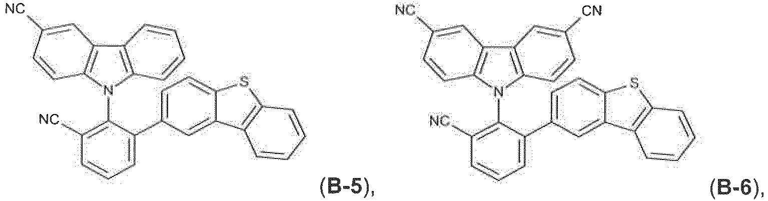 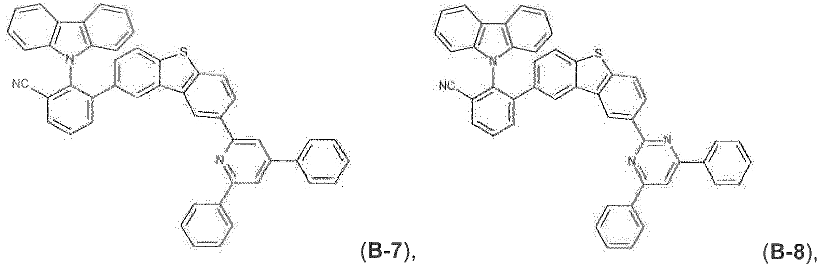 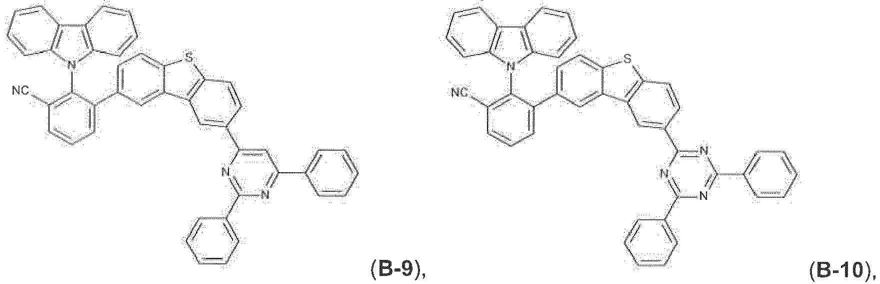 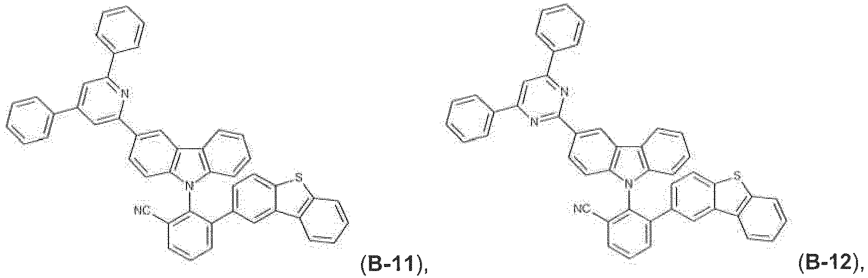 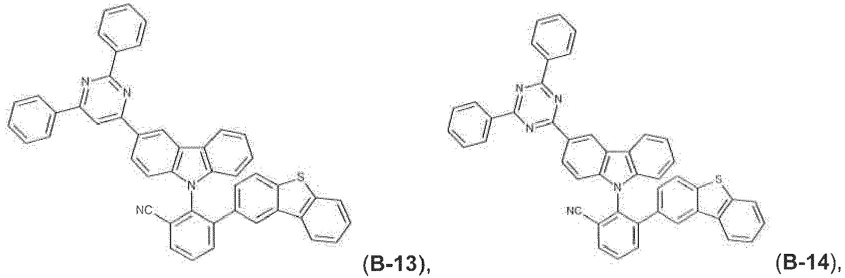 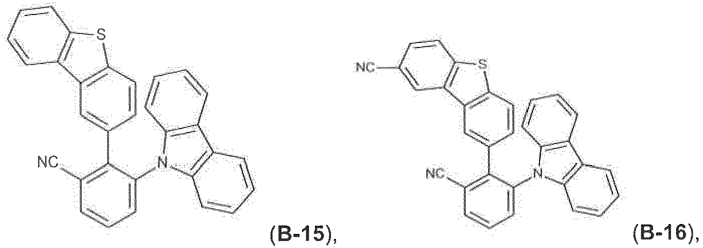 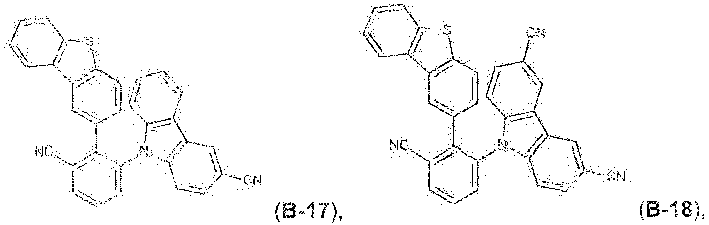 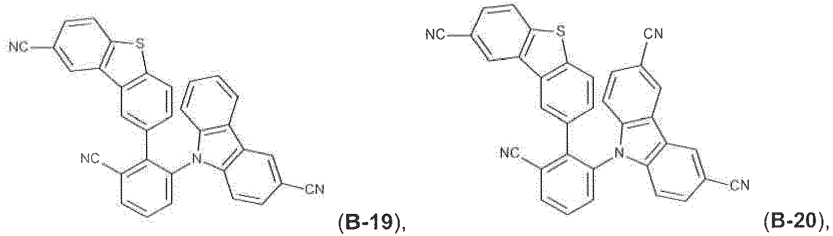 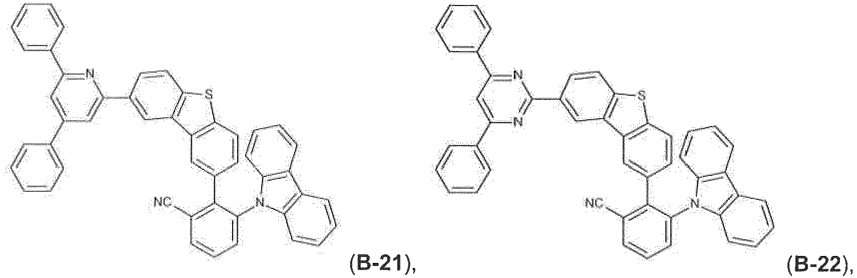 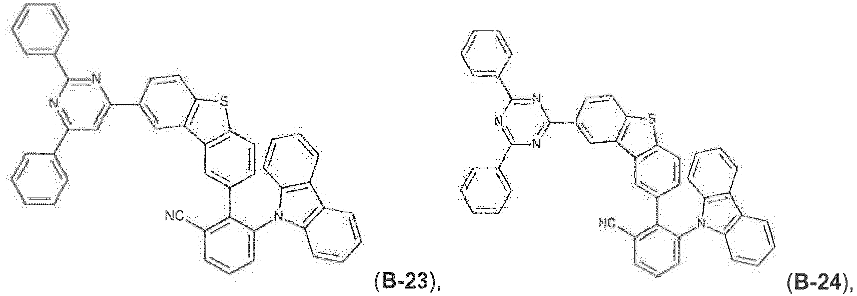 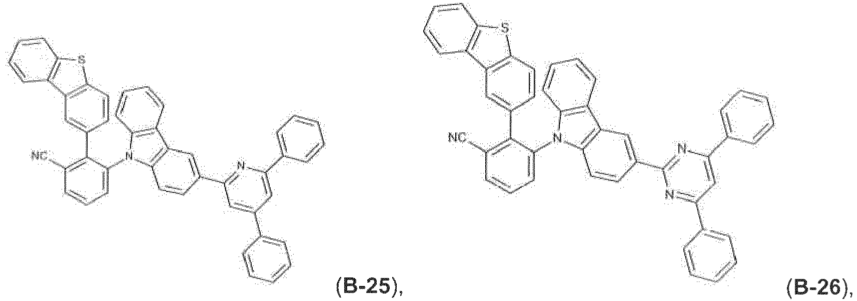 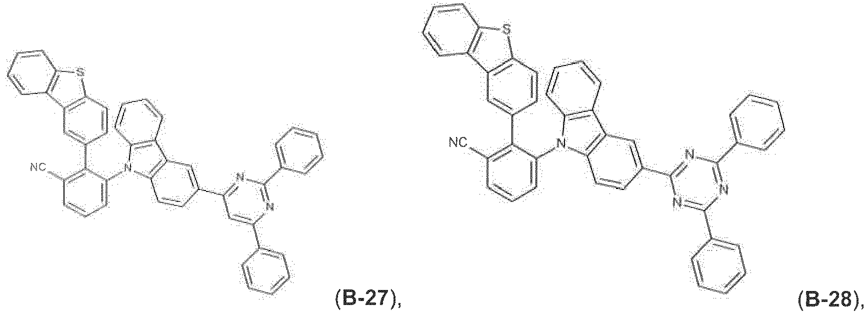 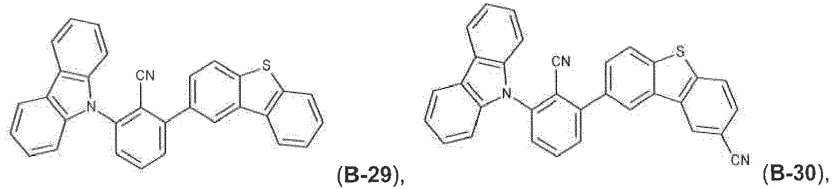 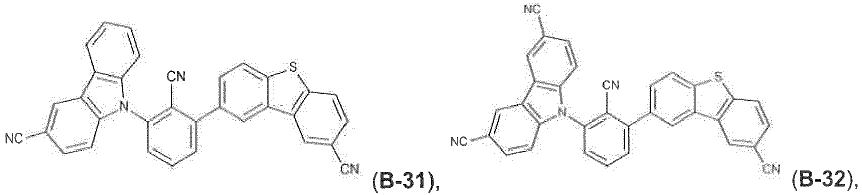 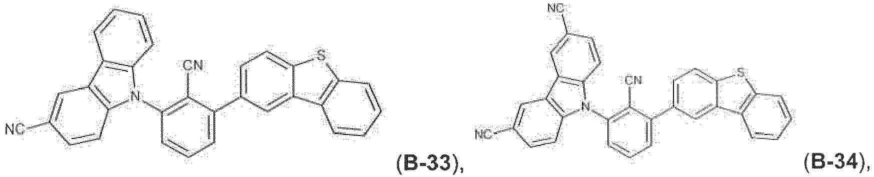 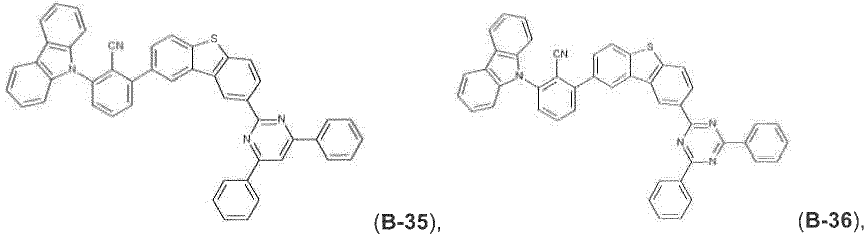 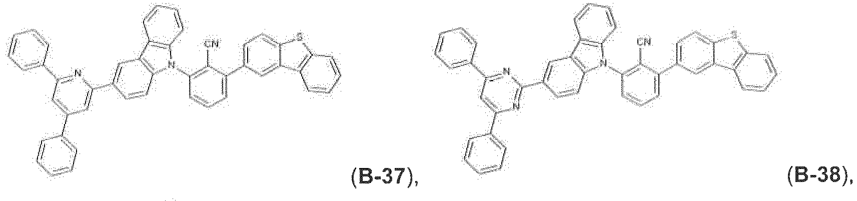 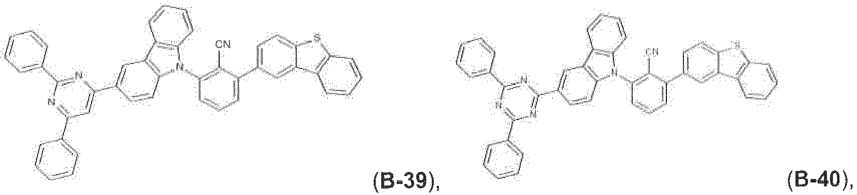 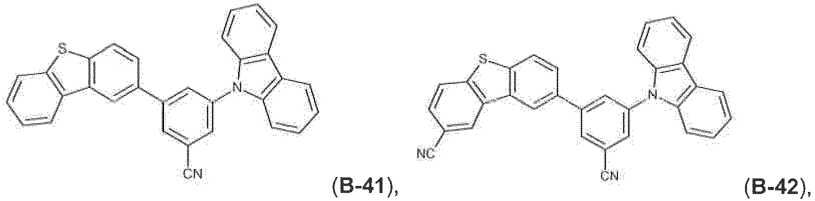 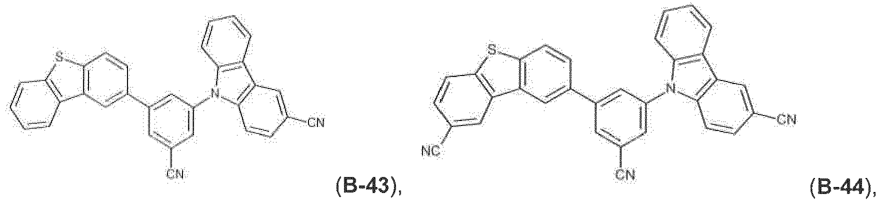 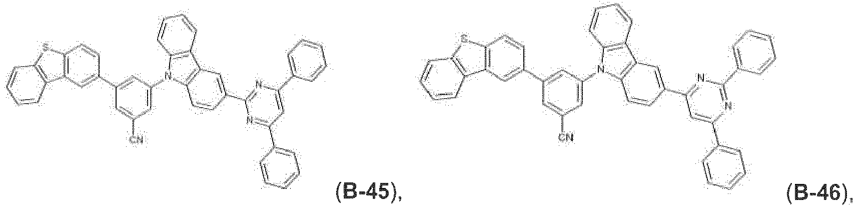 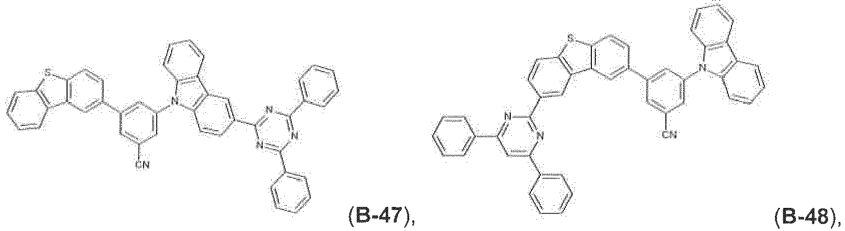 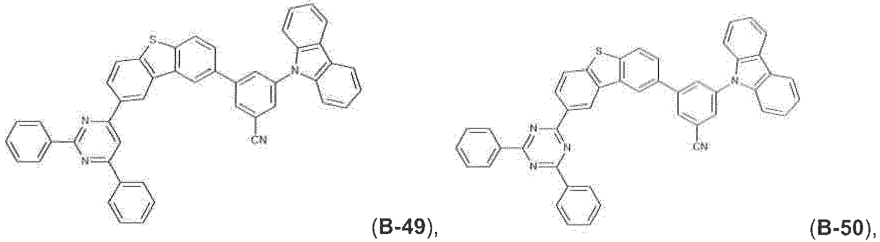 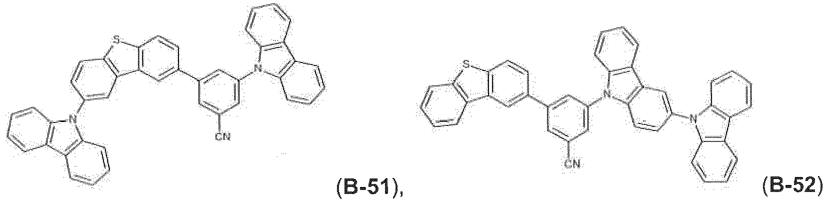 and

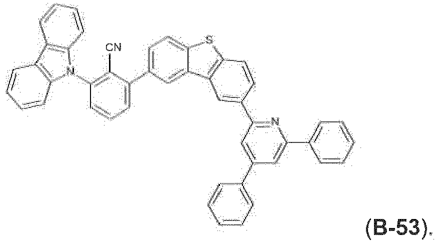

Among compounds of formula (**Ib**) and (**Ib'**) compounds compounds of formula 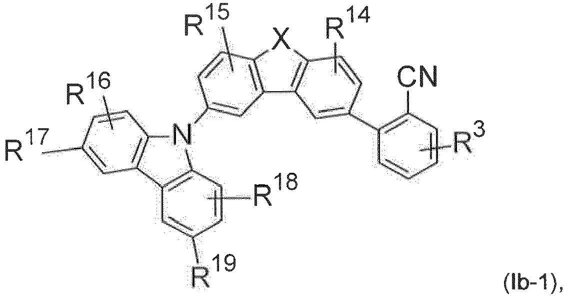 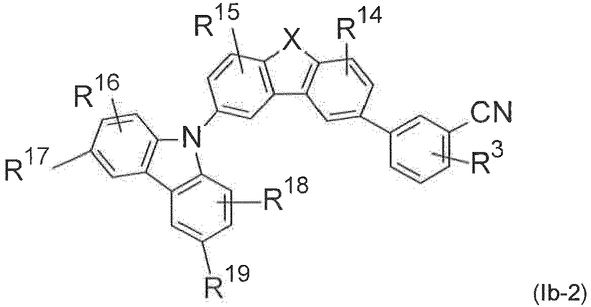 and 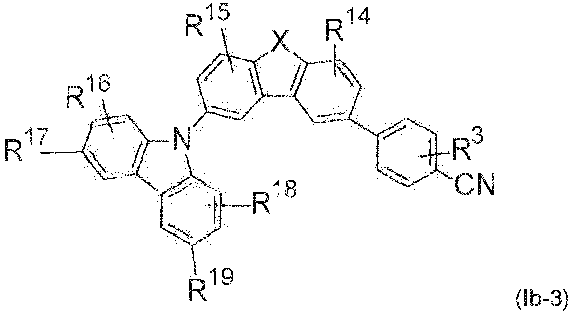 are more preferred, wherein R³ is H, or CN and X, R¹⁴ to R¹⁹ are defined above, or below.

Preferably, R¹⁴, R¹⁵, R¹⁶ and R¹⁸ are independently of each other H, or a C₁-C₂₅alkyl group.
Preferably, R¹⁷ and R¹⁹ are independently of each other H, CN, a group of formula 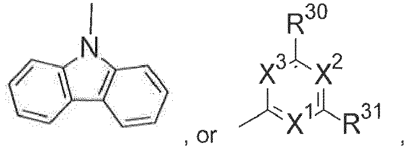 wherein
X¹, X² and X³ are independently of each other N, or CH,
R³⁰ and R³¹ are independently of each other H, or a phenyl group, with the proviso that at least one of X¹, X² and X³ is N.

Examples of particularly preferred compounds of formula (**Ib**) are shown below: 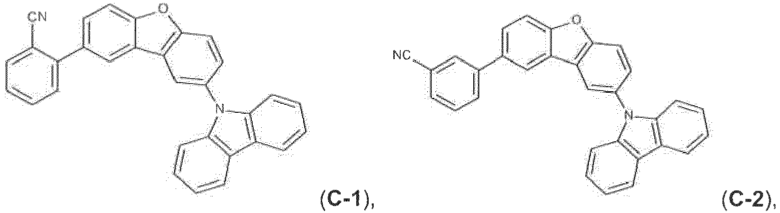 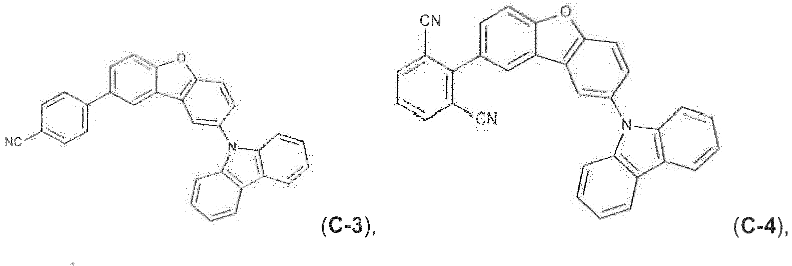 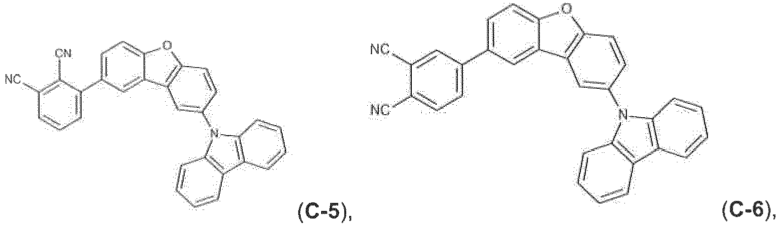 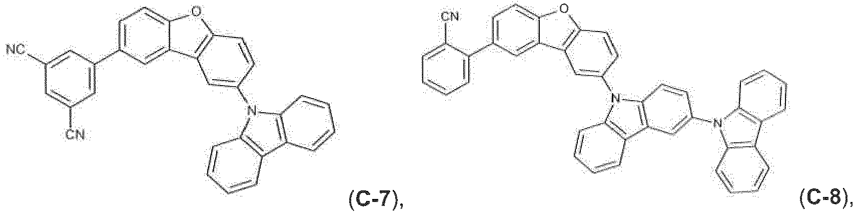 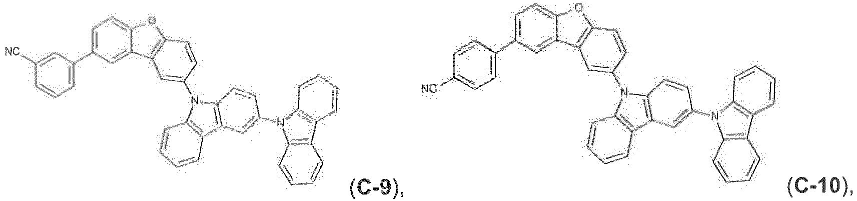 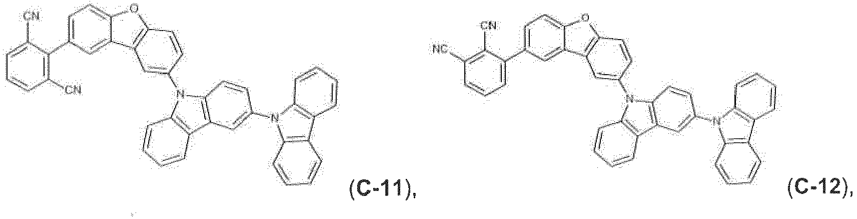 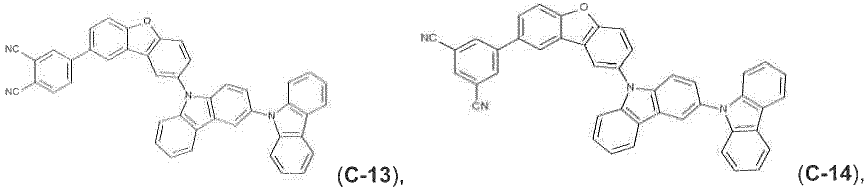 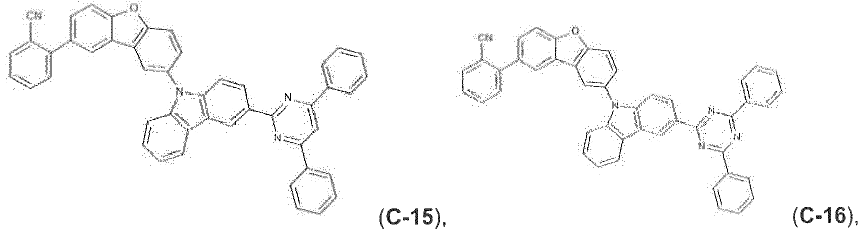 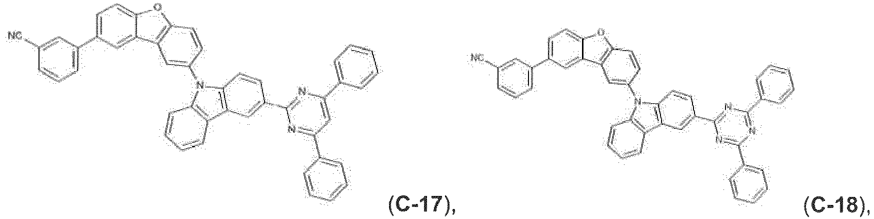 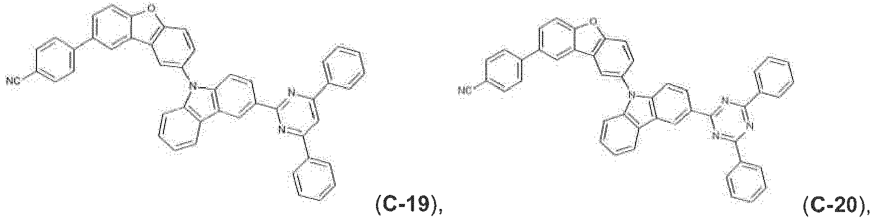 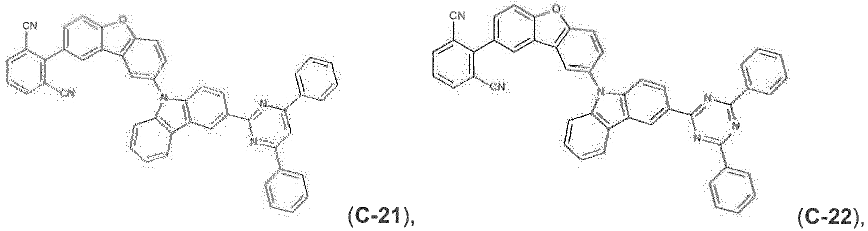 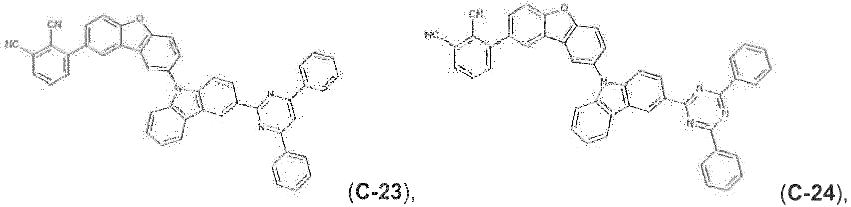 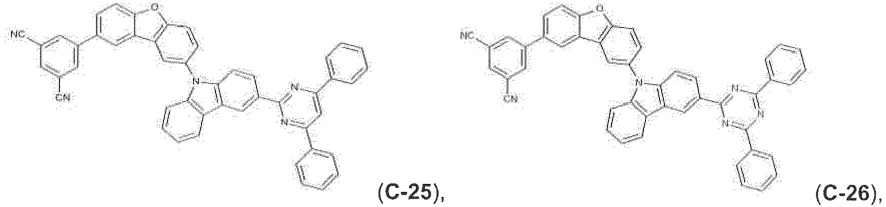 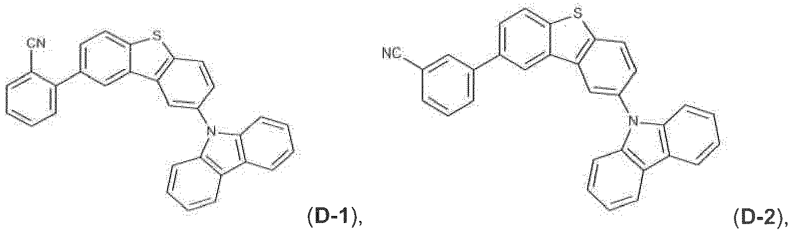 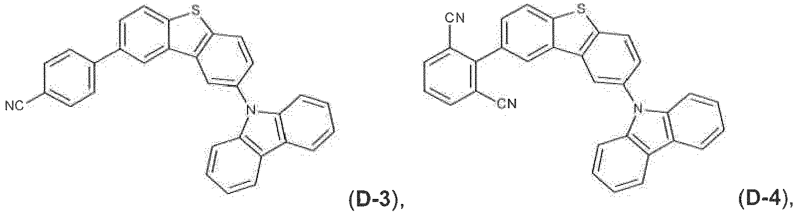 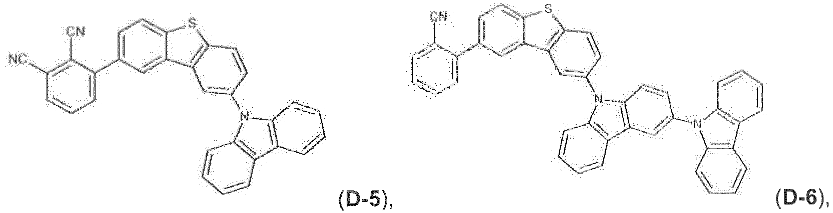 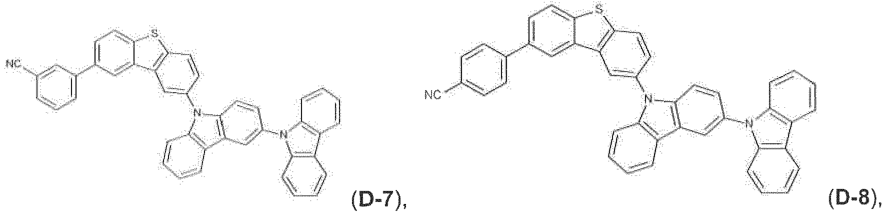 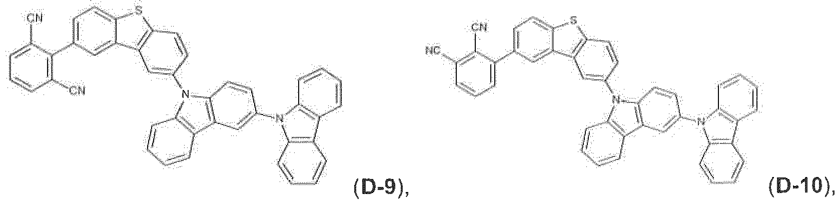 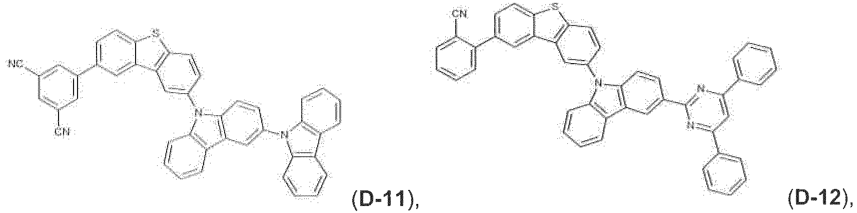 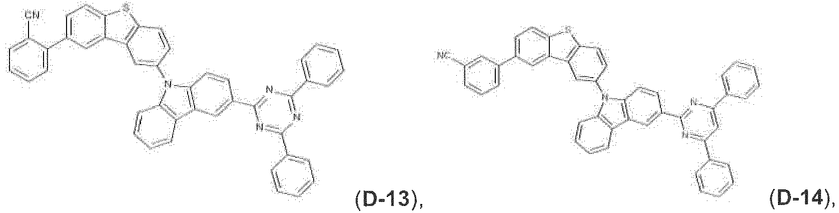 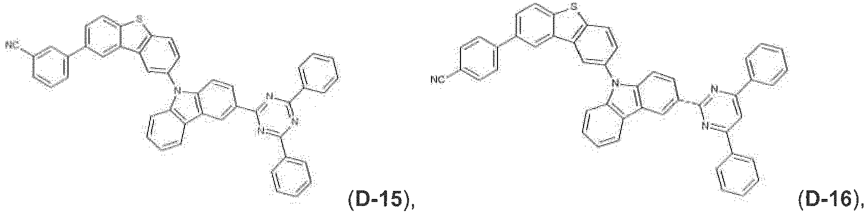 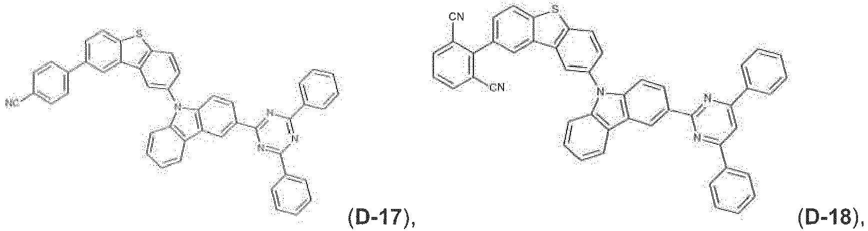 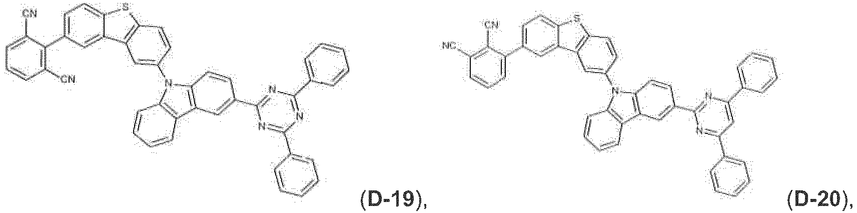 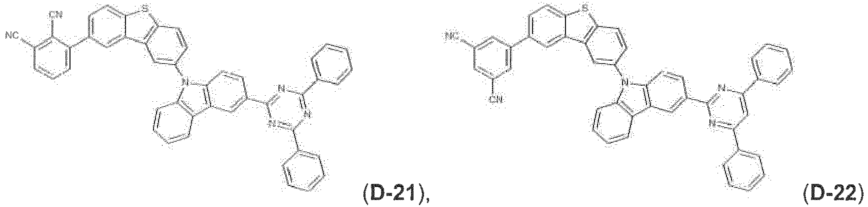 and

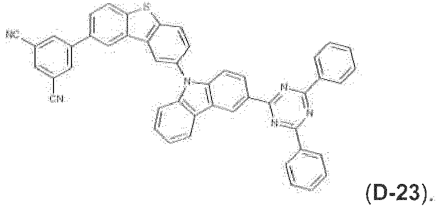

Among the compounds of formula (**I**) those are preferred, wherein X is O.

Compounds (**Ia'**) and (**Ib'** are preferred. Compounds of formula (**Ia-1**), (**Ia-2**), (**Ia-3**), (**Ia-4**), (I**b-1**), (**Ib-2**) and (**Ib-3**), such as, for example, compounds (**A-1**) to (**A-60**), (**B-1**) to (**B-53**), (**C-1**) to (**C-26**) and (**D-1**) to (**D-23**), are more preferred. Compounds of formula (**Ia-1**), (**Ia-2**), (**Ia-3**), (**Ia-4**), (**Ib-1**), (**Ib-2**) and (**Ib-3**), wherein X is O, are most preferred.

C₁-C₂₅alkyl (C₁-C₁₈alkyl) is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups (C₁-C₁₈alkoxy groups) are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, un-decyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₆-C₂₄aryl (C₆-C₁₈aryl), which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted by G. Phenyl, 1-naphthyl and 2-naphthyl are examples of a C₆-C₁₀aryl group.

C₂-C₃₀heteroaryl represents a ring with five to seven ring atoms or a condensed ring system, wherein nitrogen, oxygen or sulfur are the possible hetero atoms, and is typically a heterocyclic group with five to 30 atoms having at least six conjugated π-electrons such as thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, carbazolyl, or phenoxazinyl, which can be unsubstituted or substituted by G. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

G is as defined above and is especially C₁-C₁₈alkyl, very especially C₁-C₈alkyl, such as methyl, ethyl, n-propyl, iso-propyl, n-butyl, isobutyl, tert-butyl, sec-butyl, hexyl, octyl, or 2-ethyl-hexyl.

The wording "substituted by G" means that one, or more, especially one to three substituents G might be present. If a substituent occurs more than one time in a group, it can be different in each occurrence.

As described above, the aforementioned groups may be interrupted by D. Interruptions are of course possible only in the case of groups containing at least 2 carbon atoms connected to one another by single bonds. C₁-C₁₈alkyl interrupted by one or more units D is, for example, (CH₂CH₂O)₁₋₉-R^{x}, where R^{x} is H or C₁-C₁₀alkyl or C₂-C₁₀alkanoyl (e.g. CO-CH(C₂H₅)C₄H₉), CH₂-CH(OR^{y'})-CH₂-O-R^{y}, where R^{y} is C₁-C₁₈alkyl, C₅-C₁₂cycloalkyl, phenyl, C₇-C₁₅phenylalkyl, and R^{y'} embraces the same definitions as R^{y} or is H; C₁-C₈alkylene-COO-R^{z}, e.g. CH₂COOR_{z}, CH(CH₃)COOR^{z}, C(CH₃)₂COOR^{z}, where R^{z} is H, C₁-C₁₈alkyl, (CH₂CH₂O)₁₋₉-R^{x}, and R^{x} embraces the definitions indicated above; CH₂CH₂-O-CO-CHCH₂; CH₂CH(OH)CH₂-O-CO-C(CH₃)=CH₂.

The compounds of formula (**I**) can be synthesized starting from appropriately substituted halogenated benzonitrile derivatives. Suitable benzonitrile derivatives are commercially available such as, for example, as 3-bromo-5-fluoro-benzonitrile, 2-bromo-4-fluoro-benzonitrile, 2-bromo-6-fluoro-benzonitrile, 2-bromo-3-fluoro-benzonitrile, 3-bromo-4-fluoro-benzonitrile, 3-fluoro-2-iodo-benzonitrile, 2-fluoro-3-iodo-benzonitrile, 3-fluoro-5-iodo-benzonitrile, 3-bromo-5-iodo-benzonitrile or 3-bromo-4-iodo-benzonitrile. Alternatively they can be prepared starting from suitable benzoic acid derivatives, converting them to the acid chlorides using, for example, oxalyl chloride (WO2008/79836), or thionylchloride (US 2012/277224). The acid chlorides can be converted to the amides (using, for example, aqueous ammonia (EP2070908)) which can be converted to the corresponding nitriles using, for example, thionylchloride (US2003/236260) or phosphorus pentoxide (US2009/203677).

The halogenated benzonitrile derivatives can be coupled with boronic acids or acid esters using Suzuki reactions as described, for example, in Chem. Rev. 1995, 95, 2457 - 2483.

Halogenated benzonitrile derivatives can be coupled with Amine derivatives using Ullmann or Buchwald-Hartwig conditions as described below. It is also possible to react Cl- or F-substituted benzonitriles with amines using nucleophilic substitution reaction conditions using bases as described, for example, in US2005/87751.

Nitrile substituted carbazoles can be synthesized starting from the corresponding bromo or iodo substituted carbazoles using Rosenmund-von Braun reactions in DMF as described, for example, by D. A. Patrick, European Journal of Medicinal Chemistry (1997) 32. 781-793. Alternatively nitrile substituted phenyl hydrazine can be reacted with cyclohexanone to yield nitrile substituted 2,3,4,9-tetrahydro-1H-carbazole that can be oxidized to nitrile substituted carbazoles by using chloranil as described, for example, by B. M. Barclay, Journal of the Chemical Society, 1945, 530.

Alternatively carbazole can be reacted to the corresponding aldehyde using Vilsmeier-Haack conditions followed by transforming the aldehyde to the nitrile as described by K. Moriyama, Tetrahedron, 201, 68, 4588. Bromo or iodo substituted dibenzofurans can be reacted with CuCN in DMF to yield nitrile substituted dibenzofurans. Alternatively, dibenzofuran carbaldehydes can be reacted with hydroxylamine followed by dehydration to yield nitrile substituted dibenzofurans, or dibenzofuran carboxylic acids can be transformed to the corresponding dibenzofuran carboxylic amides followed by dehydration to yield nitrile substituted dibenzofurans.

The carbazole substituted dibenzofurans can be synthesized by reacting halogenated dibenzofuran derivatives with nitrile substituted carbazoles using copper catalyzed Ullmann conditions as described, for example, in EP2301921, H. Gilman and D. A. Shirley, J. Am. Chem. Soc. 66 (1944) 888; D. Li et al., Dyes and Pigments 49 (2001) 181 - 186 and Eur. J. Org. Chem. (2007) 2147-2151. The reaction can be performed in solvent or in a melt. Suitable solvents are, for example, (polar) aprotic solvents such as dimethyl sulfoxide, dimethylformamide, NMP, tridecane or alcohols.

Alternatively, the carbazole substituted dibenzofurans can be synthesized by reacting halogenated dibenzofuran derivatives with nitrile substituted carbazoles using palladium catalyzed Buchwald-Hartwig conditions as described, for example, in Björn Schlummer, Ulrich Scholz, Adv. Synth. Catal. 2004, 346, 1599 -1626.

Suitable base skeletons of the formula 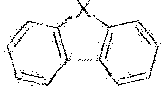 are either commercially available (especially in the cases when X is S, O, NH), or can be obtained by processes known to those skilled in the art. Reference is made to WO2010079051 and EP1885818.

The halogenation can be performed by methods known to those skilled in the art. Preference is given to brominating or iodinating in the 3 and 6 positions (dibromination) or in the 3 or 6 positions (monobromination) of the base skeleton of the formula (II) 2,8 positions (dibenzofuran and dibenzothiophene) or 3,6 positions (carbazole).

Optionally substituted dibenzofurans, dibenzothiophenes and carbazoles can be dibrominated in the 2,8 positions (dibenzofuran and dibenzothiophene) or 3,6 positions (carbazole) with bromine or NBS in glacial acetic acid or in chloroform. For example, the bromination with Br₂ can be effected in glacial acetic acid or chloroform at low temperatures, e.g. 0°C. Suitable processes are described, for example, in M. Park, J.R. Buck, C.J. Rizzo, Tetrahedron, 54 (1998) 12707-12714 for X= NPh, and in W. Yang et al., J. Mater. Chem. 13 (2003) 1351 for X= S. In addition, 3,6-dibromocarbazole, 3,6-dibromo-9-phenylcarbazole, 2,8-dibromodibenzothiophene, 2,8-dibromodibenzofuran, 2-bromocarbazole, 3-bromodibenzothiophene, 3-bromodibenzofuran, 3-bromocarbazole, 2-bromodibenzothiophene and 2-bromodibenzofuran are commercially available.

Monobromination in the 4 position of dibenzofuran (and analogously for dibenzothiophene) is described, for example, in J. Am. Chem. Soc. 1984, 106, 7150. Dibenzofuran (dibenzothiophene) can be monobrominated in the 3 position by a sequence known to those skilled in the art, comprising a nitration, reduction and subsequent Sandmeyer reaction.

Monobromination in the 2 position of dibenzofuran or dibenzothiophene and monobromination in the 3 position of carbazole are effected analogously to the dibromination, with the exception that only one equivalent of bromine or NBS is added.

Alternatively, it is also possible to utilize iodinated dibenzofurans, dibenzothiophenes and carbazoles. The preparation is described, inter alia, in Tetrahedron. Lett. 47 (2006) 6957-6960, Eur. J. Inorg. Chem. 24 (2005) 4976-4984, J. Heterocyclic Chem. 39 (2002) 933-941, J. Am. Chem. Soc. 124 (2002) 11900-11907, J. Heterocyclic Chem, 38 (2001) 77-87.

For the nucleophilic substitution, Cl- or F-substituted dibenzofurans, dibenzothiophenes and carbazoles are required. The chlorination is described, inter alia, in J. Heterocyclic Chemistry, 34 (1997) 891-900, Org. Lett., 6 (2004) 3501-3504; J. Chem. Soc. [Section] C: Organic, 16 (1971) 2775-7, Tetrahedron Lett. 25 (1984) 5363-6, J. Org. Chem. 69 (2004) 8177-8182. The fluorination is described in J. Org. Chem. 63 (1998) 878-880 and J. Chem.

Soc., Perkin Trans. 2, 5 (2002) 953-957. 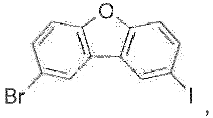 is described in EP1885818.

It has been found that the compounds of the formula **I** are particularly suitable for use in applications in which charge carrier conductivity is required, especially for use in organic electronics applications, for example selected from switching elements such as organic transistors, e.g. organic FETs and organic TFTs, organic solar cells and organic light-emitting diodes (OLEDs), the compounds of the formula **I** being particularly suitable in OLEDs for use as host material in a light-emitting layer and/or as hole and/or exciton blocker material and/or as electron transport material, especially in combination with a phosphorescence emitter, very especially blue, or green emitting phosphorescence emitters. In the case of use of the inventive compounds of the formula I in OLEDs, OLEDs which have good efficiencies and a long lifetime and which can be operated especially at a low use and operating voltage are obtained. Furthermore, the compounds of the formula **I** can be used as conductor/complementary materials in organic electronics applications selected from switching elements and organic solar cells.

In the emission layer or one of the emission layers of an OLED, it is also possible to combine an emitter material with a matrix material of the compound of the formula **I** and a further matrix material which has, for example, a good hole transport property. This achieves a high quantum efficiency of this emission layer.

Suitable structures of organic electronic devices are known to those skilled in the art and are specified below.

The organic transistor generally includes a semiconductor layer formed from an organic layer with hole transport capacity and/or electron transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or electron transport capacity. In addition, it is possible for further layers known to those skilled in the art to be present in the organic solar cell. The layer with electron transport capacity may comprise the compounds of formula **I.**

### Structure of the inventive OLED

The inventive organic light-emitting diode (OLED) generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different from the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an electron/exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an electron/exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc, MTDATA, or dipyrazino[2,3-f:2',3'-h]quinoxaline-2,3,6,7,10,11-hexacarbonitrile (HAT-CN). Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (in-dolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 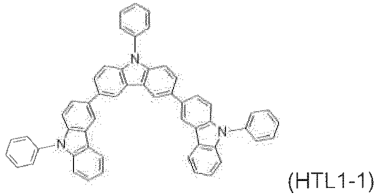 and 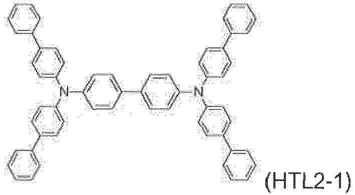 constitute the hole transport layer.
Customarily used hole-transporting molecules are selected from the group consisting of 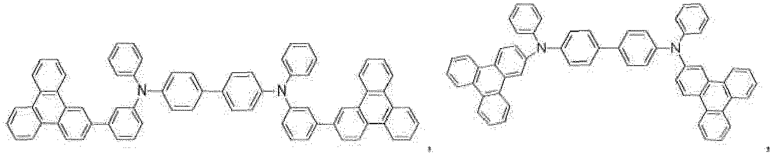 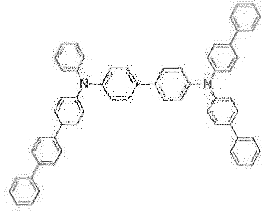 (4-phenyl-N-(4-phenylphenyl)-N-[4-[4-(N-[4-(4-phenyl-phenyl)phenyl]anilino)phenyl]phenyl]aniline), 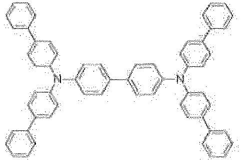 (4-phenyl-N-(4-phenylphenyl)-N-[4-[4-(4-phenyl-N-(4-phenylphenyl)anilino)phenyl]phenyl]aniline), 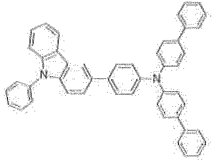 (4-phenyl-N-[4-(9-phenylcarbazol-3-yl)phenyl]-N-(4-phenylphenyl)aniline), 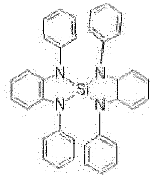 (1,1',3,3'-tetraphenylspiro[1,3,2-benzodiazasilole-2,2'-3a,7a-dihydro-1,3,2-benzodiazasilole]), 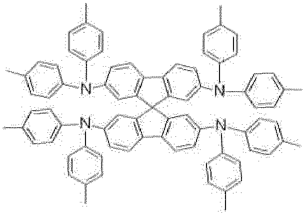 (N2,N2,N2',N2',N7,N7,N7',N7'-octakis(p-tolyl)-9,9'-spirobi[fluorene]-2,2',7,7'-tetramine),4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]-cyclohexane (TAPC), N,N'-bis(4-methylphenyl)-N,N'-bis(4-ethylphenyl)-[1,1'-(3,3'-dimethyl)-biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PE-DOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohex-aazatriphenylene (HAT). In addition, dibenzofurane and dibenzothiophene compounds mentioned as host below, such as, for example, 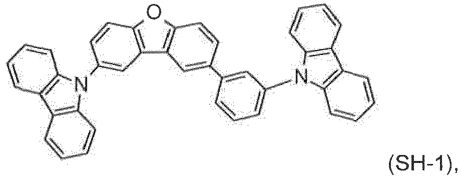 may be used as hole transport material and exciton blocker material.

In a preferred embodiment it is possible to use metal carbene complexes as hole transport materials. Suitable carbene complexes are, for example, carbene complexes as described in WO2005/019373A2, WO2006/056418 A2, WO2005/113704, WO2007/115970, WO2007/115981, WO2008/000727 and WO2014147134. One example of a suitable carbene complex is Ir(DPBIC)₃ with the formula: 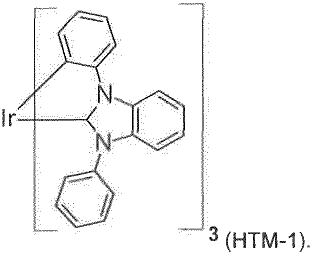 Another example of a suitable carbene complex is Ir(ABIC)₃ with the la:

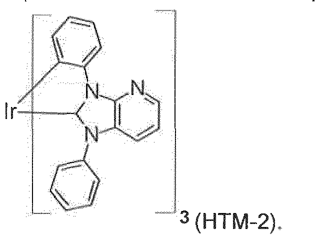

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6H-naphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254. Preferred mixtures comprise the aforementioned carbene complexes, such as, for example, the carbene complexes **HTM-1** and **HTM-2**, and MoO₃ and/or ReO₃. In a particularly preferred embodiment the hole transport layer comprises from 0.1 to 10 wt % of MoO₃ and 90 to 99.9 wt % carbene complex, especially of a carbene complex **HTM-1** and **HTM-2**, wherein the total amount of the MoO₃ and the carbene complex is 100 wt %. In another particularly preferred embodiment the hole transport layer comprises from 0.1 to 10 wt % of MoO₃ and 90 to 99.9 wt % dibenzofurane compound, especially compound (**SH-1**), wherein the total amount of the MoO₃ and the dibenzofurane compound is 100 wt %.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer. Suitable metal complexes for use as electron/exciton blocker material are, for example, carbene complexes as described in WO2005/019373A2, WO2006/056418A2, WO2005/113704, WO2007/115970, WO2007/115981, WO2008/000727 and WO2014147134. Explicit reference is made here to the disclosure of the WO applications cited, and these disclosures shall be considered to be incorporated into the content of the present application. One example of a suitable carbene complex is compound **HTM-1.** Another example of a suitable carbene complex is compound **HTM-2.** In addition, all hole transport materials having sufficient high triplet energy are suitable exciton blocking materials. Additional examples of hole transporting materials having sufficient high triplet energy are (**SH-1**) and (**SH-11**).

### Emitting layer (e)

The light-emitting layer (e) comprises at least one emitter material. In principle, it may be a fluorescence or phosphorescence emitter, suitable emitter materials being known to those skilled in the art. The at least one emitter material is preferably a phosphorescence emitter The phosphorescence emitter compounds used with preference are based on metal complexes, and especially the complexes of the metals Ru, Rh, Ir, Pd and Pt, in particular the complexes of Ir, have gained significance. The compounds of the formula I can be used as the matrix in the light-emitting layer.

Suitable metal complexes for use in the inventive OLEDs are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266, WO2012/172482, WO2015000955 and PCT/EP2014/066272.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldi-benzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetylacetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable: tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenylphenanthroline)europium(III), tris(dibenzoylmethane)mono(4,7-di-methylphenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)-benzoylmethane)]mono(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbene complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, WO2015000955 and PCT/EP2014/066272.

Preferably, the light emitting layer (e) comprises at least one carbine complex as phosphorescence emitter. Suitable carbine complexes are, for example, compounds of the formula 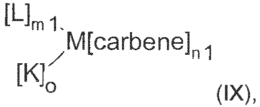 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom;
Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹;
n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 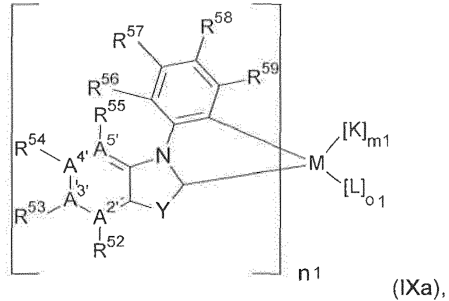 which are described in WO2011/073149, where M, n1, Y, A^{2'}, A^{3'}, A^{4'}, A^{5'}, R⁵¹, R⁵², R⁵³, R⁵⁴, R⁵⁵, R⁵⁶, R⁵⁷, R⁵⁸, R⁵⁹, K, L, m1 and o1 are each defined as follows:
M is Ir, or Pt,
n1 is an integer selected from 1, 2 and 3,
Y is NR⁵¹, O, S or C(R²⁵)₂,
A^{2'}, A^{3'}, A^{4'}, and A^{5'} are each independently N or C, where 2 A' = nitrogen atoms and at least one carbon atom is present between two nitrogen atoms in the ring,
R⁵¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R⁵², R⁵³, R⁵⁴ and R⁵⁵ are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵³ and R⁵⁴ together with A^{3'} and A^{4'} form an optionally substituted, unsaturated ring optionally interrupted by at least one further heteroatom and having a total of 5 to 18 carbon atoms and/or heteroatoms,
R⁵⁶, R⁵⁷, R⁵⁸ and R⁵⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R⁵⁶ and R⁵⁷, R⁵⁷ and R⁵⁸ or R⁵⁸ and R⁵⁹, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R⁵⁵ and R⁵⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R²⁵ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula **IX** is preferably a compound of the formula: 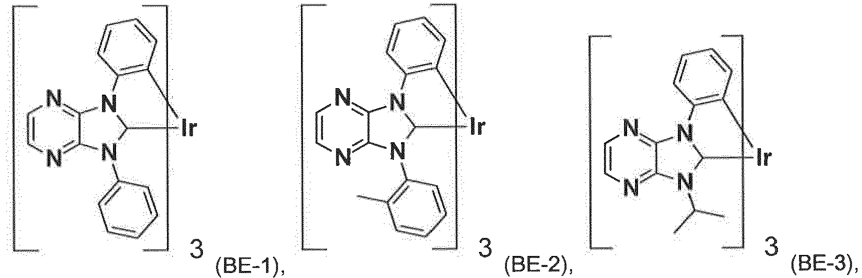 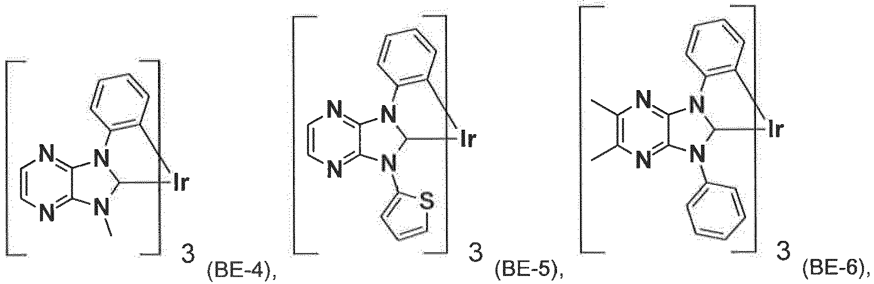 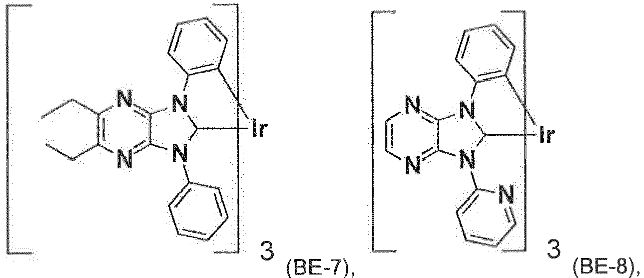 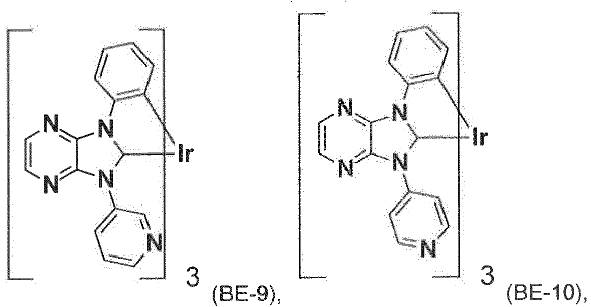 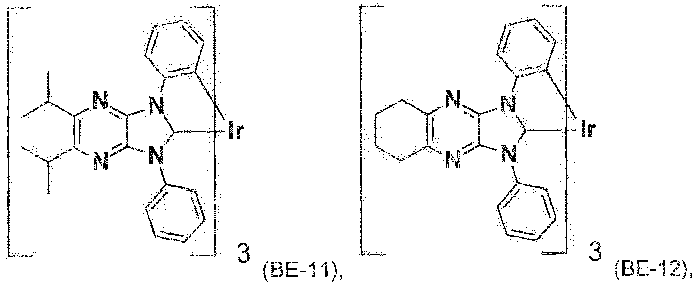 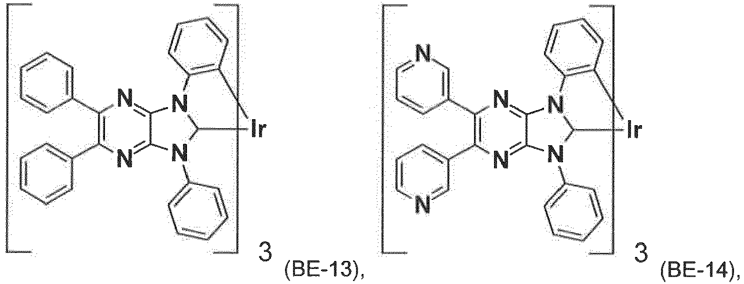 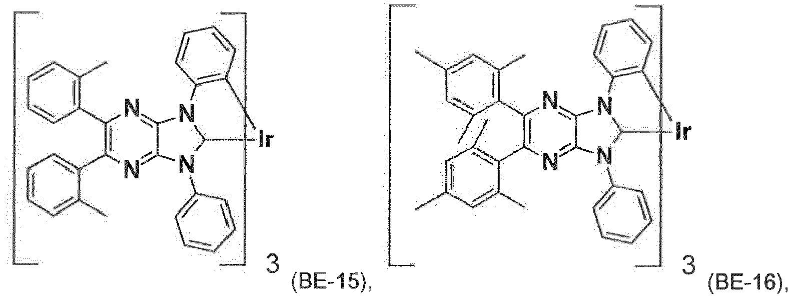 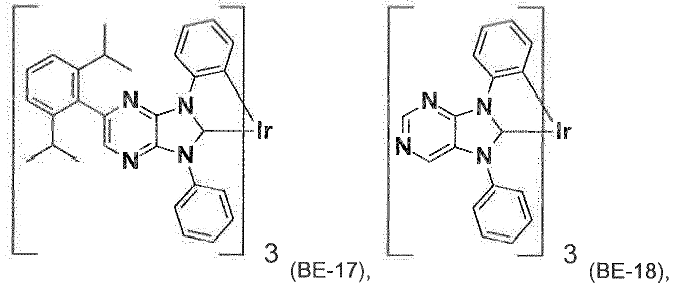 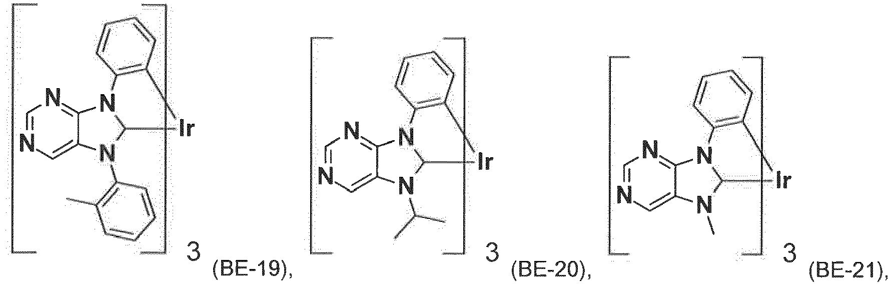 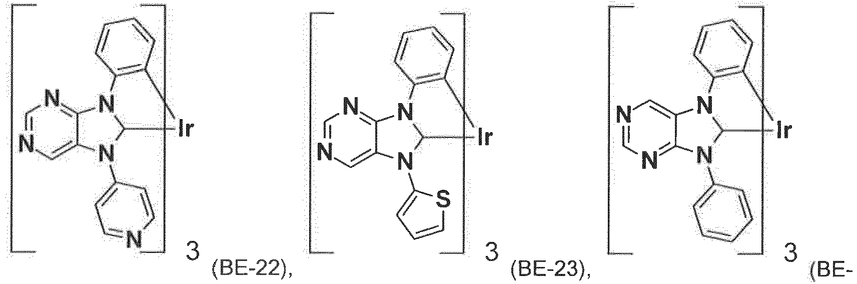 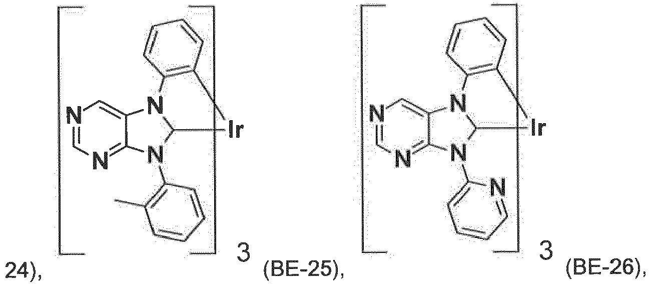 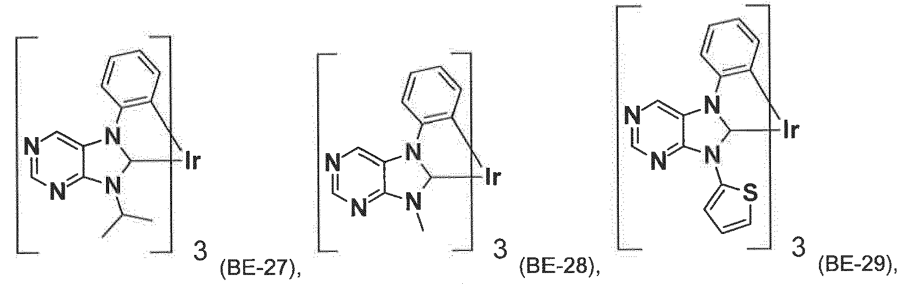 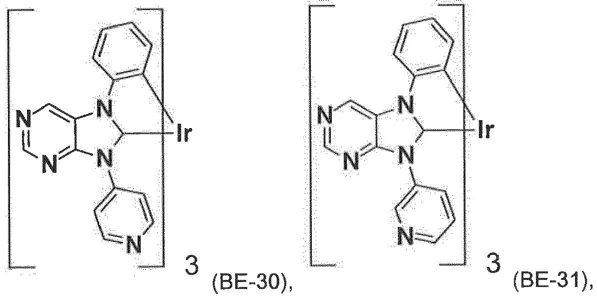 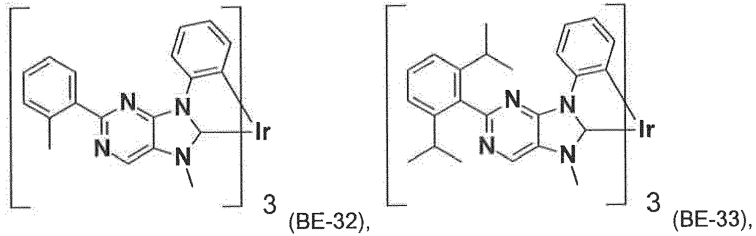 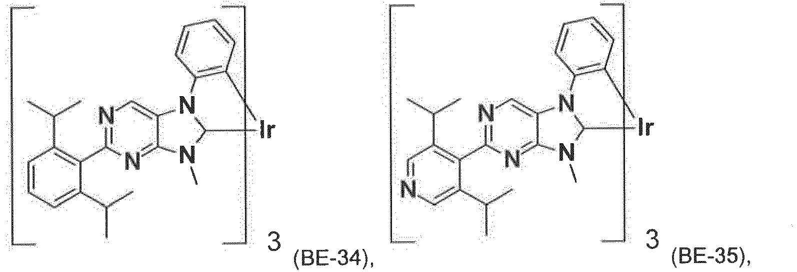 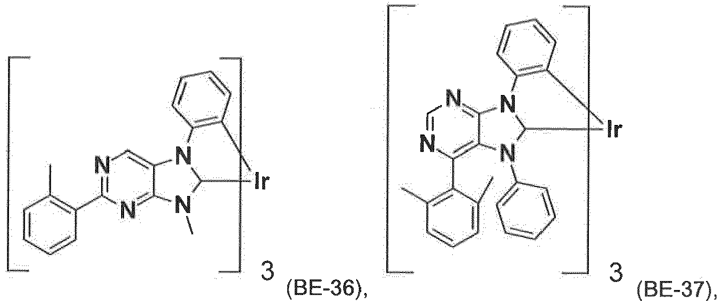 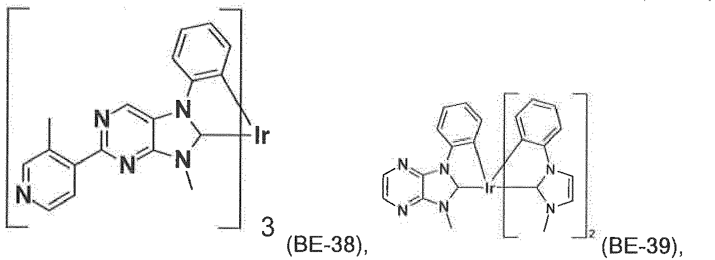 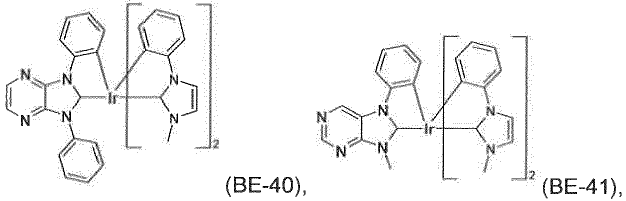 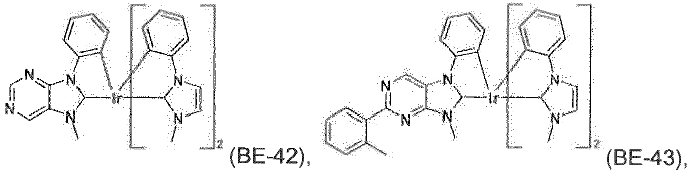 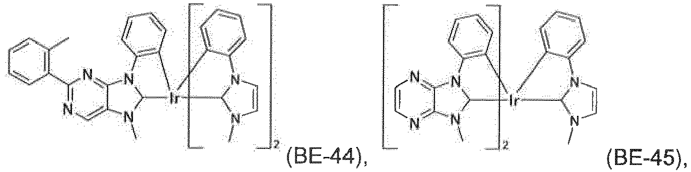 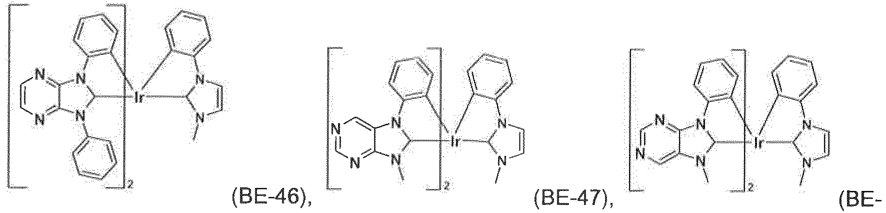 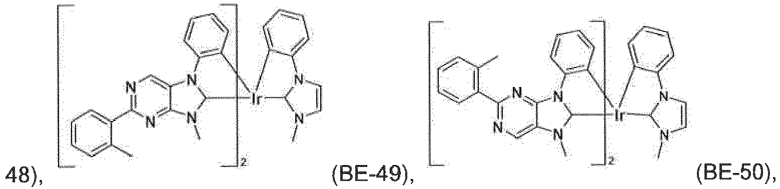 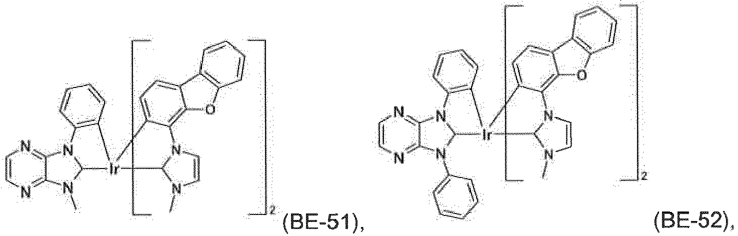 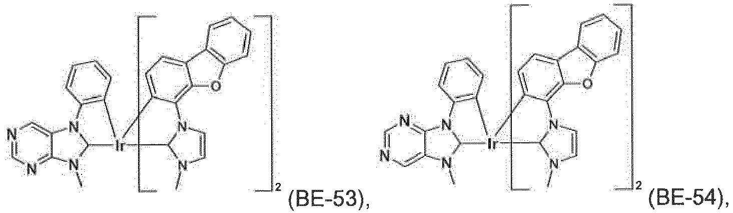 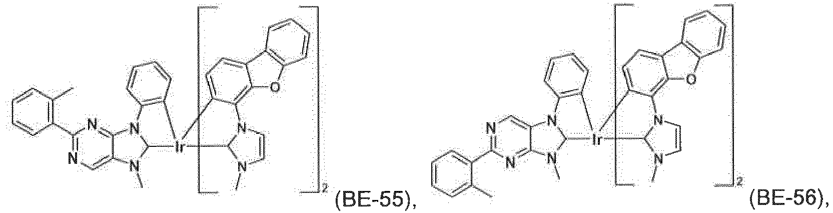 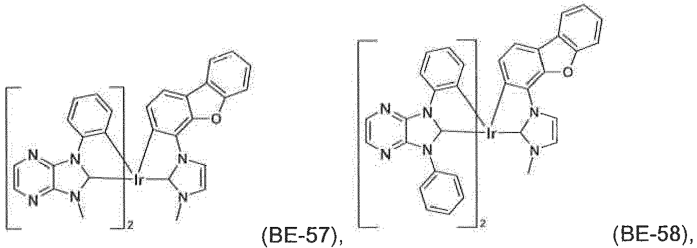 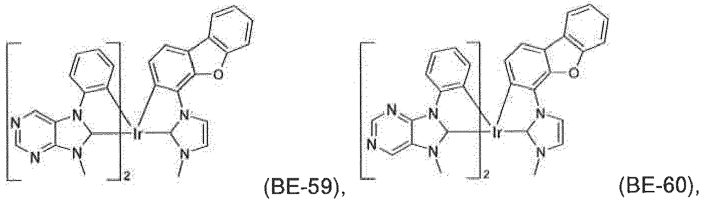 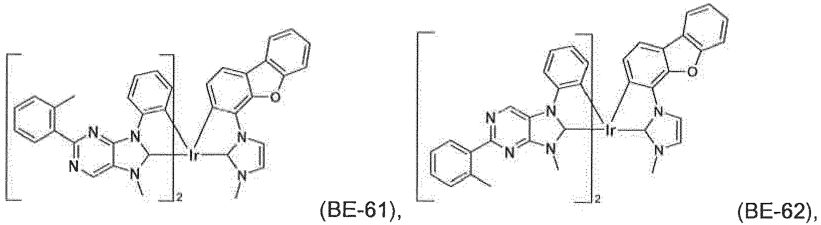 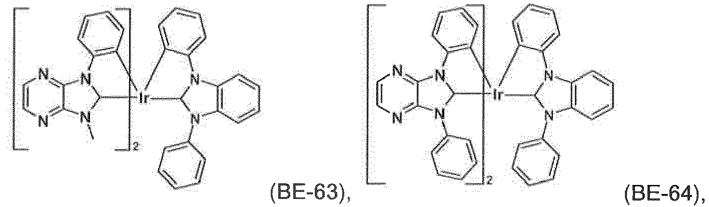 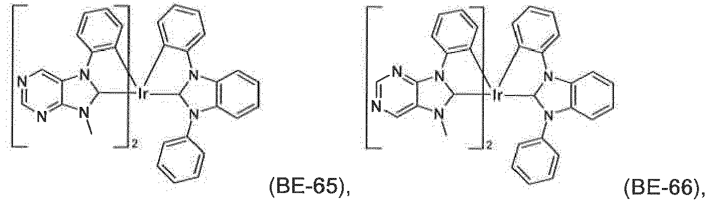 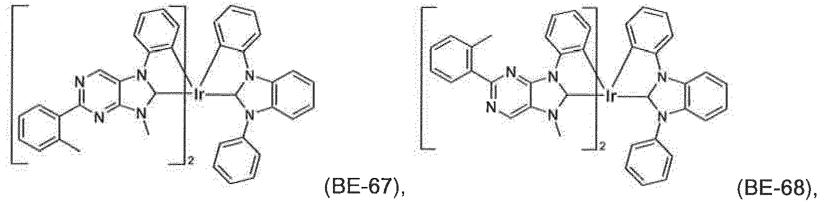 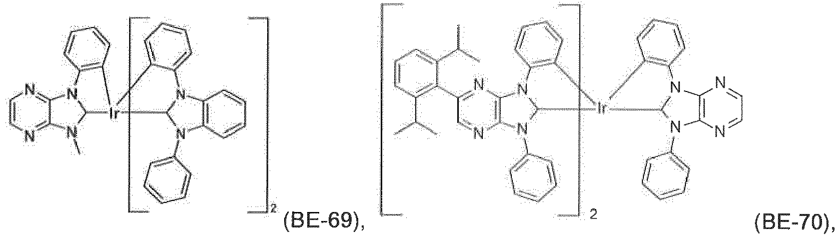 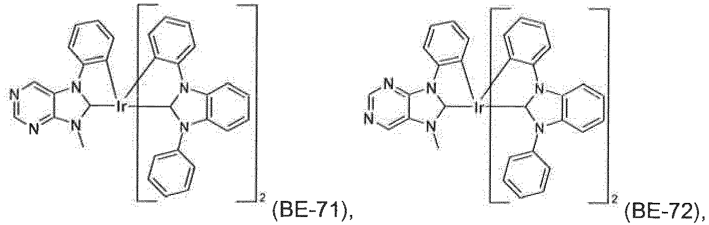 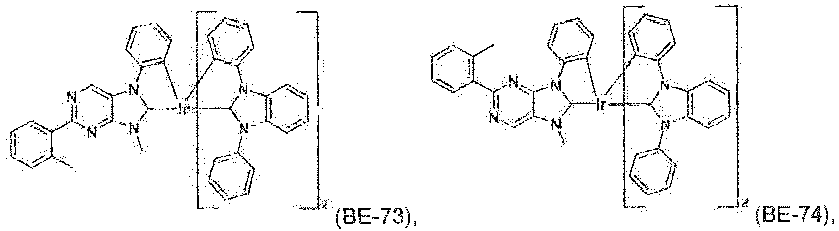 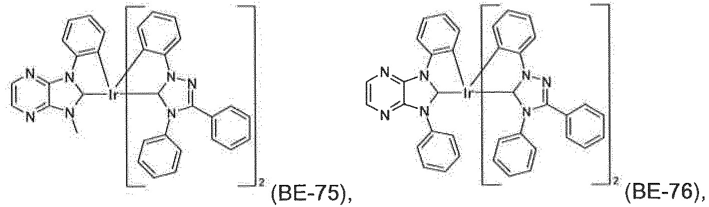 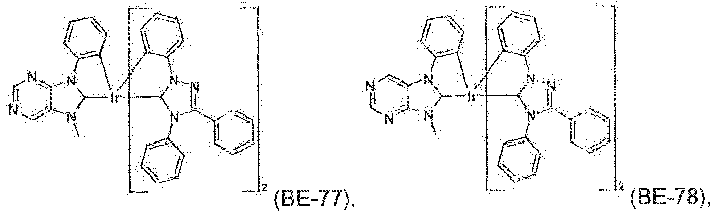 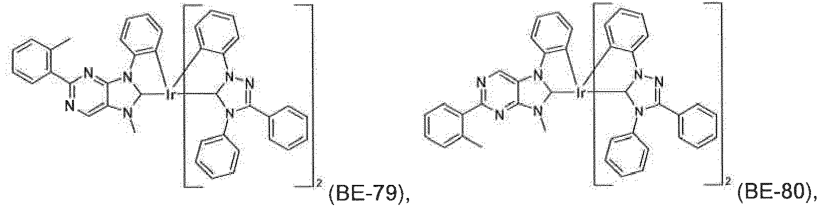 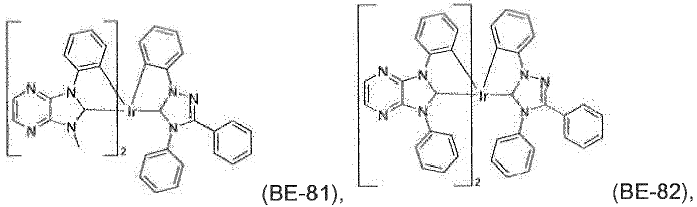 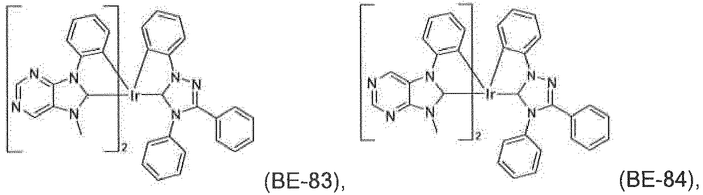 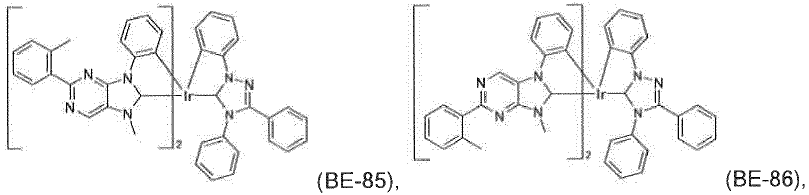 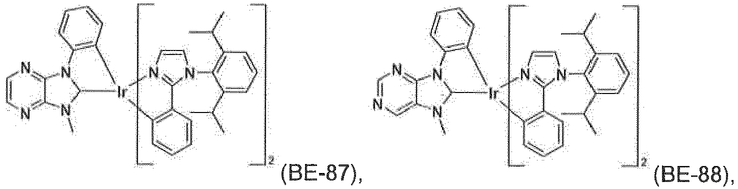 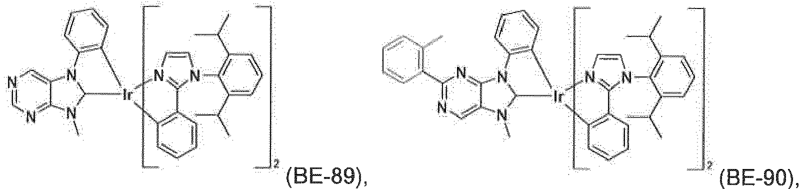 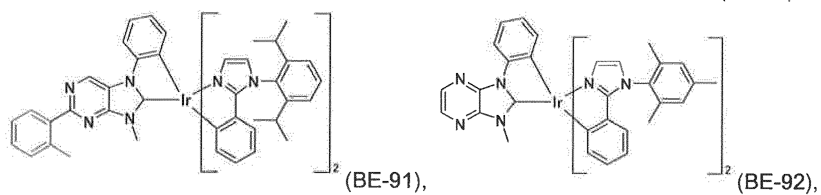 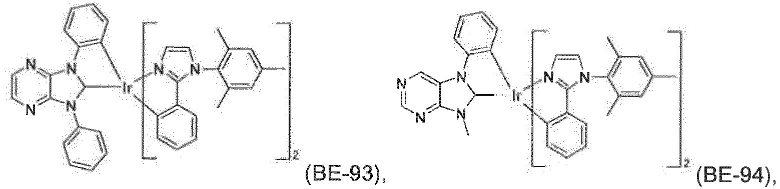 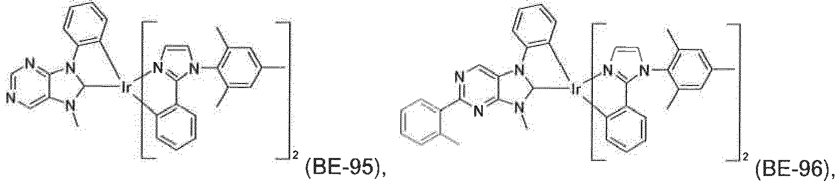 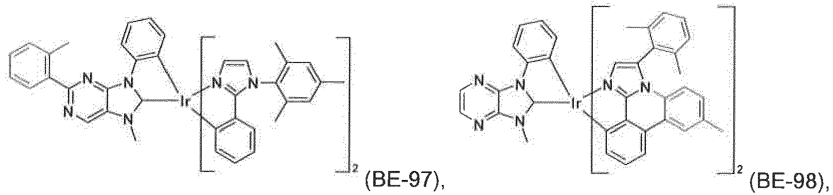 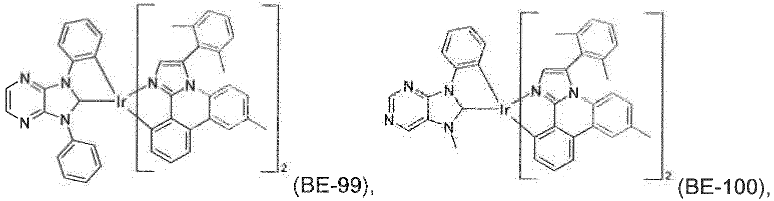 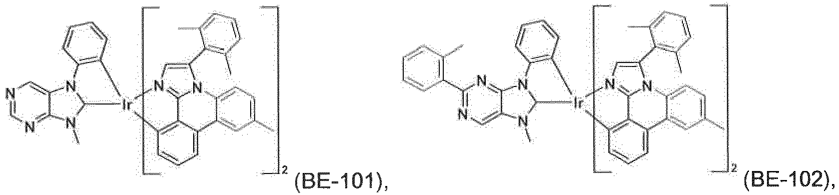 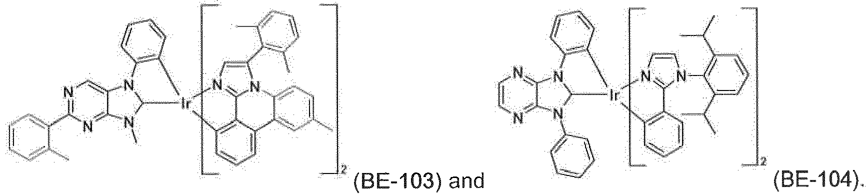 Further suitable non-carbene emitter materials are mentioned below: 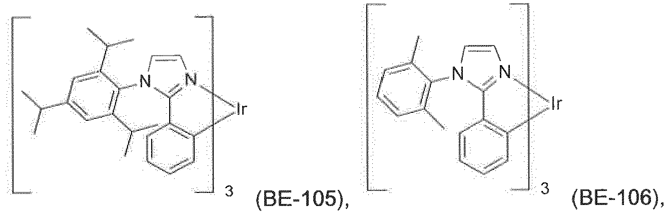 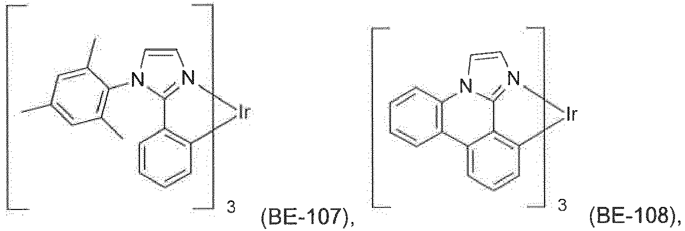 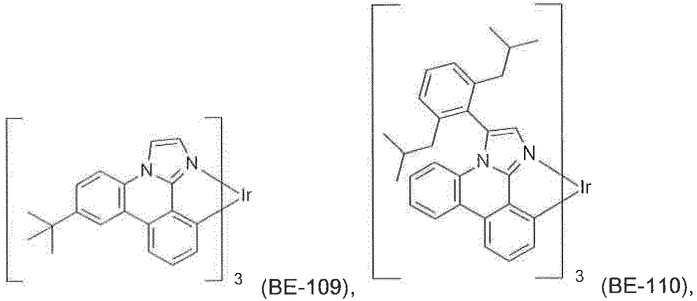 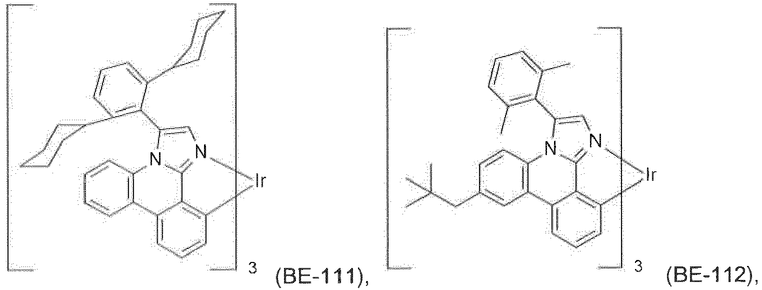 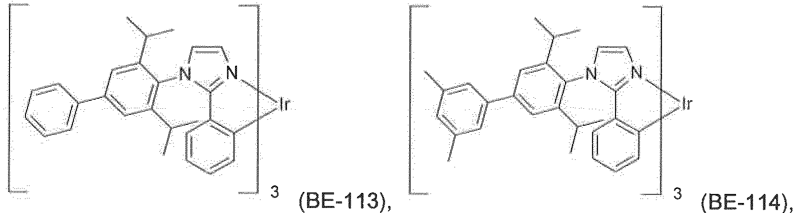 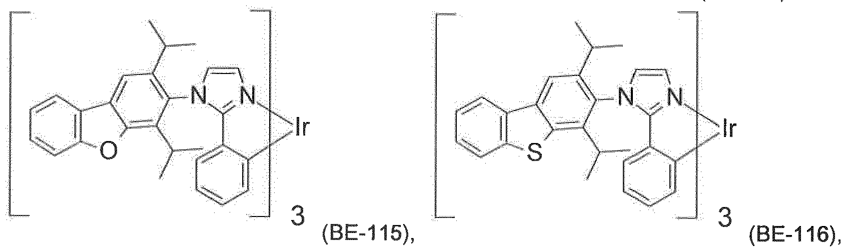 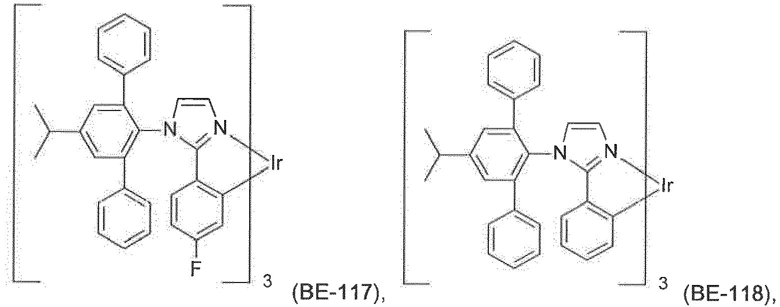 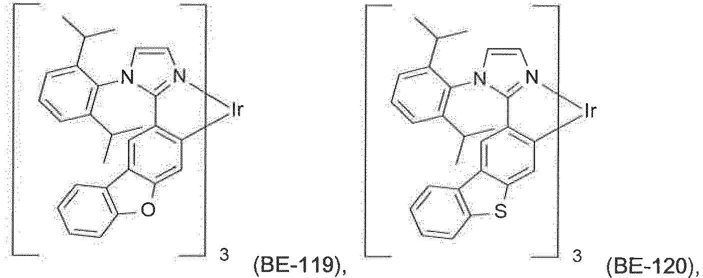 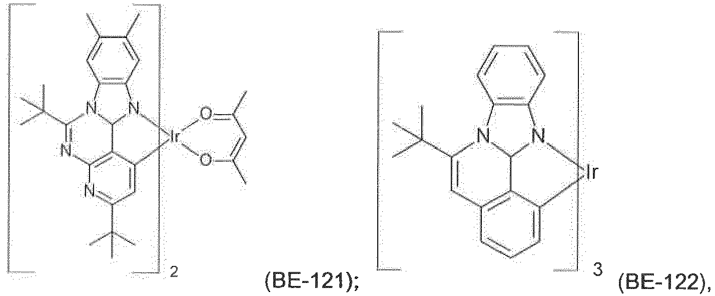 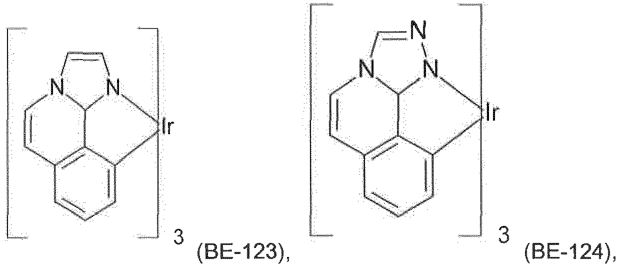 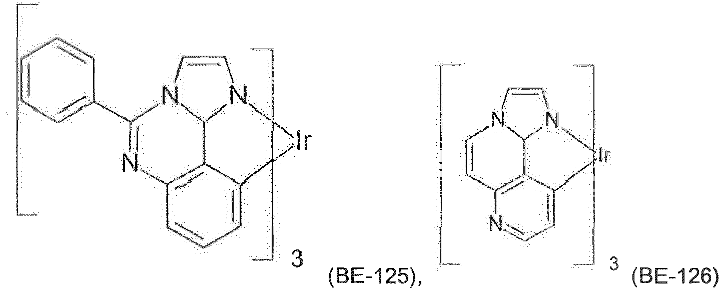 and

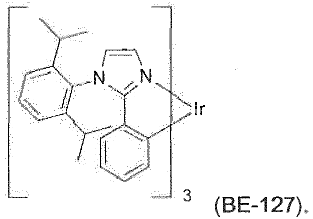

The compound of formula **IX** is more preferably a compound (**BE-1**), (**BE-2**), (**BE-7**), (**BE-12**), (**BE-16**), (**BE-64**), or (**BE-70**). The most preferred phosphorescent blue emitters are compounds (**BE-1**) and (**BE-12**).

The homoleptic metal-carbene complexes may be present in the form of facial or meridional isomers, preference being given to the facial isomers.

Suitable carbene complexes of formula (**IX**) and their preparation process are, for example, described in WO2011/073149.

The compounds of the present invention can also be used as host for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO 2012053627; US6921915, US20090039776; JP2007123392 and European patent application no. 14180422.9.

Examples of suitable phosphorescent green emitters are shown below:

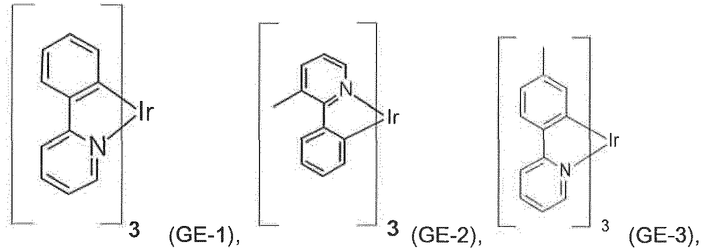

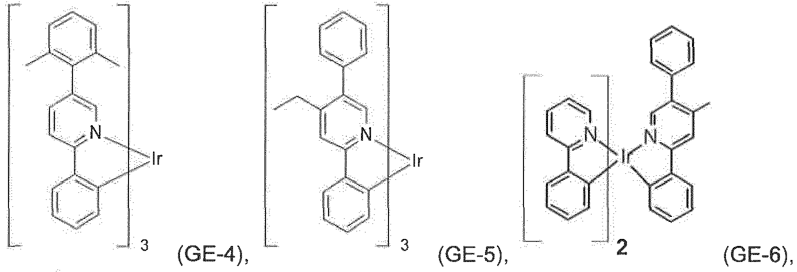

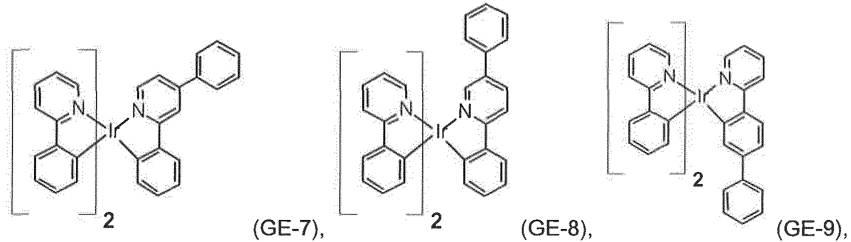

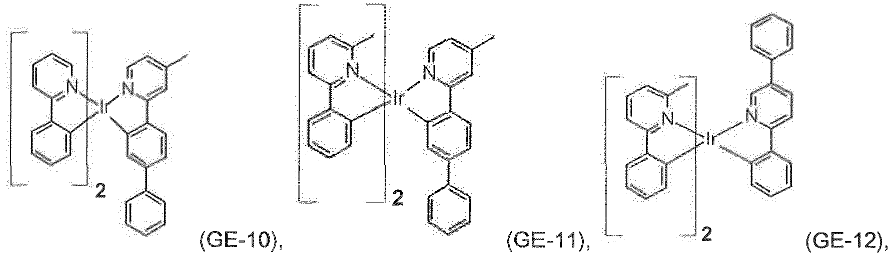

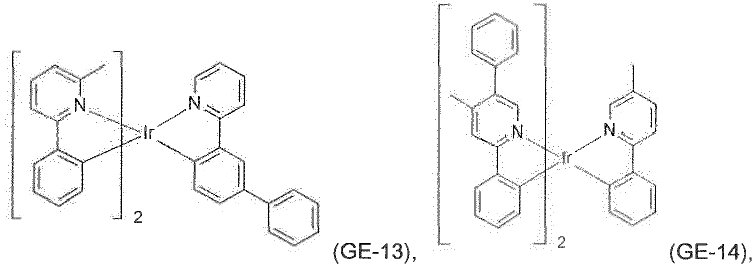

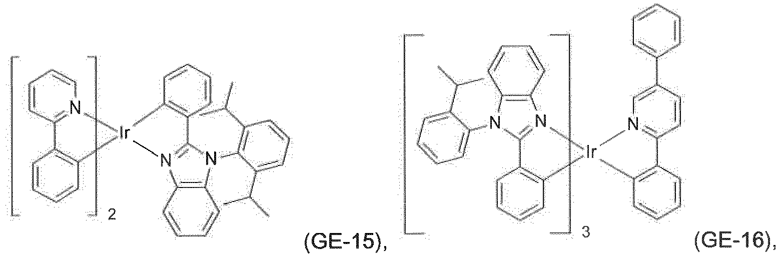

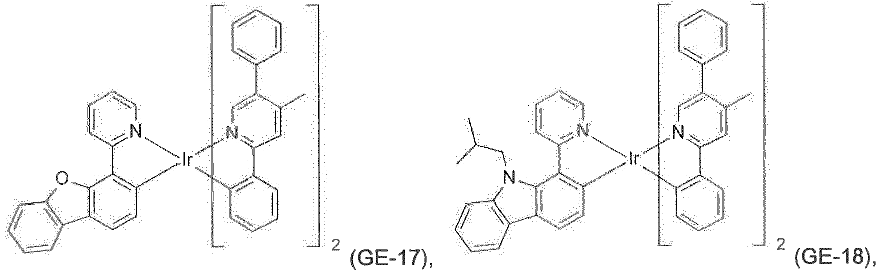

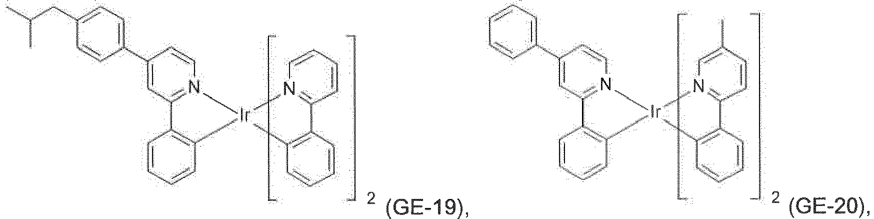

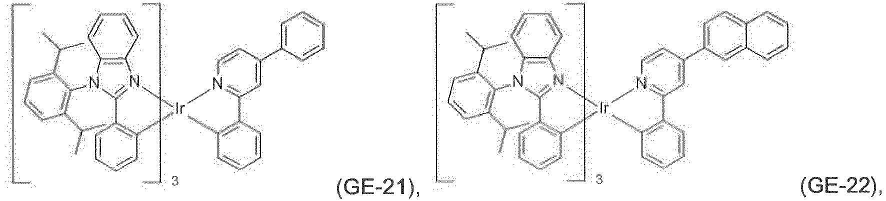

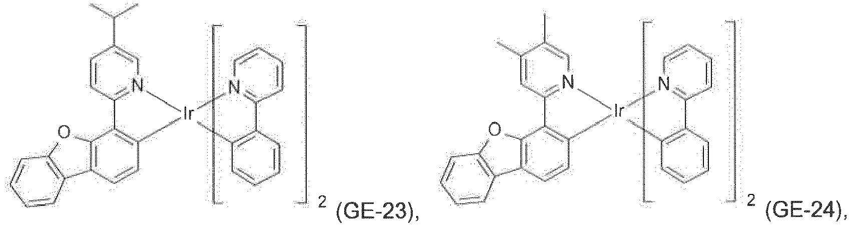

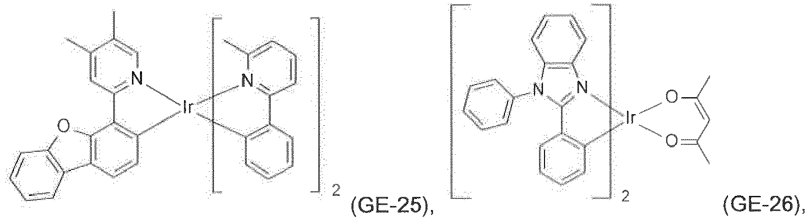

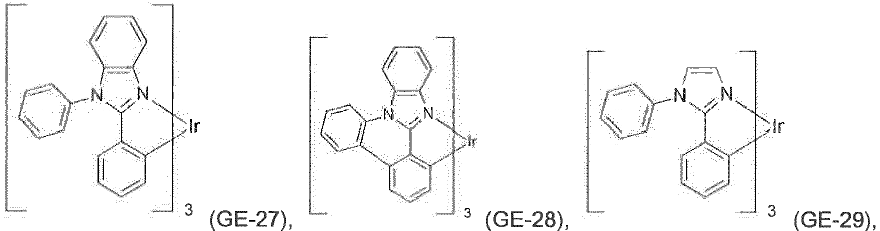

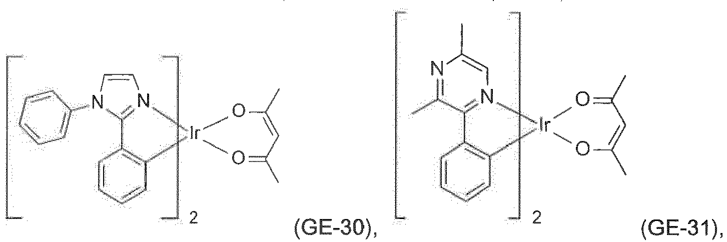

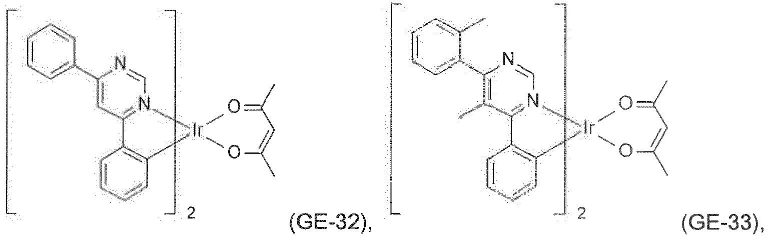

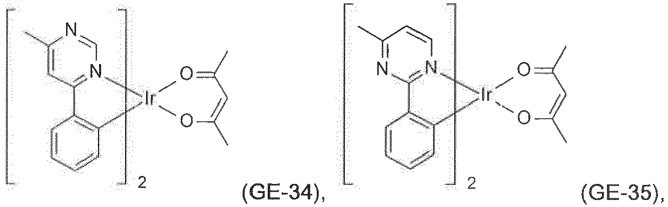

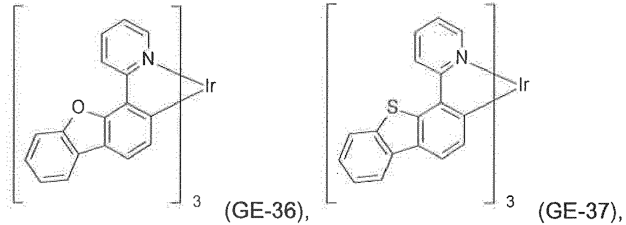

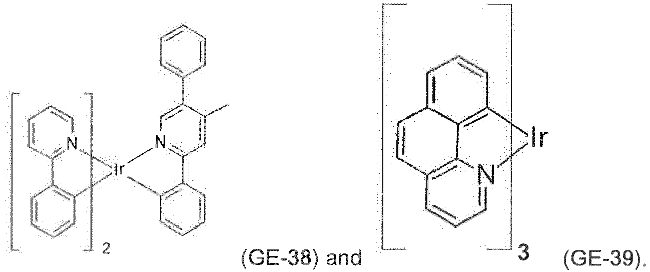

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In a preferred embodiment of the present invention, at least one compound of formula I, especially a compound of formula (**Ia-1**), (**Ia-2**), (**Ia-3**), (**Ia-4**), (**Ib-1**), (**Ib-2**),or (**Ib-3**), such as, for example, a compound (**A-1**) to (**A-60**), (**B-1**) to (**B-53**), (**C-1**) to (**C-26**), or (**D-1**) to (**D-23**), very especially a compound of formula (**Ia-1**), (**Ia-2**), (**Ia-3**), (**Ia-4**), (**Ib-1**), (**Ib-2**),or (**Ib-3**), wherein X is O, is used as matrix material.

In a preferred embodiment, the light-emitting layer is formed from 2 to 40% by weight, preferably 5 to 35% by weight, of at least one of the aforementioned emitter materials and 60 to 98% by weight, preferably 75 to 95% by weight, of at least one of the aforementioned, or belowmentioned matrix materials - in one embodiment at least one compound of the formula I - where the sum total of the emitter material and of the matrix material adds up to 100% by weight.

Suitable metal complexes for use together with the compounds of the formula I as matrix material in OLEDs are, for example, also carbene complexes as described in WO 2005/019373 A2, WO 2006/056418 A2, WO 2005/113704, WO 2007/115970, WO 2007/115981, WO 2008/000727 and WO2014147134.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709, WO2014/009317 (in particular compound A-24), WO2014/044722 and WO2014/072320 (in particular page 25 to 29 of WO2014/072320).

The above-mentioned small molecules are more preferred than the above-mentioned (co)polymers of the small molecules.

Further suitable host materials, are described in WO2011137072 (for example, 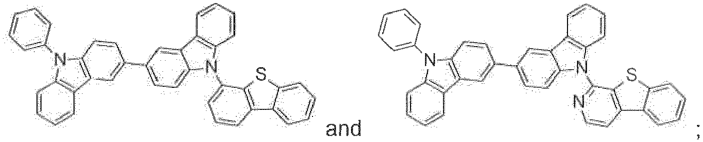 best results are achieved if said compounds are combined with 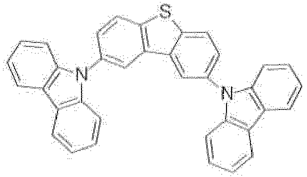 ); WO2012048266 (for example, 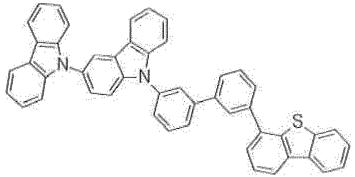 and 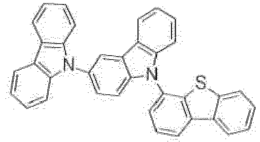 ); WO2012162325 (for example, 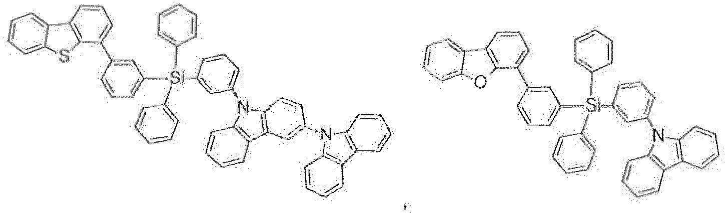 and 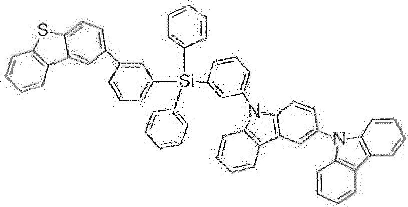 ); and EP2551932 (for example, 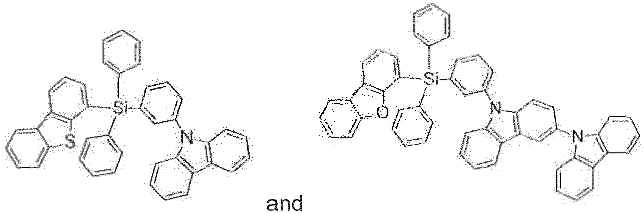 ).

In a particularly preferred embodiment, one or more compounds of the general formula (XII) specified hereinafter are used as second host material. 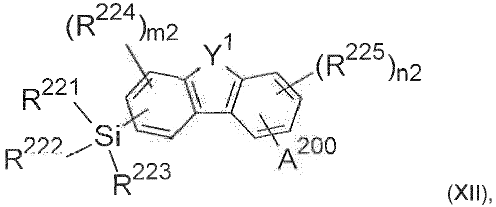 wherein
Y¹ is NR, S, O or PR;
R is aryl, heteroaryl, alkyl, cycloalkyl, or heterocycloalkyl;
A²⁰⁰ is -NR²⁰⁶R²⁰⁷, -P(O)R²⁰⁸R²⁰⁹, -PR²¹⁰R²¹¹, -S(O)₂R²¹², -S(O)R²¹³, -SR²¹⁴, or -OR²¹⁵; R²²¹, R²²² and R²²³ are independently of each other aryl, heteroaryl, alkyl, cycloalkyl, or heterocycloalkyl, wherein at least on of the groups R²²¹, R²²², or R²²³ is aryl, or heteroaryl; R²²⁴ and R²²⁵ are independently of each other alkyl, cycloalkyl, heterocycloalkyl, aryl, heteroaryl, a group A²⁰⁰, or a group having donor, or acceptor characteristics;
n2 and m2 are independently of each other 0, 1, 2, or 3;
R²⁰⁶ and R²⁰⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and R²⁰⁸, R²⁰⁹, R²¹⁰, R²¹¹, R²¹², R²¹³, R²¹⁴ und R²¹⁵ are independently of each other aryl, heteroaryl, alkyl, cycloalkyl, or heterocycloalkyl. Compounds of formula XII, such as, for example, 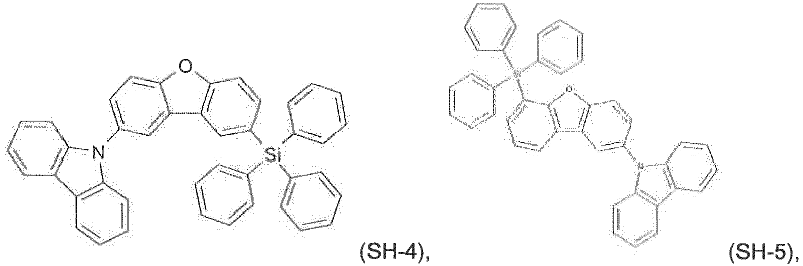 or 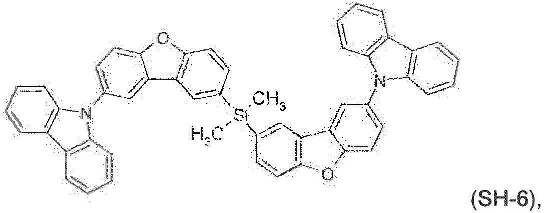 are described in WO2010079051 (in particular pages on 19 to 26 and in tables on pages 27 to 34, pages 35 to 37 and pages 42 to 43).

Additional host materials on basis of dibenzofurane are, for example, described in US2009066226, EP1885818B1, EP1970976, EP1998388 and EP2034538. Examples of particularly preferred host materials are shown below: 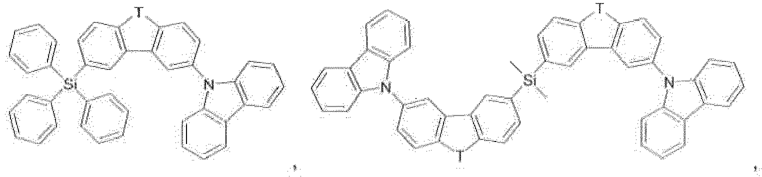 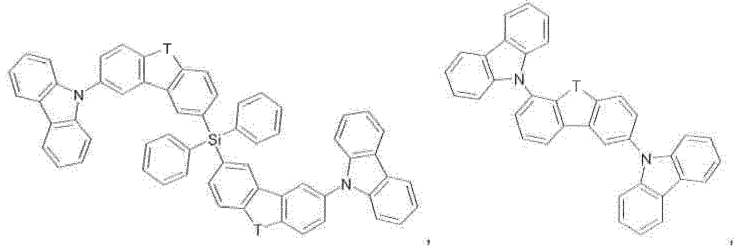 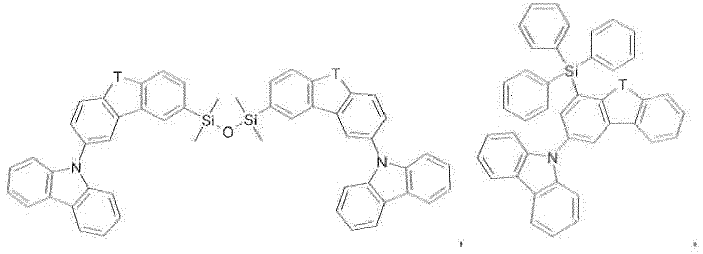 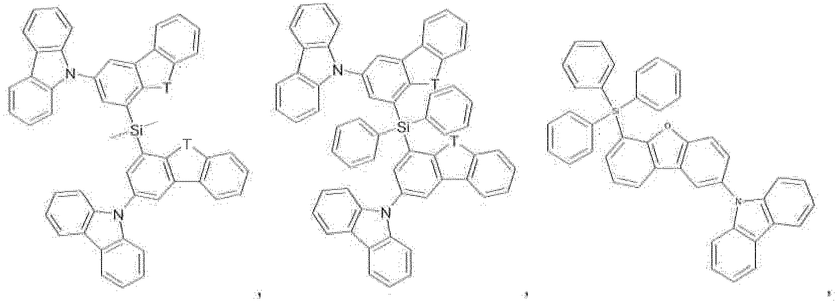 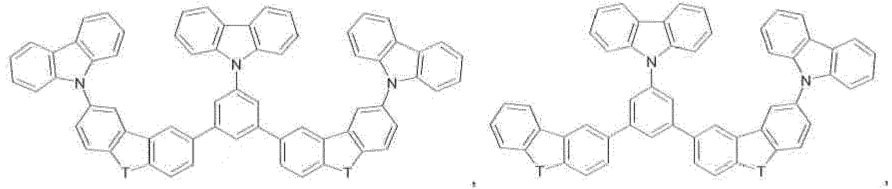 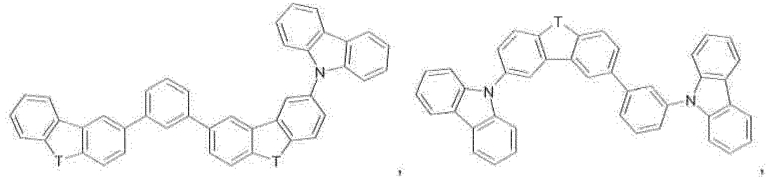 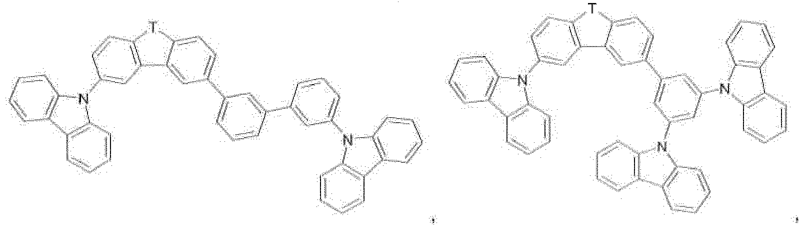 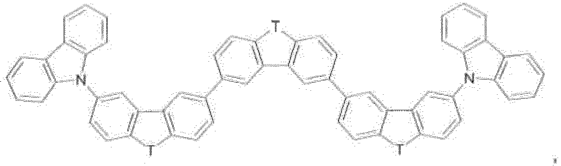 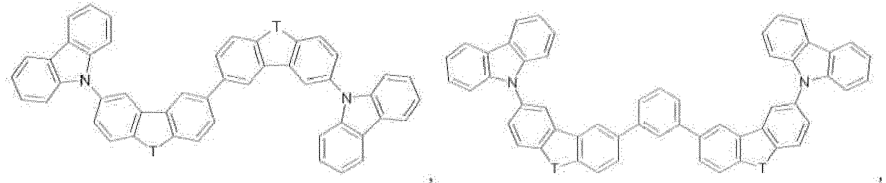 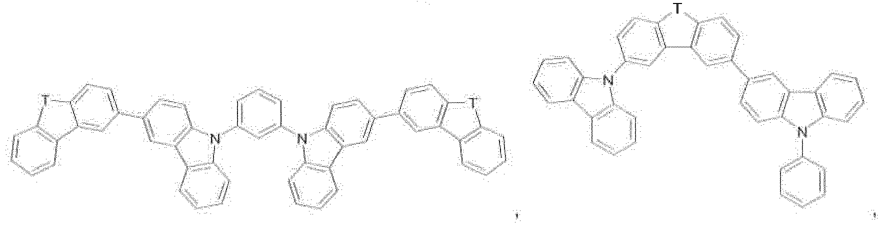 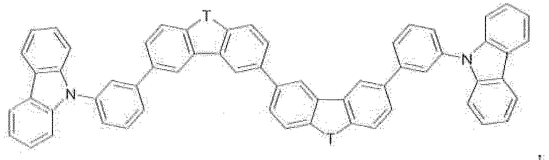 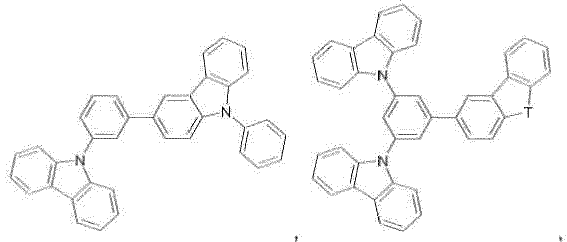 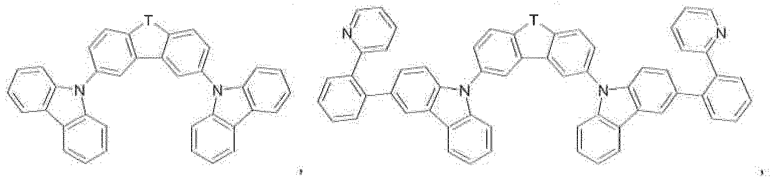 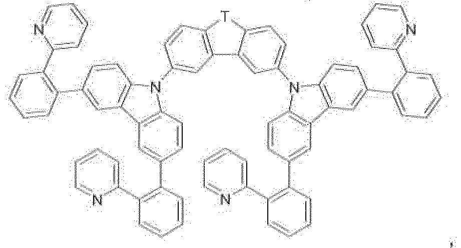 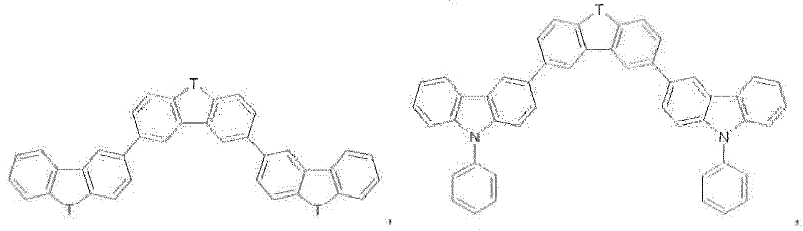 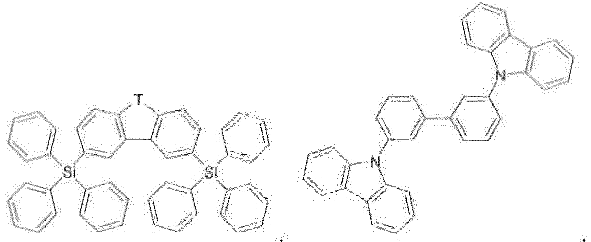 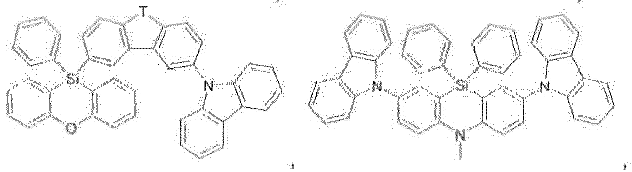 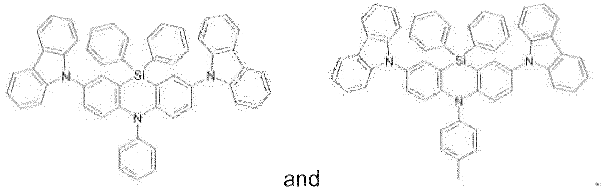 In the above-mentioned compounds T is O, or S, preferably O. If T occurs more than one time in a molecule, all groups T have the same meaning. Compounds (**SH-1**), 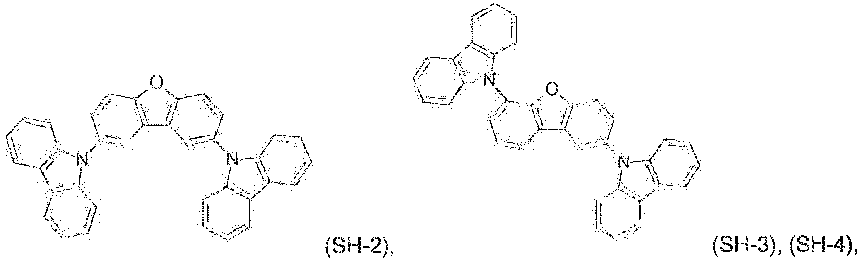 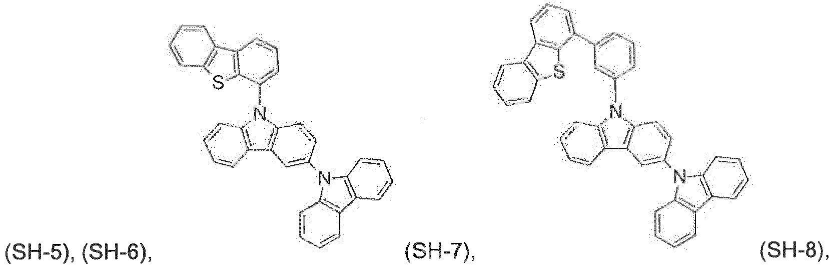 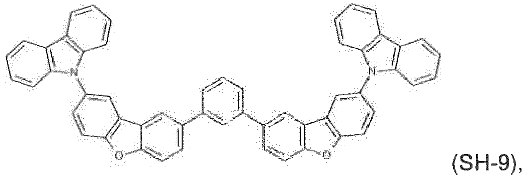 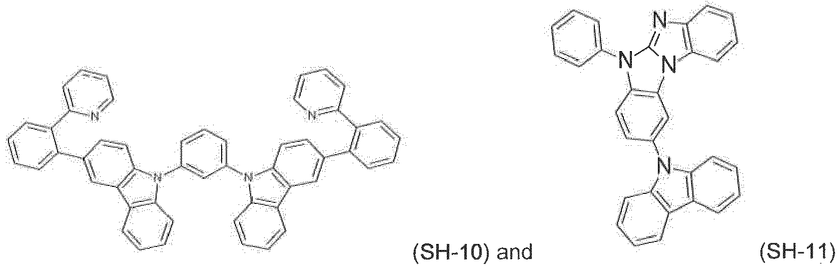 are most preferred.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

In a preferred embodiment of the present invention, at least one compound of the formula I, especially a compound of formula (**Ia-1**), (**Ia-2**), (**Ia-3**), (**Ia-4**), (**Ib-1**), (**Ib-2**),or (**Ib-3**), such as, for example, a compound (**A-1**) to (**A-60**), (**B-1**) to (**B-53**), (**C-1**) to (**C-26**), or (**D-1**) to (**D-23**), very especially a compound of formula (**Ia-1**), (**Ia-2**), (**Ia-3**), (**Ia-4**), (**Ib-1**), (**Ib-2**),or (**Ib-3**), wherein X is O, is used as hole/exciton blocking material.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine *S*,*S*-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH-1**), (**SH-2**), (**SH-3**), (**SH-4**), (**SH-5**), (**SH-6**), (**SH-7**), (**SH-8**), (**SH-9**) and (**SH-10**) may be used as hole/exciton blocking materials.

### Electron transport layer (g):

Electron transport layer may include a material capable of transporting electrons. Electron transport layer may be intrinsic (undoped), or doped. Doping may be used to enhance conductivity. Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

In a preferred embodiment of the present invention, at least one compound of the formula I, especially a compound of formula (**Ia-1**), (**Ia-2**), (**Ia-3**), (**Ia-4**), (**Ib-1**), (**Ib-2**), or (**Ib-3**), such as, for example, a compound (**A-1**) to (**A-60**), (**B-1**) to (**B-53**), (**C-1**) to (**C-26**), or (**D-1**) to (**D-23**), very especially a compound of formula (**Ia-1**), (**Ia-2**), (**Ia-3**), (**Ia-4**), (**Ib-1**), (**Ib-2**), or (**Ib-3**), wherein X is O, is used as electron transport material.

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**VIII**) below, preferably a compound of the formula (**VIIIaa**) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (**formula VII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**VII**) 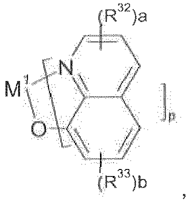 in which
R³² and R³³ are each independently F, C₁-C₈-alkyl, or C₆-C₁₄-aryl, which is optionally substituted by one or more C₁-C₈-alkyl groups, or
two R³² and/or R³³ substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**VII**) is 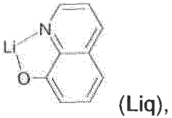 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**VIII**), 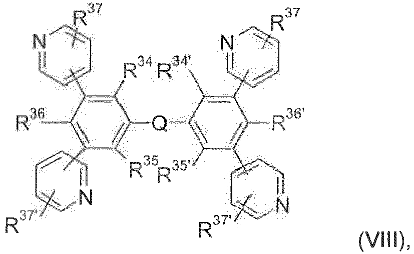 in which
R³⁴, R³⁵, R³⁶, R³⁷, R^{34'}, R^{35'}, R^{36'} and R^{37'}are each independently H, C₁-C₁₈-alkyl, C₁-C₁₈-alkyl which is substituted by E and/or interrupted by D, C₆-C₂₄-aryl, C₆-C₂₄-aryl which is substituted by G, C₂-C₂₀-heteroaryl or C₂-C₂₀-heteroaryl which is substituted by G,
Q is an arylene or heteroarylene group, each of which is optionally substituted by G;
D is -CO-; -COO-; -S-; -SO-; -SO₂-; -O-; -NR⁴⁰-; -SiR⁴⁵R⁴⁶-; -POR⁴⁷-; -CR³⁸=CR³⁹-; or -C≡C-; E is -OR⁴⁴; -SR⁴⁴; -NR⁴⁰R⁴¹; -COR⁴³; -COOR⁴²; -CONR⁴⁰R⁴¹; -CN; or F;
G is E, C₁-C₁₈-alkyl, C₁-C₁₈-alkyl which is interrupted by D , C₁-C₁₈-perfluoroalkyl, C₁-C₁₈-alkoxy, or C₁-C₁₈-alkoxy which is substituted by E and/or interrupted by D, in which R³⁸ and R³⁹ are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R⁴⁰ and R⁴¹ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or
R⁴⁰ and R⁴¹ together form a 6-membered ring;
R⁴² and R⁴³ are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁴ is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R⁴⁵ and R⁴⁶ are each independently C₁-C₁₈-alkyl, C₆-C₁₈-aryl or C₆-C₁₈-aryl which is substituted by C₁-C₁₈-alkyl,
R⁴⁷ is C₁-C₁₈-alkyl, C₆-C₁₈-aryl or C₆-C₁₈-aryl which is substituted by C₁-C₁₈-alkyl.

Preferred compounds of the formula (**VIII**) are compounds of the formula (**VIIIa**) 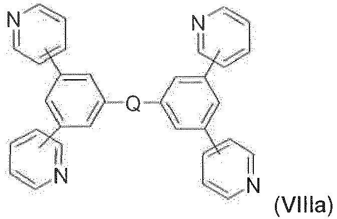 in which Q is: 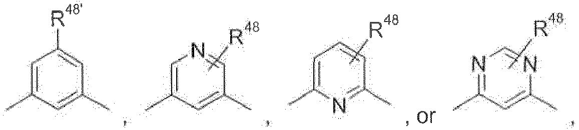 R⁴⁸ is H or C₁-C₁₈-alkyl and R^{48'} is H, C₁-C₁₈-alkyl or

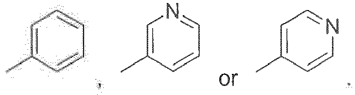

Particular preference is given to a compound of the formula

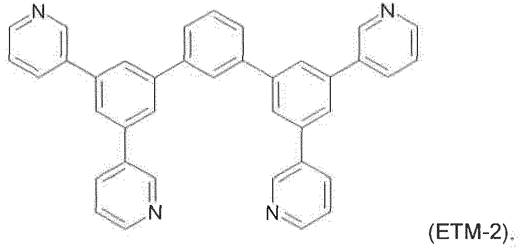

In a further, very particularly preferred embodiment, the electron-transport layer comprises a compound Liq and a compound **ETM-2.**

In a preferred embodiment, the electron-transport layer comprises the compound of the formula (VII) in an amount of 99 to 1% by weight, preferably 75 to 25% by weight, more preferably about 50% by weight, where the amount of the compounds of the formulae (VII) and the amount of the compounds of the formulae (VIII) adds up to a total of 100% by weight.

The preparation of the compounds of the formula (VIII) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in

WO2011/157790 are preferred, wherein dibenzofuran compound 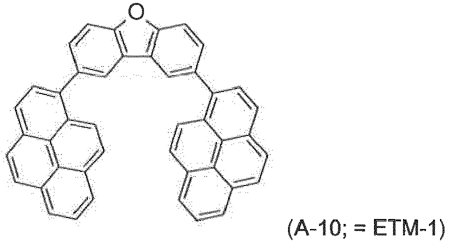 is most preferred.

In a preferred embodiment, the electron-transport layer comprises **Liq** in an amount of 99 to 1% by weight, preferably 75 to 25% by weight, more preferably about 50% by weight, where the amount of **Liq** and the amount of the dibenzofuran compound(s), especially **ETM-1**, adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 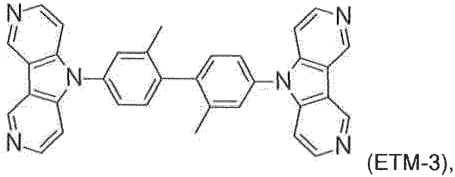 US2012/0261654 , such as, for example, a compound of formula 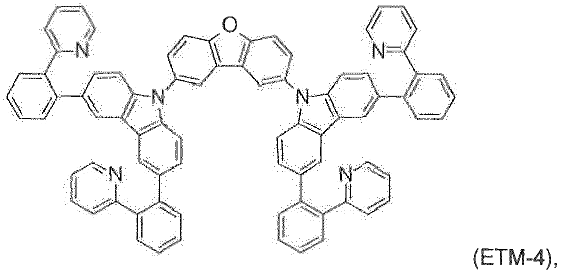 and WO2012/115034, such as for example, such as, for example, a compound of formula

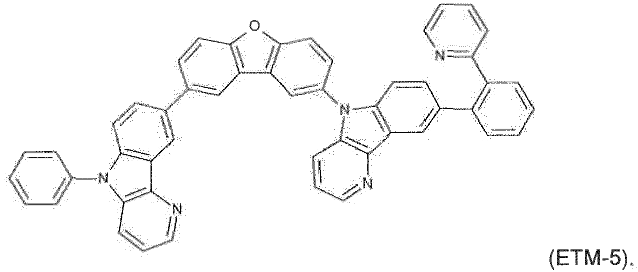

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 A;
hole injection layer (b): 50 to 1000 Å, preferably 200 to 800 Å,
hole-transport layer (c): 50 to 1000 Å, preferably 100 to 800 Å,
electron/exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 A,
electron injection layer (h): 10 to 500 Å, preferably 20 to 100 A,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula I in at least one layer of the OLED, preferably in the light-emitting layer (preferably as a matrix material), charge transport layer and/or in the charge/exciton blocking layer makes it possible to obtain OLEDs with high efficiency and with low use and operating voltage. Frequently, the OLEDs obtained by the use of the compounds of the formula **I** additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Example 1

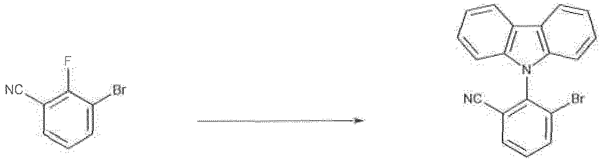

a) 22 g (110.0 mmol) 3-bromo-4-fluoro-benzonitrile, 19.3 g (115.5 mmol) 9H-carbazole and 140.1 g (660 mmol) K₃PO₄ are added to 150 ml DMF and the suspension is heated to 60°C for 3 h. The reaction mixture is slowly added to 2 l H₂O and stirred for 30 min. The suspension is filtered, washed three times with H₂O (500 ml each) and the residue is dried at 80°C / 125 mbar overnight to yield 38.4 g of crude product. The white solid is added to 75 ml 1-Methoxy-2-propanol, heated o reflux to form a clear solution that is cooled to RT and then to 0°C. The suspension is filtered; the residue is washed twice with cold 1-Methoxy-2-propanol (10 ml each) and dried at 80°C / 125 mbar overnight to yield 32.8 g (85.9% of theory) 3-bromo-2-carbazol-9-yl-benzonitrile as white solid.
   ¹H-NMR (400 MHz, CDCl₃): δ 8.18 (d, J = 8.0 Hz, 2H), 8.07 (dxd, J₁ = 8.0 Hz, J₂ = 1.6 Hz, 1H), 7.87 (dxd, J₁ = 8.0 Hz, J₂ = 1.2 Hz, 1H), 7.51 (t, J = 8.0 Hz, 1H), 7.47 - 7.34 (m, 4H), 7.01 (d, J = 8.0 Hz, 2H). 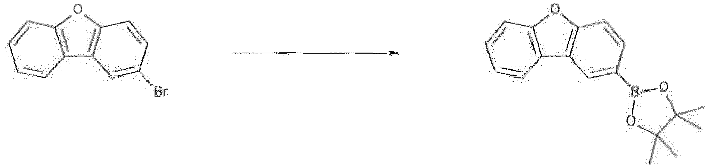
b) 40 g (160.3 mmol) 2-bromo-dibenzofuran are dissolved in 250 ml THF abs. and cooled to -78°C. 65 ml (175.5 mmol) BuLi (2.7M in hexane) are added within 55 min while keeping the internal temperature below -73°C. The resulting suspension is stirred for 30 min followed by the addition of 33.5 g (176.3 mmol) 2-isopropoxy-4,4,5,5-tetramethyl-1,3,2-dioxaborolane within 30 min while keeping the internal temperature below -73°C. The reaction mixture is then slowly brought to RT, then 200 ml of buffer solution pH = 7 are added and the pH brought to 7 by the addition of HCI 4M. The organic solvent is then evaporated on the rotavap; the aqueous phase is extracted three times with EtOAc (250 ml each). The combined organic phases are washed three times with H₂O (200 ml each), dried over Na₂SO₄, filtered and evaporated. The crude product (51.7 g) is purified by CombiFlash chromatography (solvent: Heptane / CH₂Cl₂ (0 to 40%)) to yield 34.1g of a colorless oil. 100 ml MeOH are added, the mixtue is heated to reflux, cooled to RT and then to 0°C. The suspension is filtered and the residue is dried at 30°C / 125 mbar overnight to yield 28.3 g (60.0% of theory) 2-dibenzofuran-2-yl-4,4,5,5-tetramethyl-1,3,2-dioxaborolane as a white solid.
   ¹H-NMR (300 MHz, CDCl₃): 8.45 (s (1H), 7.99 - 7.92 (m. 2H), 7.59 - 7.55 (m, 2H), 7.48 (t, J = 7.2 Hz, 1H), 7.37 (t, J = 7.8 Hz, 1H), 1.40 (s, 12H). 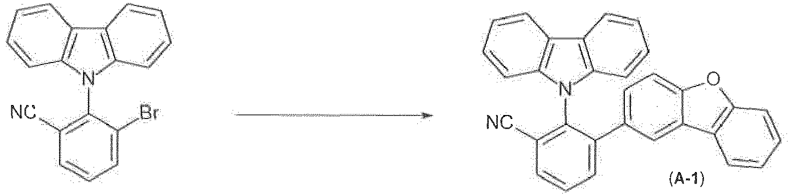
c) 15 g (43.2 mmol) 3-bromo-2-carbazol-9-yl-benzonitrile and 15.3 g (51.8 mmol) 2-dibenzofuran-2-yl-4,4,5,5-tetramethyl-1,3,2-dioxaborolane are dissolved in 200 ml THF and the solution is evacuated and purged three times with argon. 0.77 g (2.6 mmol) P(t-Bu)₃·HBF₄ and 1.22 g (1.3 mmol) Pd₂(dba)₃ are added and the solution is evacuated and purged three times with argon and heated to 50°C. A solution of 27.5 g (129.6 mmol) K₃PO₄ in 80 ml H₂O (evacuated and purged three times with argon) is added with a syringe and the reaction mixture is heated to reflux for 90 min. The THF is evaporated on the rotavap and to the resulting suspension are added 200 ml EtOAc and stirred for 30 min. The suspension is filtered and the residue is washed twice with EtOAc (10 ml each) and dried at 80°C / 125 mbar overnight to yield 18.0 g of crude product. The product is added to 70 ml EtOAc, heated to reflux for 1h, cooled to RT, washed twice with cold EtOAc (10 ml each) and dried at 80°C /125 mbar overnight to yield 14.7 g (78.3% of theory) 2-carbazol-9-yl-3-dibenzofuran-2-yl-benzonitrile as a white solid.
   ¹H-NMR (300 MHz, CDCl₃): δ 8.00 (d, J = 10.0 Hz, 1H), 7.96 (dxd, J₁ = 7.6 Hz, J₂ = 1.2 Hz, 1H), 7.92 (dxd, J₁ = 7.6 Hz, J₂ = 1.2 Hz, 1H), 7.73 (t, J = 7.6 Hz, 1H), 7.52 - 7.49 (m, 2H), 7.45 - 7.11 (m, 8H), 7.05 (d, J = 7.6 Hz, 2H), 7.00 (dxd, J₁ = 8.4 Hz, J₂ = 1.6 Hz, 1H).

### Example 2

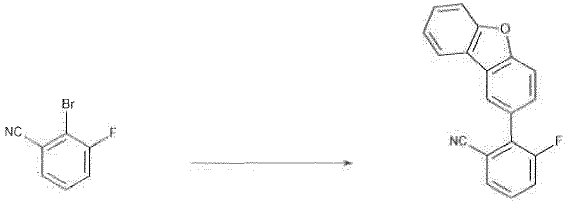

a) 8.2 g (41 mmol) 2-bromo-3-fluoro-benzonitrile and 13.3 g (45.2 mmol) 2-dibenzofuran-2-yl-4,4,5,5-tetramethyl-1,3,2-dioxaborolane are dissolved in 200 ml THF and the solution is evacuated and purged three times with argon. 0.73 g (2.5 mmol) P(t-Bu)₃·HBF₄ and 1.16 g (1.2 mmol) Pd₂(dba)₃ are added and the solution is evacuated and purged three times with argon and heated to 50°C. A solution of 26.1 g (123.0 mmol) K₃PO₄ in 80 ml H₂O (evacuated and purged three times with argon) is added with a syringe and the reaction mixture is heated to reflux for 90 min. The THF is evaporated on the rotavap and to the resulting suspension are added 200 ml EtOAc. The organic phase is washed three times with H₂O (50 ml each), dried over Na₂SO₄, filtered and evaporated to yield 26.8 g crude product. To the crude product are added 135 ml 2-propanol and the suspension is heated to reflux. 40 ml MEK are added to form a clear yellow solution that is cooled to RT and then to 0°C. The resulting suspension is filtered, the solid is washed twice with cold 2-propanol (10 ml each) and dried at 80°C / 125 mbar overnight to yield 10.3 g (87.3% of theory) 2-dibenzofuran-2-yl-3-fluoro-benzonitrile as a white solid.
   ¹H-NMR (400 MHz, CDCl₃): δ 8.09 (br s, 1H), 7.98 (d, J = 6.8 Hz, 1H), 7.71 (d, J = 8.4 Hz, 1H), 7.63 - 7.56 (m, 3H), 7.52 - 7.35 (m, 4H). 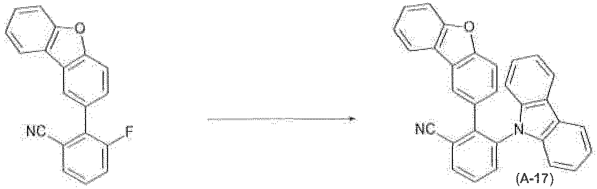
b) 9.73 g (33.7 mmol) 2-dibenzofuran-2-yl-3-fluoro-benzonitrile, 5.98 g (35.4 mmol) 9H-carbazol and 42.9 g (202.2 mmol) K₃PO₄ are added to 100 ml DMF and the suspension is heated to 120°C for 5 h. The reaction mixture is cooled to RT, filtered and the solvent is evaporated on the rotavap. The residue is dissolved in 200 ml THF, dried over Na₂SO₄, filtered and the solvent is evaporated on the rotavap to yield 12.8 g crude product. The crude product is decocted twice for 1h in EtOAc (80 ml each), cooled to RT, filtered and dried at 80°C / 125 mbar to yield 6.65 g (45.4% of theory) 3-carbazol-9-yl-2-dibenzofuran-2-yl-benzonitrile as a white solid.
   ¹H-NMR (300 MHz, CDCl₃): δ 8.01 - 7.94 (m, 3H), 7.79 (dxd, J₁ = 8.0 Hz, J₂ = 1.2 Hz 1H), 7.73 (t, J = 1.2 Hz, 1H), 7.67 (t, J = 8.0 Hz, 1H), 7.59 (d, J = 8.0 Hz, 1H), 7.44 - 7.14 (m, 9H), 7.08 (d, J = 8.0 Hz, 2H).

### Example 3

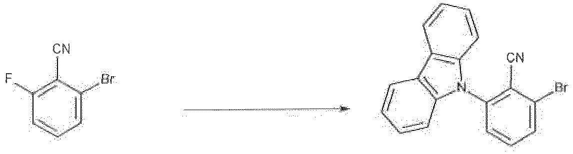

a) 25 g (121.2 mmol) 2-bromo-6-fluoro-benzonitrile, 21.5 g (127.3 mmol) 9H-carbazole and 154.4 g (727.5 mmol) K₃PO₄ are added to 250 ml DMF and the suspension is heated to 120°C for 1 h. The reaction mixture filtered hot and the filtrate is evaporated on the rotavap. The residue is dissolved in 500 ml THF, dried over Na₂SO₄, filtered and the solvent is evaporated on the rotavap to yield 50.0 g crude product. Crystallization from 500 ml 1-Methoxy-2-propanol yields 37.9 g (90.2% of theory) 2-bromo-6-carbazol-9-yl-benzonitrile as a white solid.
   ¹H-NMR (400 MHz, CDCl₃): δ 8.15 (d, J = 8.0 Hz, 2H), 7.84 (dxd, J₁ = 8.0 Hz, J₂ = 0.8 Hz, 1H), 7.65 (t, J = 8.0 Hz, 1H), 7.57 (dxd, J₁ = 8.0 Hz, J₂ = 0.8 Hz, 1H), 7.47 - 7.42 (m, 2H), 7.37 - 7.32 (m, 2H), 7.21 (d, J = 8.0 Hz, 2H). 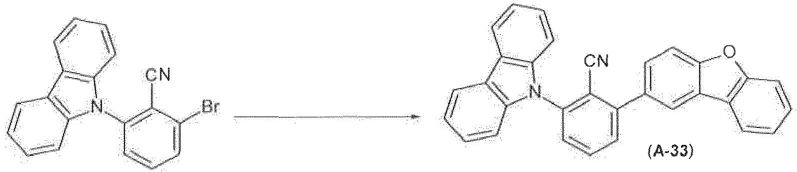
b) 8.0 g (23.0 mmol) 2-bromo-6-carbazol-9-yl-benzonitrile and 8.2 g (27.6 mmol) 2-dibenzofuran-2-yl-4,4,5,5-tetramethyl-1,3,2-dioxaborolane are dissolved in 180 ml Toluene. 26 ml H₂O, 42 ml EtOH and 7.33 g (69.1 mmol) Na₂CO₃ are added and the suspension is evacuated and purged three times with argon. 1.37 g (1.15 mmol) Tetrakis-triphenylphosphin palladium are added and the suspension is evacuated and purged three times with argon and heated to reflux for 2 h. The reaction mixture is cooled to RT and the organic solvents evaporated on the rotavap. The resulting suspension in H₂O is extracted three times with CH₂Cl₂ (200 ml each) and the combined organic phases are washed three times with H₂O (100 ml each), then dried over Na₂SO₄ and evaporated on the rotavap to yield 12.9 g crude product. The crude product is first decocted in 70 ml MeOH and then 50 ml EtOAc to yield after drying at 80°C /125 mbar overnight 8.68 g (86.9% of theory) 2-carbazol-9-yl-6-dibenzofuran-2-yl-benzonitrile as a white solid.
   ¹H-NMR (300 MHz, CDCl₃): δ 8.26 (d, J = 1.2 Hz, 1H), 8.17 (d, J = 8.0 Hz, 2H), 8.03 (d, J = 8.0 Hz, 1H), 7.88 (t, J = 8.0 Hz, 1H), 7.79 - 7.71 (m, 3H), 7.64 - 7.60 (m, 2H), 7.54 - 7.45 (m, 3H), 7.42 - 7.30 (m, 5H).

### Example 4

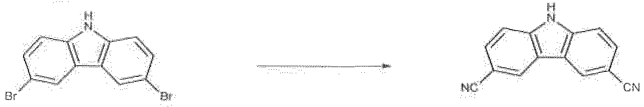

a) 200 g (0.615 mol) 3,6-dibromocarbazole and 137.8 g (1.54 mol) CuCN are added to 1.6 l DMF and the mixture is stirred at reflux for 24 h. Then the mixture is cooled to room temperature and 800 ml NH₄OH solution (25 %) are added and stirred for 2 h. Then the mixture is filtered and the solid is suspended in 1.5 l of ethylendiamine solution (20% in water). This mixture is stirred for 48 h. The solid is filtered and washed with water several times. After drying, 135 g of solid are recovered. The solid is purified by recrystallization from DMF. 74 g (55 % of theory) 9H-carbazole-3,6-dicarbonitrile as off-white solid are obtained.
   ¹H-NMR (400 MHz, CDCl₃): δ 12.35 (br s, 1H), 8.78 (s, 2H), 7.84 (dxd, J₁= 8.4 Hz, J₂ = 1.4 Hz, 2H), 7.71 (d, J = 8.4 Hz, 2H). 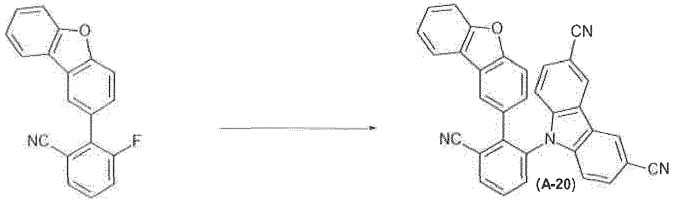
b) 9.0 g (31.3 mmol) 2-dibenzofuran-2-yl-3-fluoro-benzonitrile, 6.8 g (31.3 mmol) 9H-carbazole-3,6-dicarbonitrile and 39.9 g (188.0 mmol) K₃PO₄ are added to 200 ml DMF and the suspension is heated to 120°C for 24 h. The solvent is evaporated on the rotavap. The residue is decocted in 250 ml MeOH twice for 2 h, filtered hot and dried at 80°C / 125 mbar overnight to yield 12.5 g crude product. To the solid are added 500 ml THF and the suspension is heated to reflux, then 30 g silica are added, stirred for 1h, filtered hot and evaporated to yield 12.3 g product that is decocted twice for 3 h in 1-Methoxy-2-propanol (250 ml each), filtered hot and dried at 80°C / 125 mbar overnight to yield 4.2 g (27.7% of theory) 9-(3-cyano-2-dibenzofuran-2-yl-phenyl)carbazole-3,6-dicarbonitrile as off-white solid.
   ¹H-NMR (400 MHz, CDCl₃): δ 8.26 (s, 2H), 8.12 -8.08 (m, 1H), 7.81- 7.92 (m, 2H), 7.66 (d, J = 1.6 Hz, 1H), 7.64 (d, J = 1.6 Hz, 1H), 7.61 (d, J = 1.6 Hz, 1H), 7.56 (d, J = 8.0 Hz, 1H), 7.47 - 7.39 (m, 2H), 7.30 - 7.18 (m, 4H), 7.06 (dxd, J₁ = 8.0 Hz, J₂ = 2.0 Hz, 1H).

### Example 5

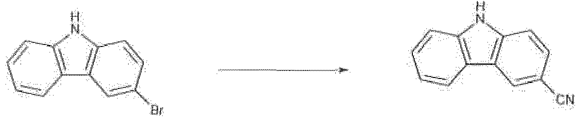

a) 100 g (0.406 mol) 3-bromo-9H-carbazole and 58.2 g (0.650 mol) CuCN are added to 800 ml of DMF and the suspension is heated to reflux for 20 h. The resulting solution is cooled to room temperature and then poured on 4 l H₂O. The suspension is filtered and the residue is washed three times with H₂O (1 I each). The white solid is suspended in 1.5 l of 10% ammonia in water, stirred for three hours, filtered, washed three times with water (1 I each) and dried at 80°C / 125 mbar overnight. The resulting solid is dissolved in 1 l of boiling THF; 100 g of silica are added, stirred for 1 h and then filtered hot. The filtrate is evaporated to 400 ml, cooled to room temperature, filtered, washed three times with cold THF (50 ml each) and dried at 80°C / 125 mbar overnight to yield 43.3 g (55.6% of theory) 9H-carbazole-3-carbonitrile as a white solid.
   ¹H-NMR (400 MHz, CDCl₃): δ 8.39 (br s, 2H), 8.10 (d, J = 8.0 Hz, 1H), 7.67 (dxd, J₁ = 8.4 Hz, J₂ = 1.6 Hz, 1H), 7.54 - 7.47 (m, 3H), 7.35 - 7.31 (m, 1H). 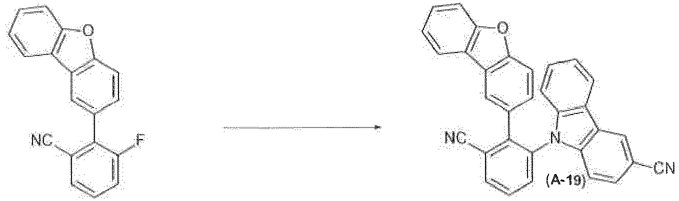
b) 9.0 g (31.3 mmol) 2-dibenzofuran-2-yl-3-fluoro-benzonitrile, 6.0 g (31.3 mmol) 9H-carbazole-3-carbonitrile and 49.2 g (232.0 mmol) K₃PO₄ are added to 200 ml DMF and the suspension is heated to 120°C for 19 h. The reaction mixture is filtered hot through Hyflo and the filtrate is evaporated on the rotavap. The residue is dissolved in 500 ml CH₂Cl₂, extracted three times with H₂O (250 ml each), dried over Na₂SO₄, filtered and evaporated on the rotavap to yield 25.3 g of crude product as a brown solid. The residue is decocted in 300 ml MeOH for 2 h, cooled to RT, filtered and dried at 80°C / 125 mbar to yield 11.9 of product that is crystallized from 70 ml 1-Methoxy-2-propanol to yield 10.1 g (70.2% of theory) 9-(3-cyano-2-dibenzofuran-2-yl-phenyl)carbazole-3-carbonitrile as white solid.
   ¹H-NMR (300 MHz, CDCl₃): δ 8.23 (s, 1H), 8.06 (dxd, J₁ = 7.2 Hz, J₂ = 1.8 Hz, 1H), 7.97 (d, J = 7.0 Hz, 1H), 7.82 - 7.27 (m, 2H), 7.63 (br s, 1H), 7.56 - 7.36 (m, 5H), 7.31 - 7.06 (m, 7H).

### Example 6

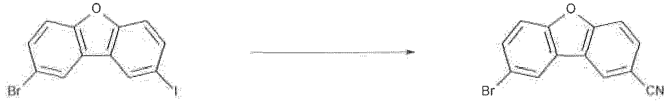

a) To 350 ml DMF are added 50 g (0.134 mol) 2-bromo-8-iodo-dibenzofuran and 12.0 g (0.134 mol) CuCN and the suspension is heated to 100°C for 17 h. The reaction mixture is filtered hot and the filtrate is added slowly to 3 l H₂O. The white suspension is stirred for 15 min, filtered and the residue is washed twice with H₂O (500 ml each) and then dried at 80°C / 125 mbar overnight to yield 39.3 g of an off white solid. To the crude product are added 600 ml THF and the suspension is heated to reflux, 60 g silica are added and the heating is continued for 30 min. The suspension is filtered hot and the residue is washed twice with THF (100 ml each). The solvent is evaporated on the rotavap to 1/0 g of a white suspension that is cooled to 0°C. Filtration, washing twice with cold THF (10 ml each) and drying at 80°C / 125 mbar yielded 27.1 g of a white solid that contains 79.4% of the desired product according to GC along with 9.0% of starting material, 2.0% of the iodo isomer and 4.7% of the dinitrile derivative. CombiFlash chromatography (solvent (Heptane / CH₂Cl₂ = 4:6) yields 17.5 g (47.7% of theory) of a mixture of 88.6% 8-bromodibenzofuran-2-carbonitrile, 7.2% 8-iododibenzofuran-2-carbonitrile and 2.6% dibenzofuran-2,8-dicarbonitrile as a white solid.
   ¹H-NMR (400 MHz, CDCl₃): δ 8.22 (s, 1H), 8.08 (d, J = 2.0 Hz, 1H), 7.77 (dxd, J₁ = 8.4 Hz, J₂ = 1.6 Hz, 1H), 7.66 - 7.63 (m. 2H), 7.49 (d, J = 8.8 Hz, 1H). 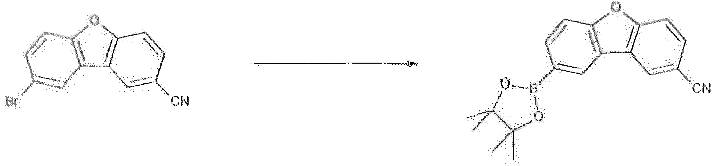
b) 13.9 g (51.3 mmol) of the mixture from step a), 15.7 g (61.6 mmol) 4,4,5,5-tetramethyl-2-(4,4,5,5-tetramethyl-1,3,2-dioxaborolan-2-yl)-1,3,2-dioxaborolane and 30.2 g (0.308 mol) KOAc are added to 350 ml DMF. The white suspension is evacuated and purged with argon three times, then 2.1 g (2.57 mmol) [1,1'-Bis(diphenylphosphino)ferrocen] PdCl₂ are added and the orange suspension is evacuated and purged with argon three times and then heated to 65°C for 50 h. The black reaction mixture is filtered hot through Hyflo and the residue is washed twice with DMF (50 ml each). The filtrate is concentrated to 200 g solution and then added slowly to 1.8 l H₂O. The suspension is stirred for 2 h and filtered. The residue is washed three times with H₂O (250 ml each) and dried at 80°C / 125 mbar overnight to yield 17.6 g crude product as a brown solid. The solid is dissolved in 300 ml CH₂Cl₂, filtered through a plug of silica and the silica is washed three times with CH₂Cl₂ (300 ml each). The solution is evaporated to yield 12.9 g of a slightly yellow solid. The crude product is heated with 500ml Heptane to reflux for 30 min. 5 g char coal are added to the suspension and refluxed for another 15 min. Filtration through Hyflo gives a colorless solution. 400 ml of the solvent are distilled off and then the solution is slowly cooled to RT and then to 0°C. The suspension is filtered, the residue is washed twice with cold Heptane (20 ml each) and then dried at 80°C / 125 mbar overnight to yield 9.7 g (59.3% of theory) 8-(4,4,5,5-tetramethyl-1,3,2-dioxaborolan-2-yl)dibenzofuran-2-carbonitrile as a white solid.
   ¹H-NMR (400 MHz, CDCl₃): δ 8.46 (s, 1H), 8.26 (d, J = 2.4 Hz, 1H), 8.00 (dxd, J₁ = 8.4 Hz, J₂ = 1.2 Hz, 1H), 7.74 (dxd, J₁ = 8.4 Hz, J₂ = 1.6 Hz, 1H), 7.64 (d, J = 8.4 Hz, 1H), 7.60 (d, J = 8.4 Hz, 1H), 1.40 (s, 12H). 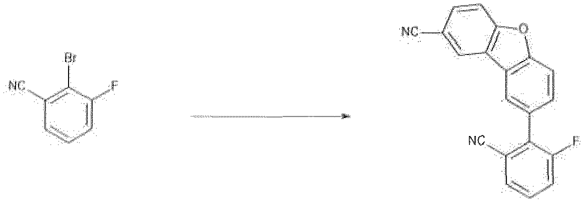
c) 15.0 g (74.2 mmol) 2-bromo-3-fluoro-benzonitrile, 26.2 g (82.0 mmol) 8-(4,4,5,5-tetramethyl-1,3,2-dioxaborolan-2-yl)dibenzofuran-2-carbonitrile are dissolved in 300 ml THF and the solution is evacuated and purged three times with argon. 1.32 g (4.5 mmol) P(t-Bu)₃·HBF₄ and 2.1 g (2.2 mmol) Pd₂(dba)₃ are added and the solution is evacuated and purged three times with argon and heated to 50°C. A solution of 47.3 g (222.7 mmol) K₃PO₄ in 120 ml H₂O (evacuated and purged three times with argon) is added with a syringe and the reaction mixture is heated to reflux for 45 min. The THF is evaporated on the rotavap and to the resulting suspension are added 200 ml EtOAc. The organic phase is washed three times with H₂O (50 ml each), dried over Na₂SO₄, filtered and evaporated to yield 33.4 g crude product. To the crude product are added 170 ml 2-propanol and the suspension is heated to reflux. 340 ml MEK are added to form a slightly turbid yellow solution that is filtered hot. The filtrate is heated to reflux again, cooled to RT and then to 0°C. The resulting suspension is filtered, the solid is washed twice with cold 2-propanol (10 ml each) and dried at 80°C / 125 mbar overnight to yield 18.2 g (78.5% of theory) 8-(2-cyano-6-fluoro-phenyl)dibenzofuran-2-carbonitrile as a white solid.
   ¹H-NMR (400 MHz, CDCl₃): δ 8.31 (s, 1H), 8.11 (s, 1H), 7.80 - 7.75 (m, 2H), 7.71 - 7.63 (m, 3H), 7.54 - 7.45 (m, 2H). 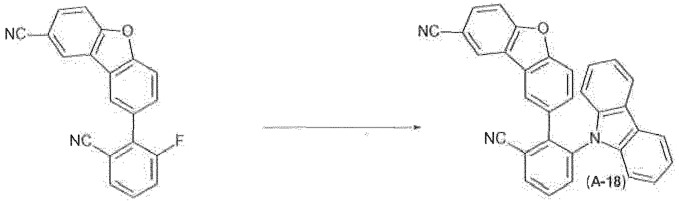
d) 5.0 g (16.0 mmol) 8-(2-cyano-6-fluoro-phenyl)dibenzofuran-2-carbonitrile, 2.81 g (16.8 mmol) 9H-carbazol and 20.4 g (96.1 mmol) K₃PO₄ are added to 150 ml DMF and the suspension is heated to reflux for 16 h. The reaction mixture is filtered hot through Hyflo and the solvent is evaporated on the rotavap. To the residue are added 150 ml MeOH, the mixture is heated to reflux and then cooled to RT. The suspension is filtered, the residue is washed twice with MeOH (10 ml each) and dried at 80°C / 125 mbar overnight to yield 5.7 g crude product. The solid is suspended in a mixture of 30 ml 2-propanol and 50 ml 1-methoxy-2-propanol and heated to reflux for 2h. The suspension is cooled to RT, filtered, washed twice with 2-propanol (10 ml each) and dried at 80°C / 125 mbar overnight to yield 4.2 g (53.3% of theory) 8-(2-carbazol-9-yl-6-cyano-phenyl)dibenzofuran-2-carbonitril as a white solid.
   ¹H-NMR (400 MHz, CDCl₃): δ 8.02 (dxd, J₁ = 7.6 Hz, J₂ = 1.6 Hz, 1H), 7.95 (d, J = 8.0 Hz, 2H), 7.84 - 7.81 (m, 2H), 7.74 (t, J = 8.0 Hz, 1H), 7.69 (d, J = 2.0 Hz, 1H), 7.59 (dxd, J₁ = 8.4 Hz, J₂ = 1.6 Hz, 1H), 7.44 (d, J = 8.4 Hz, 1H), 7.34 - 7.15 (m, 6H), 7.06 (d, J = 8.0 Hz, 2H).

### Example 7

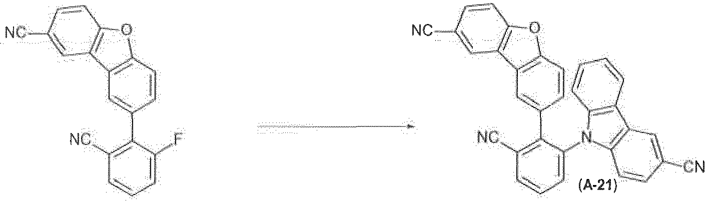

5.0 g (16.0 mmol) 8-(2-cyano-6-fluoro-phenyl)dibenzofuran-2-carbonitrile, 3.23 g (16.8 mmol) 9H-carbazole-3-carbonitrile and 20.4 g (96.1 mmol) K₃PO₄ are added to 150 ml DMF and the suspension is heated to reflux for 16 h. The reaction mixture is filtered hot through Hyflo and the solvent is evaporated on the rotavap. To the residue are added 150 ml MeOH, the mixture is heated to reflux and then cooled to RT. The suspension is filtered, the residue is washed twice with cold MeOH (10 ml each) and dried at 80°C / 125 mbar overnight to yield 6.4 g crude product. The solid is recrystallized twice from 1-methoxy-2-propanol and dried at 80°C / 125 mbar overnight to yield 3.4 g (43.8% of theory) 9-[3-cyano-2-(8-cyanodibenzofuran-2-yl)phenyl]carbazole-3-carbonitrile as a white solid.
¹H-NMR (400 MHz, DMSO-d₆): δ 8.60 (s, 1H), 8.39 (s, 1H), 8.30 (dxd, J₁ = 7.6 Hz, J₂ = 1.2 Hz, 1H), 8.15 (d, J = 1.2 Hz, 1H), 8.11 (d, J = 8.0 Hz, 1H), 7.99 (dxd, J₁ = 8.0 Hz, J₂ = 1.2 Hz, 1H), 7.93 - 7.88 (m 2H), 7.79 (d, J = 8.8 Hz, 1H), 7.66 (br s, 1H), 7.44 (d, J = 8.8 Hz, 1H), 7.43 - 7.23 (br m, 4H), 7.26 (dxd, J₁ = 8.8 Hz, J₂ = 2.0 Hz, 1H).

### Example 8

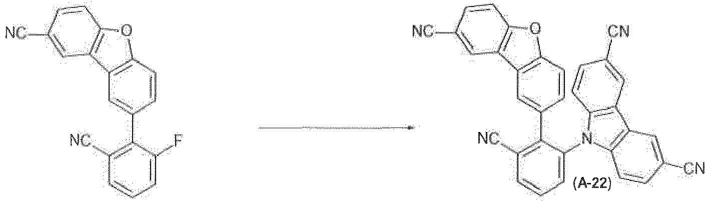

6.0 g (19.2 mmol) 8-(2-cyano-6-fluoro-phenyl)dibenzofuran-2-carbonitrile, 4.38 g (20.2 mmol) 9H-carbazole-3,6-dicarbonitrile and 24.5 g (115.3 mmol) K₃PO₄ are added to 200 ml DMF and the suspension is heated to reflux for 40 h, filtered hot through Hyflo and the solvent is evaporated on the rotavap. To the residue are added 150 ml MeOH, the mixture is heated to reflux and then cooled to RT. The suspension is filtered; the residue is washed twice with MeOH (10 ml each) and dried at 80°C / 125 mbar overnight to yield 8.3 g crude product. The solid is heated to reflux with 150ml dioxane, filtered hot through Hyflo and the solvent is evaporated on the rotavap. The product is decocted twice with 1-methoxy-2-propanol (50 ml each), filtered hot and dried at 80°C / 125 mbar overnight to yield 4.3 g 9-[3-cyano-2-(8-cyanodibenzofuran-2-yl)phenyl]carbazole-3,6-dicarbonitrile as a white solid.
¹H-NMR (400 MHz, CDCl₃): δ 8.31 (s, 2H), 8.13 (dxd, J₁ = 7.2 Hz, J₂ = 1.6 Hz, 1H), 8.03 (d, J = 1.2 Hz, 1H), 7.87 - 7.79 (m, 3H), 7.73 (dxd, J₁ = 8.0 Hz, J₂ = 1.6 Hz, 1H), 7.66 (br d, J = 8.0 Hz, 2H), 7.58 (d, J = 8.4 Hz, 1H), 7.24 (d, J = 8.8 Hz, 1H), 7.19 (br d, J = 8.4 Hz, 2H), 7.04 (dxd, J₁ = 8.8 Hz, J₂ = 2.0 Hz, 1H).

### Example 9

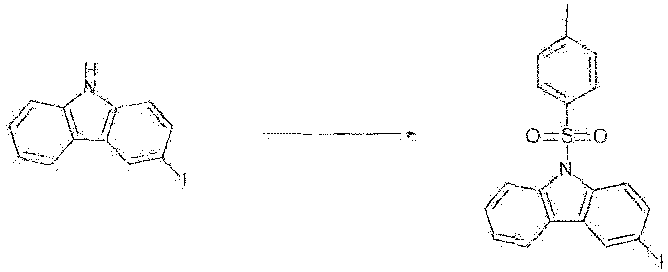

a) 100 g (0.341 mol) 3-iodo-9H-carbazole are added to 1.6 I toluene, then 80.4 g (0.409 mol) 4-methylbenzenesulfonyl chloride and 8.1 g (0.024 mol) tetrabutylammonium hydrogen sulfate are added. To the stirred suspension 460 g 50% aqueous KOH solution are slowly added within 50 minutes while keeping the temperature below 25°C with a water bath. After stirring for additional 2 hours the reaction mixture is diluted with 250 ml H₂O, the phases are separated and the organic phase is washed four times with H₂O (250 ml each), dried over MgSO₄, filtered and evaporated to yield 148.1 g of a white solid. The crude product is suspended in 300 ml of 2-propanol, heated to reflux for 2 hours, cooled to RT filtered, washed three times with 2-propanol (50 ml each) and dried at 80°C / 125 mbar overnight to yield 138.2 g (90.6% of theory) 3-iodo-9-(p-tolylsulfonyl) carbazole as white solid.
   ¹H-NMR (400 MHz, CDCl₃): δ 8.31 (d, J = 8.4 Hz, 1H), 8.20 (d, J = 1.6 Hz, 1H), 8.10 (d, J = 8.4 Hz, 1H), 7.82 (d, J = 8.0 Hz, 1H), 7.75 (dxd, J₁ = 8.8 Hz, J₂ = 2.0 Hz, 1H), 7.67 (d, J = 8.4 Hz, 2H), 7.51 (t, J = 7.2 Hz, 1H), 7.36 (t, J = 7.2 Hz, 1H), 7.10 (d, J = 8.4 Hz, 2H), 2.25 (s, 3H). 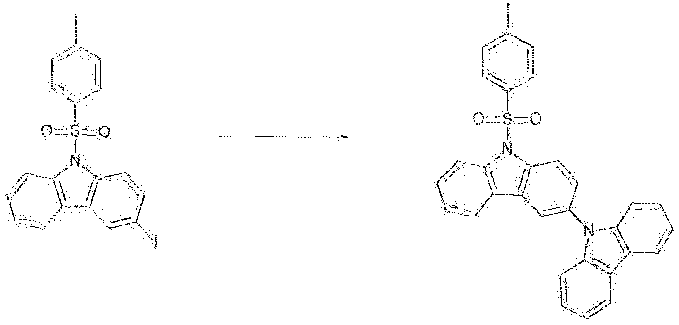
b) 54.8 g (0.137 mol) 3-iodo-9-(p-tolylsulfonyl) carbazole, 27.5 g (0.164 mol) 9H-carbazole, 9.4 g (0.082 mol) trans-1,2-diamino-cyclohexane, 183.2 g (0.863 mol) K₃PO₄ and 14.6 g (0.076 mol) Cul are added to 1 l toluene and the grey suspension is heated to reflux for 48 h. The reaction mixture is filtered hot through Hiflo and washed four times with toluene (100 ml each). To the clear filtrate 100 g of silica are added and the suspension is heated to reflux for 1 hour, filtered hot and washed three times with toluene (100 ml each). The filtrate is evaporated to yield 68.6 g of a brown solid. To the crude product 600 ml of cyclohexane/toluene = 2:1 are added and heated to reflux to give a clear solution. Then 200 ml of cyclohexane are added and the solution is cooled to RT and then to 0°C. The resulting suspension is filtered; the residue is washed three times with cold cyclohexane (75 ml each) and dried at 80°C / 125 mbar overnight to yield 39.0 g (58.5% of theory) 3-carbazol-9-yl-9-(p-tolylsulfonyl)carbazole as white solid.
   ¹H-NMR (400 MHz, CDCl₃): δ 8.55 (d, J = 8.8 Hz, 1H), 8.40 (d, J = 8.4 Hz, 1H), 8.17 (d, J = 7.6 Hz, 2H), 8.09 (d, J = 2.0 Hz, 1H), 7.89 (d, J = 7.6 Hz, 1H), 7.83 (d, J = 8.4 Hz, 2H), 7.67 (dxd, J₁ = 8.4 Hz, J₂ = 2.0 Hz, 1H), 7.56 (t, J = 8.4 Hz, 1H), 7.44 - 7.26 (m, 7H), 7.20 (d, J = 8.0 Hz, 2H), 2.36 (s, 3H). 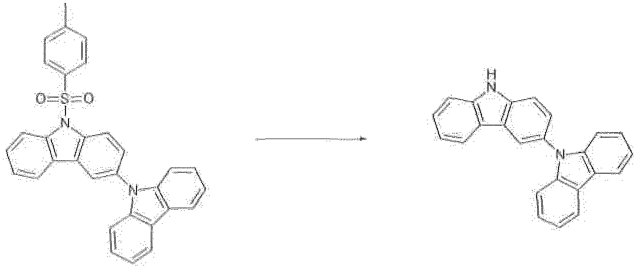
c) 10.6 g (160.3 mmol) KOH 85% are dissolved in 450 ml EtOH, then 39.0 g (80.1 mmol) 3-carbazol-9-yl-9-(p-tolylsulfonyl)carbazole dissolved in 180 ml THF are added and the clear solution is heated to reflux for 6 h. The reaction mixture is cooled to RT and then added drop by drop to 1 l H₂O. The resulting suspension is filtered and the residue is washed four times with H₂O (200 ml each) and then dried at 80°C / 125 mbar overnight to yield 25.6 g of an off white solid. The crude product is suspended in 150 ml MeOH and heated to reflux for 2 h. The suspension is cooled to RT and then to 0°C, filtered, washed twice with cold MeOH (20 ml each) and dried at 60°C / 125 mbar overnight to yield 23.4 g of an off white solid. This procedure is repeated twice to yield 20.76 g (77.9% of theory) 3-Carbazol-9-yl-9H-carbazole as a white solid.
   ¹H-NMR (400 MHz, CDCl₃): δ 8.35 - 8.20 (m, 3H), 8.16 (br s, 1H), 8.08 (d, J = 7.6 Hz, 1H), 7.64 - 7.28 (m, 11H). 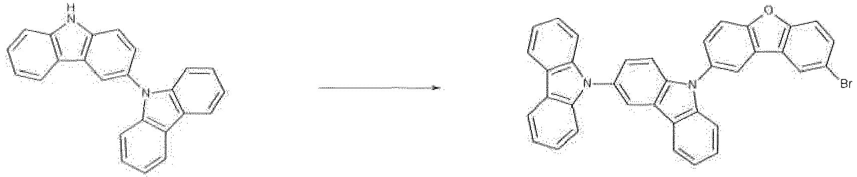
d) 18.6 g (49.7 mmol) 2-bromo-8-iodo-dibenzofuran, 13.8 g (41.5 mmol) 3-carbazol-9-yl-9H-carbazole, 23.7 g (373.1 mmol) Cu and 19.9 g (124.4 mmol) K₂CO₃ are added to 200 ml DMA and the suspension is heated to 140°C for 46 h. The reaction mixture is filtered hot through Hyflo and the filtrate is added to 2 l H₂O. The suspension is filtered and washed three times with H₂O (500 ml each) and dried at 80°C / 125 mbar overnight to yield 29.6 g crude product. CombiFlash chromatography (solvent: heptane / CH₂Cl₂ 0 to 25%) yields 11.6 g (48.5% of theory) of a mixture of 9-(8-bromodibenzofuran-2-yl)-3-carbazol-9-yl-carbazole and 9-(8-iododibenzofuran-2-yl)-3-carbazol-9-yl-carbazole as a white solid.
   ¹H-NMR (400 MHz, CDCl₃): δ 8.33 (s, 1H), 8.21 - 8.12 (m, 5H), 7.85 (d, J = 8.8 Hz, 1H), 7.76 (dxd, J₁ = 8.8 Hz, J₂ = 2.0 Hz, 1H), 7.65 (dxd, J₁ = 8.8 Hz, J₂ = 2.0 Hz, 1H), 7.57 - 7.51 (m, 3H), 7.51 -7.41 (m, 6H), 7.37 -7.29 (m, 3H). 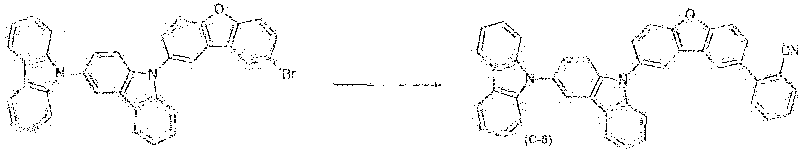
e) 10.53 g (18.3 mmol) 9-(8-bromodibenzofuran-2-yl)-3-carbazol-9-yl-carbazole, 5.54 g (23.7 mmol) 2-(4,4,5,5-tetramethyl-1,3,2-dioxaborolan-2-yl) benzonitrile, 5.0 g 50% NaOH solution (aqueous) are added to 150 ml dioxane and the reaction mixture is evacuated and purged three timest with argon. 0.37 g (1.28 mmol) P(t-Bu)₃ HBF₄ and 0.20 g (0.91 mmol) Pd(OAc)₂ are added and the reaction mixture is evacuated and purged three times with argon. The reaction mixture is heated to reflux for 5 h and cooled to RT. 20 ml H₂O are added and the mixture is extracted three times with EtOAc (100 ml each). The combined organic phases are washed five times with H₂O (100 ml each), dried over Na₂SO₄, filtered and evaporated on the rotavap to yield 14.6 g of a white solid. CombiFlash chromatography (solvent: heptane / CH₂Cl₂ 0 to 70%) yields 8.5 g of a white solid. 100 ml MeOH are added and the suspension is heated to reflux for 2 h, cooled to RT, filtered and dried at 80°C / 125 mbar overnight to yield 6.6 g (60.4 % of theory) 2-[8-(3-carbazol-9-ylcarbazol-9-yl)dibenzofuran-2-yl]benzonitrile as a white solid.
   ¹H-NMR (400 MHz, CDCl₃): δ 8.32 (s, 1H), 8.25 (d, J = 2.0 Hz, 1H), 8.19 (d, J = 7.6 Hz, 2H), 8.17 - 8.14 (m, 2H), 7.89 (d, J = 8.4 Hz, 1H), 7.82 - 7.66 (m, 5H), 7.63 - 7.54 (m, 3H), 7.52 - 7.40 (m, 7H), 7.36 - 7.28 (m, 3H).

### Example 10

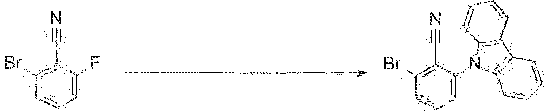

a) 10.0 g (0.050 mol) 2-bromo-6-fluorobenzonitrile, 10.0 g (0.060 mol) 9*H*-carbazole, and 10.4 g (0.075 mol) K₂CO₃ are added to 150 ml of DMSO and the suspension is heated to 60°C for 3 h under Nitrogen. The resulting solution is cooled to room temperature and then poured into water. The suspension is filtered and the residue is washed three times with MeOH. The solid is suspended in 150 ml of MeOH, which is stirred at 60°C for 30 minutes. The suspension is cooled to room temperature, filtered, and dried to yield 15.9 g (91.5% of theory) 2-bromo-6-(carbazol-9-yl)benzonitrile as a light yellow solid.
   ¹H-NMR (400 MHz, CDCl₃): δ 8.14 (d, J = 7.2 Hz, 2H), 7.84 (d, J = 8.0 Hz, 1H), 7.66 (t, J = 8.4 Hz, 1H), 7.58 (d, J = 8.0 Hz,1H), 7.44 (t, J = 8.4 Hz, 2H), 7.34 (t, J = 7.2 Hz, 2H), 7.21 (d, J = 8.0 Hz, 2H). 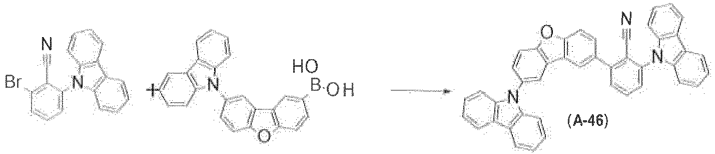
b) 5.00 g (0.014 mol) 2-bromo-6-(carbazol-9-yl)benzonitrile, 6.52 g (0.017 mol) 8-(carbazol-9-yl)dibenzofuran-2-boronic acid, 0.832 g (0.720 mmol) Pd(PPh₃)₄, and 14.4ml 2M Na₂CO₃ aq. are added to 30 ml of toluene and the suspension is heated to 60°C for 8 h under Nitrogen. The resulting solution is cooled to room temperature. And then, dichloromethane and water are added and the organic phase is separated. The organic phase is washed with water and dried with magnesium sulfate and evaporated under reduced pressure to give a crude material as a black solid. The crude material is purified by chromatography on NH-silica gel using Hexane/CH₂Cl₂ (9/1-1/1) as eluent and then is recrystallized from toluene to yield 6.27 g (73% of theory) 2-(carbazol-9-yl)-6-[8-(carbazol-9-yl)dibenzofuranyl-2-yl]benzonitrile as a white solid. Product confirmed by LCMS m/z = 600 (M+1).
   ¹H-NMR (400 MHz, CDCl₃): δ 8.23 - 8.15 (m, 6H), 7.90 - 7.80 (m, 4H), 7.74 (d, J = 7.6 Hz, 1H), 7.73 (dxd, J₁ = 8.8 Hz, J₂ = 2.0 Hz, 1H), 7.62 (d, J = 7.6 Hz, 1H), 7.47 - 7.39 (m, 6H), 7.35 - 7.28 (m, 6H).

### Example 11

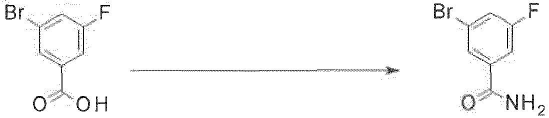

a) 20.0 g (0.091 mol) 3-bromo-5-fluorobenzoic acid in 100ml thionyl chloride is heated at reflux for 2h. The resulting solution is distilled. To the residue is added 70ml 28% ammonia in water and stirred at room temperature for 3h. The resulting solution is poured into water. The suspension is filtered and the residue is washed three times with H₂O to yield 12.3 g (61.8% of theory) 3-bromo-5-fluorobenzamide as a light yellow solid.
   ¹H-NMR (400 MHz, CDCl₃): δ 7.73 (s, 1H), 7.49 - 7.41 (m, 2H), 6.05 (br s, 2H). 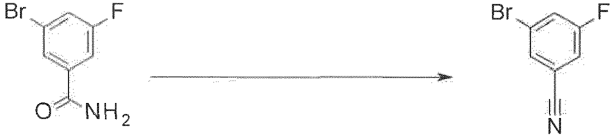
b) 12.3 g (0.056 mol) 3-bromo-5-fluorobenzamide in 75ml thionyl chloride is heated at reflux for 32h. The resulting solution is distilled. The residue is dissolved in 100ml ethyl acetate, which is poured into 250ml sat. NaHCO₃ aq. and stirred at room temperature for 3h. The resulting suspension is filtered and the filtrate is washed with brine and extracted with ethyl acetate. The organic phase is then dried over MgSO₄ and evaporated to give a crude material as an orange oil. The crude material is purified by chromatography on silica gel using Hexane/Ethyl acetate (2/1-1/1) to yield 7.60 g (67.4% of theory) 3-bromo-5-fluorobenzonitrile as a light yellow solid. 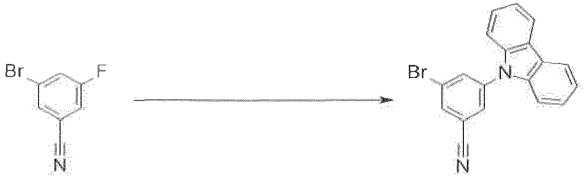
c) 5.50 g (0.027 mol) 3-bromo-5-fluorobenzonitrile, 5.50 g (0.033 mol) 9*H*-carbazole, and 13.4 g (0.041 mol) Cs₂CO₃ are added to 80 ml of DMSO and the suspension is heated to 80°C for 16 h under Nitrogen. The resulting solution is cooled to room temperature and then poured into water. The suspension is filtered and the residue is washed three times with H₂O to give a crude material as a light yellow solid. The crude material is purified by chromatography on silica gel using hexane/ethyl acetate (9/1) to yield 4.57 g (47.9% of theory) 3-bromo-5-(carbazol-9-yl)benzonitrile as a light yellow solid. Product confirmed by LCMS m/z = 347 (M+1). 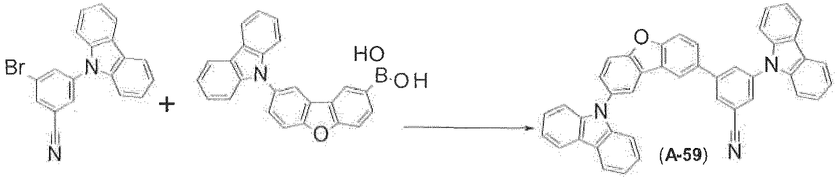
d) 4.50 g (0.013 mol) 3-bromo-5-(carbazol-9-yl)benzonitrile, 5.85 g (0.016 mol) 8-(carbazol-9-yl)dibenzofuran-2-boronic acid, 0.299 g (0.259 mmol) Pd(PPh₃)₄, and 12.9ml 2M Na₂CO₃ aq. are added to 70 ml of toluene and the suspension is heated to 110°C for 3 h under Nitrogen. The resulting solution is cooled to room temperature. And then, toluene and water are added, and the organic phase is separated. The organic phase is washed with water, dried with magnesium sulfate, and evaporated under reduced pressure to give a crude material as a black solid. The crude material is purified by chromatography on NH-silica gel using Hexane/CH₂Cl₂ (4/1-1/1) as eluent and then is recrystallized from xylene to yield 3.59 g (46.4% of theory) 3-(carbazol-9-yl)-5-[8-(carbazol-9-yl)dibenzofuranyl-2-yl]benzonitrile as a white solid. Product confirmed by LCMS m/z = 600 (M+1).
   ¹H-NMR (400 MHz, CDCl₃): δ 8.19 - 8.13 (m, 7H), 8.03 (s, 1H), 7.88 (s, 1H), 7.83 (d, J = 8.4 Hz, 1H), 7.78 (s, 2H), 7.68 (dxd , J₁ = 8.4 Hz, J₂ = 2.4 Hz, 1H), 7.46 - 7.29 (m, 12H).

### Comparative Application Example 1

A glass substrate with 120 nm-thick indium-tin-oxide (ITO) transparent electrode used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 A/sec at about 10⁻⁶ -10⁻⁸ mbar. As a hole injection layer, compound 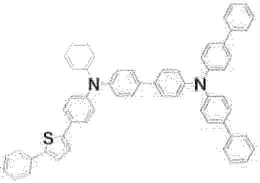 with 30 nm thickness is applied. Then compound 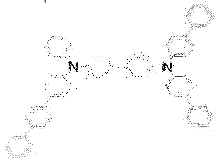 with 60 nm thickness is applied as a hole transporting layer. As an exciton and electron blocker, compound 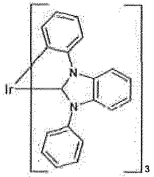 (HTM-1; for preparation, see Ir complex (7) in the application WO2005/019373) is then applied with a thickness of 10 nm. Subsequently, a mixture of 20% by weight of emitter compound, 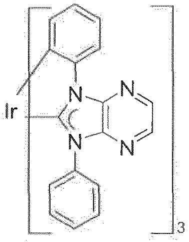 (BE-1), 15% by weight of compound (HTM-1) and 65% by weight of host 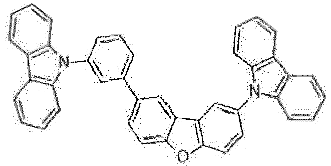 (SH-1) are applied to form a 40 nm-thick emitting layer. On the emitting layer, 5 nm-thick material (SH-1) is applied as an exciton blocker. Thereafter, compound 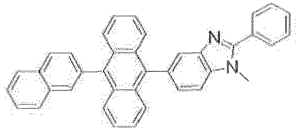 with 20 nm thickness is deposited as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U and EQE are given at luminance (L) = 1000 cd/m² except otherwise stated.

### Application Example 1

Comparative Application Example 1 is repeated except that the exciton blocker (**SH-1**) is replaced by compound 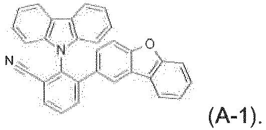 The device results are shown in Table 1.

### Application Example 2

Comparative Application Example 1 is repeated except that the exciton blocker (**SH-1**) is replaced by compound 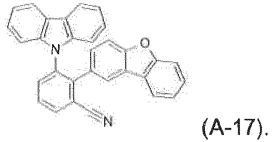 The device results are shown in Table 1.

### Application Example 3

Comparative Application Example 1 is repeated except that the exciton blocker (**SH-1**) is replaced by compound 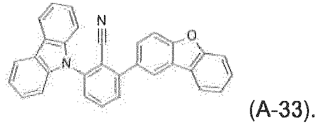 The device results are shown in Table 1.

### Application Example 4

Comparative Application Example 1 is repeated except that the exciton blocker (**SH-1**) is replaced by compound 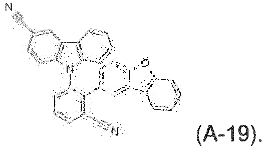 The device results are shown in Table 1.

### Application Example 5

Comparative Application Example 1 is repeated except that the exciton blocker (**SH-1**) is replaced by compound 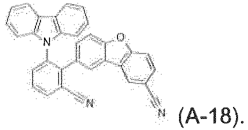 The device results are shown in Table 1.

### Application Example 6

Comparative Application Example 1 is repeated except that the exciton blocker (**SH-1**) is replaced by compound 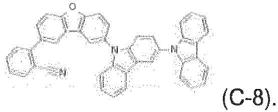 The device results are shown in Table 1.

### Application Example 7

Comparative Application Example 1 is repeated except that the exciton blocker (**SH-1**) is replaced by compound 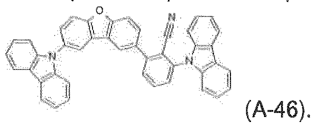 The device results are shown in Table 1.

### Application Example 8

Comparative Application Example 1 is repeated except that the exciton blocker (**SH-1**) is replaced by compound 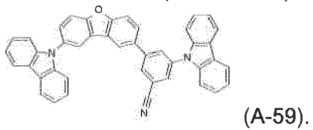 The device results are shown in Table 1.

**Table 1**

| Appl. Ex. | Exciton blocker | U [V] | EQE [%] |
|---|---|---|---|
| Comp. Appl. Ex. 1 | (SH-1) | 5.42 | 17.1 |
| Appl. Ex. 1 | (A-1) | 4.61 | 17.2 |
| Appl. Ex. 2 | (A-17) | 4.60 | 17.4 |
| Appl. Ex. 3 | (A-33) | 4.60 | 17.4 |
| Appl. Ex. 4 | (A-19) | 4.87 | 17.0 |
| Appl. Ex. 5 | (A-18) | 4.83 | 17.0 |
| Appl. Ex. 6 | (C-8) | 4.74 | 17.2 |
| Appl. Ex. 7 | (A-46) | 4.72 | 17.3 |
| Appl. Ex. 8 | (A-59) | 4.63 | 17.4 |

The results shown in Table 1 demonstrate that the driving voltage U is reduced with keeping EQE high enough when compounds (**A-1**), (**A-17**), (**A-33**), (**A-19**), (**A-18**), (**C-8**), (**A-46**) and (**A-59**) are used as exciton blocker instead of the reference compound (**SH-1**).

### Application Example 9

Comparative Application Example 1 is repeated except that both the host (**SH-1**) and exciton blocker (**SH-1**) are replaced by compound 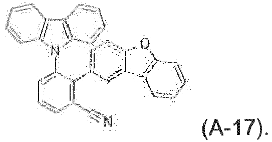 The device results are shown in Table 2.

### Application Example 10

Comparative Application Example 1 is repeated except that both the host (**SH-1**) and exciton blocker (**SH-1**) are replaced by compound 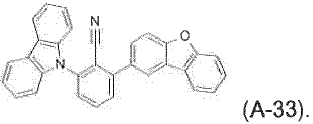 The device results are shown in Table 2.

### Application Example 11

Comparative Application Example 1 is repeated except that both the host (**SH-1**) and exciton blocker (**SH-1**) are replaced by compound 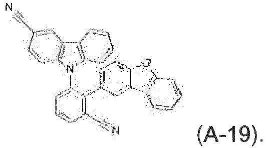 The device results are shown in Table 2.

**Table 2**

| Appl. Ex. | Host | Exciton blocker | U [V] | EQE [%] |
|---|---|---|---|---|
| Comp. Appl. Ex. 1 | (SH-1) | (SH-1) | 5.42 | 17.1 |
| Appl. Ex. 2 | (SH-1) | (A-17) | 4.60 | 17.4 |
| Appl. Ex. 9 | (A-17) | (A-17) | 4.26 | 18.8 |
| Appl. Ex. 3 | (SH-1) | (A-33) | 4.60 | 17.4 |
| Appl. Ex. 10 | (A-33) | (A-33) | 4.12 | 18.5 |
| Appl. Ex. 4 | (SH-1) | (A-19) | 4.87 | 17.0 |
| Appl. Ex. 11 | (A-19) | (A-19) | 4.14 | 17.5 |

The results shown in Table 2 demonstrate that the driving voltage U is further reduced and the EQE is improved at the same time when compounds (**A-17**), (**A-33**) and (**A-19**) are used as both the host and the exciton blocker instead of the reference compound (SH-1).

### Comparative Application Example 2

A glass substrate with 120 nm-thick ITO is cleaned and treated in the same manner as comparative application example 1. As a hole injection layer, compound 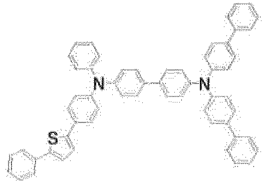 with 30 nm thickness is applied. Then 60 nm of compound (**SH-1**) doped with MoOx (∼10%) is deposited as hole transporting layer. MoOx is used to improve the hole conductivity of **SH-1.** As an exciton and electron blocker, compound (**SH-1**) is applied with a thickness of 10 nm. Subsequently, a mixture of 20% by weight of emitter compound (**BE-1**) and 80% by weight of host (**SH-1**) are applied to form a 40 nm of emitting layer. On the emitting layer, 5 nm of material (**SH-1**) is applied as an exciton blocker.

Thereafter, compound 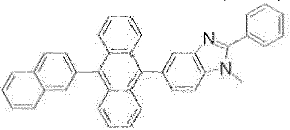 with 20 nm thickness is deposited as an electron transport layer. Finally, 1 nm of LiF is deposited as an electron injection layer and 80 nm of Al is then deposited as a cathode to complete the device. The device is sealed with a glass lid and a getter in an inert nitrogen atmosphere with less than 1 ppm of water and oxygen.

### Application Example 12

Comparative Application Example 2 is repeated except that both the host (**SH-1**) and the exciton blocker (**SH-1**) are replaced by compound 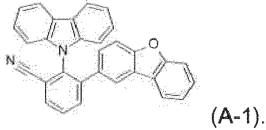 The device results are shown in Table 3.

### Application Example 13

Comparative Application Example 2 is repeated except that both the host (**SH-1**) and the exciton blocker (**SH-1**) are replaced by compound 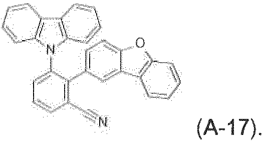 The device results are shown in Table 3.

### Application Example 14

Comparative Application Example 2 is repeated except that both the host (**SH-1**) and the exciton blocker (**SH-1**) are replaced by compound 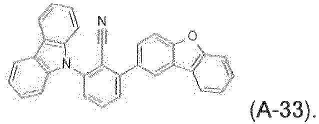 The device results are shown in Table 3.

### Application Example 15

Comparative Application Example 2 is repeated except that both the host (**SH-1**) and the exciton blocker (**SH-1**) are replaced by compound 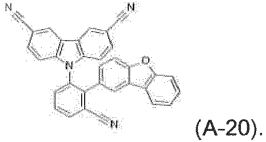 The device results are shown in Table 3.

### Application Example 16

Comparative Application Example 2 is repeated except that both the host (**SH-1**) and the exciton blocker (**SH-1**) are replaced by compound 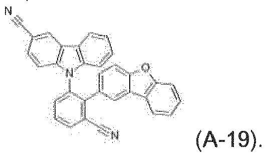 The device results are shown in Table 3.

### Application Example 17

Comparative Application Example 2 is repeated except that both the host (**SH-1**) and the exciton blocker (**SH-1**) are replaced by compound 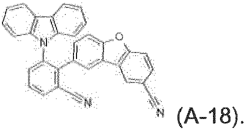 The device results are shown in Table 3.

### Application Example 18

Comparative Application Example 2 is repeated except that both the host (**SH-1**) and the exciton blocker (**SH-1**) are replaced by compound 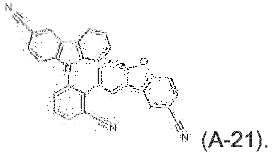 The device results are shown in Table 3.

### Application Example 19

Comparative Application Example 2 is repeated except that both the host (SH-1) and the exciton blocker (**SH-1**) are replaced by compound 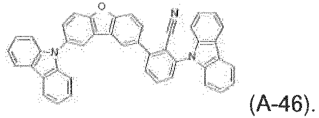 The device results are shown in Table 3.

### Application Example 20

Comparative Application Example 2 is repeated except that both the host (**SH-1**) and the exciton blocker (**SH-1**) are replaced by compound 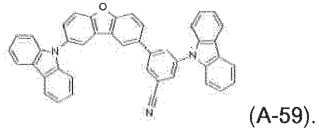 The device results are shown in Table 3.

**Table 3**

| Appl. Ex. | Host | Exciton blocker | U [V] | EQE [%] |
|---|---|---|---|---|
| Comp. Appl. Ex. 2 | (SH-1) | (SH-1) | 5.54 | 2.0 |
| Appl. Ex. 12 | (A-1) | (A-1) | 4.32 | 19.7 |
| Appl. Ex. 13 | (A-17) | (A-17) | 4.02 | 20.0 |
| Appl. Ex. 14 | (A-33) | (A-33) | 4.12 | 19.6 |
| Appl. Ex. 15 | (A-20) | (A-20) | 3.70 | 17.1 |
| Appl. Ex. 16 | (A-19) | (A-19) | 3.95 | 18.6 |
| Appl. Ex. 17 | (A-18) | (A-18) | 3.72 | 18.4 |
| Appl. Ex. 18 | (A-21) | (A-21) | 3.70 | 16.8 |
| Appl. Ex. 19 | (A-40) | (A-40) | 4.30 | 16.6 |
| Appl. Ex. 20 | (A-59) | (A-59) | 3.36 | 17.8 |

The results shown in Table 3 demonstrate that the driving voltage and EQE are improved when compounds (**A-1**), (**A-14**), (**A-33**), (**A-20**), (**A-19**), (**A-18**), (**A-21**), (**A-40**) and (**A-59**) are used as both the host and the exciton blocker instead of reference compound (**SH-1**).

## Claims

1. A compound of the formula 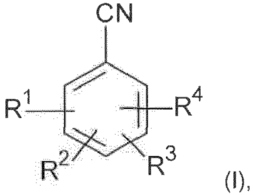 wherein
R¹ is a group of formula 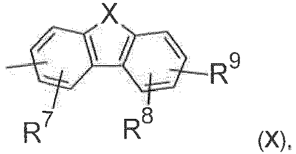 or 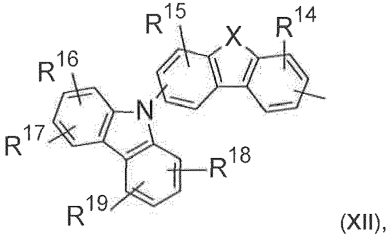
R², R³ and R⁴ are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², - SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G; a group of formula (**X**), (**XI**), (**XII**), or (**XIII**);
X is O, or S,
R⁷ to R⁹ and R¹⁴ to R¹⁹ are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², - SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G;
R²⁴, R²⁵, R²⁶, R²⁷ and R²⁸ are independently of each other a C₁-C₂₅alkyl group, a C₆-C₂₄aryl group, which can optionally be substituted by G, or a C₂-C₃₀heteroaryl group, which can optionally be substituted by G;
R³² and R³³ are independently of each other a C₆-C₁₈aryl group; a C₆-C₁₈aryl group, which is substituted by C₁-C₁₈alkyl group, or C₁-C₁₈alkoxy group; a C₁-C₁₈alkyl group; or a C₁-C₁₈alkyl group, which is interrupted by -O-;
R³⁴ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by - O-;
R³⁵ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-; D is -CO-, -COO-, -S-, -SO-, -SO₂-, -O-, -NR⁶⁵-, -SiR⁷⁰R⁷¹, -POR⁷²-, -CR⁶³=CR⁶⁴-, or -C≡C- ,
E is -OR⁶⁹, -SR⁶⁹, -NR⁶⁵R⁶⁶, -COR⁶⁸, -COOR⁶⁷, -CONR⁶⁵R⁶⁶, -CN, or NO₂;
G is E, or a C₁-C₁₈alkyl group, a C₃-C₁₈cycloalkyl group, a C₆-C₂₄aryl group, a C₆-C₂₄aryl group, which is substituted by -CN, C₁-C₁₈alkyl, or C₁-C₁₈alkyl which is interrupted by O; a C₂-C₅₀heteroaryl group, or a C₂-C₃₀heteroaryl group, which is substituted by -CN, C₁-C₁₈alkyl, C₁-C₁₈alkyl which is interrupted by O;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring;
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-; R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by - O-;
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-;
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
with the proviso, that R² is a group of formula (XI), (XII), or (XIII), if R¹ is a group of formula (X) 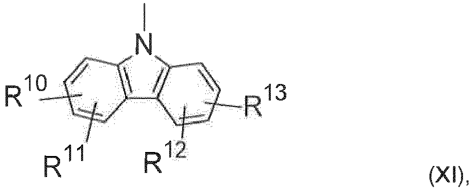 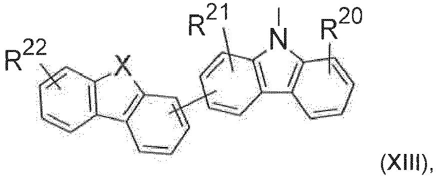
R¹⁰ to R¹³ and R²⁰ to R²³ are independently of each other H, CN, Si(R²⁴)(R²⁵)(R²⁶), P(O)(R²⁷)(R²⁸), N(R³⁰)(R³¹), a C₁-C₂₅alkyl group, which can optionally be substituted by E, and or interrupted by D, a C₂-C₂₅alkenyl group, a C₂-C₂₅alkynyl group, -OR³², - SR³³, -COR³⁴, -COOR³⁵, a C₃-C₁₈cycloalkyl group, which can optionally be substituted by G; a C₆-C₂₄aryl group, which can optionally be substituted by G, or a C₂-C₃₀heteroaryl group, which can optionally be substituted by G.

2. The compound according to claim 1, which is a compound of formula 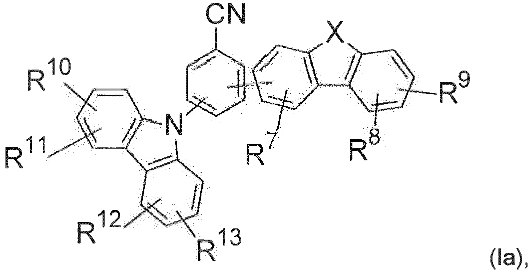 or 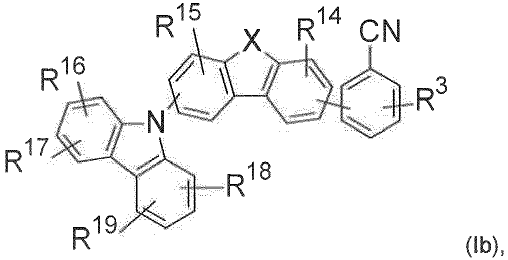 wherein X, R³ and R⁷ to R¹⁹ are as defined in claim 1.

3. The compound according to claim 2, which is a compound of formula 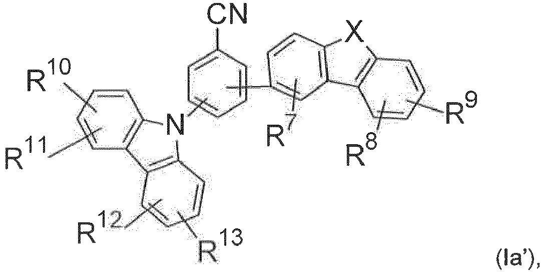 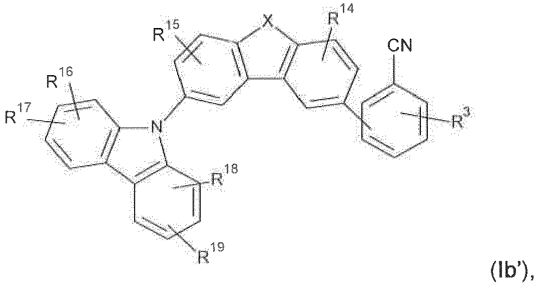 or, wherein X, R³ and R⁷ to R¹⁹ are as defined in claim 1.

4. The compound according to claim 3, which is a compound of formula 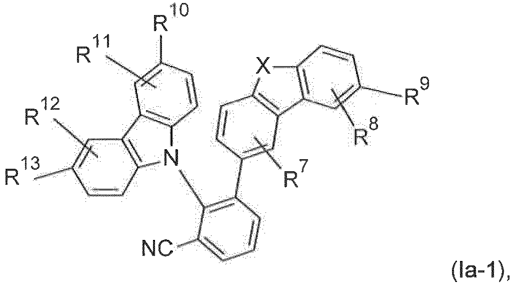 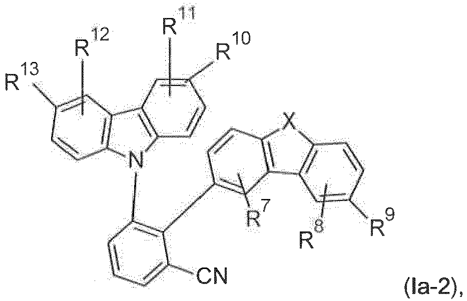 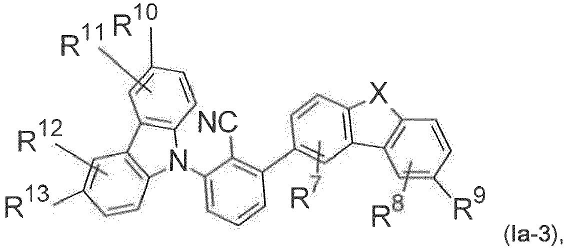 or 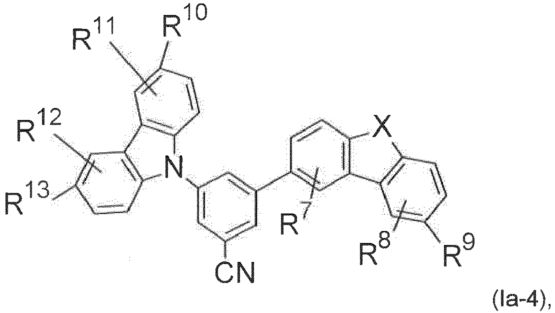 wherein X, R⁷ to R¹³ are as defined in claim 1.

5. The compound according to claim 4, wherein
R⁷, R⁸, R¹¹ and R¹² are independently of each other H, or a C₁-C₂₅alkyl group,
R⁹, R¹⁰ and R¹³ are independently of each other H, CN, a group of formula 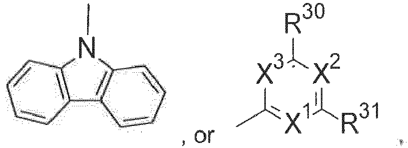
X¹, X² and X³ are independently of each other N, or CH,
R³⁰ and R³¹ are independently of each other H, or a phenyl group, with the proviso that at least one of X¹, X² and X³ is N.

6. The compound according to claim 3, which is a compound of formula 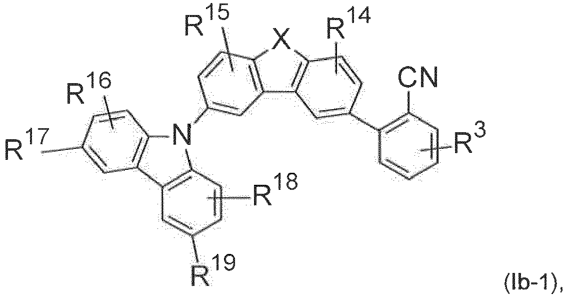 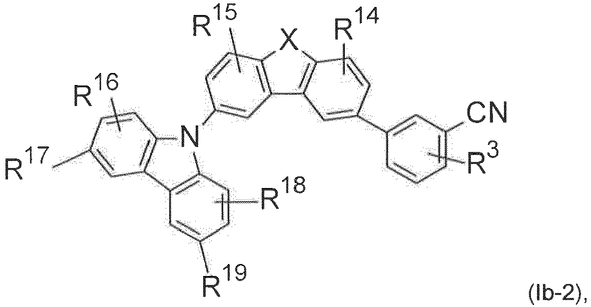 or 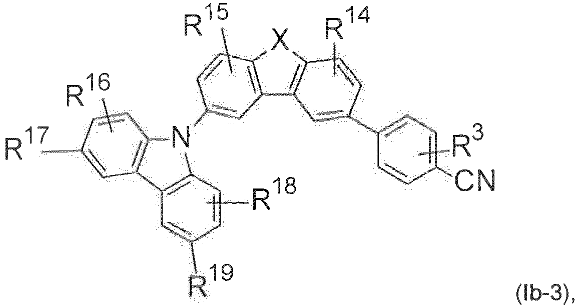 wherein R³ is H, or CN and X, R¹⁴ to R¹⁹ are as defined in claim 1.

7. The compound according to claim 6, wherein
R¹⁴, R¹⁵, R¹⁶ and R¹⁸ are independently of each other H, or a C₁-C₂₅alkyl group,
R¹⁷ and R¹⁹ are independently of each other H, CN, a group of formula 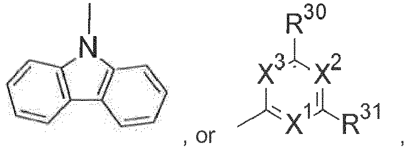
X¹, X² and X³ are independently of each other N, or CH,
R³⁰ and R³¹ are independently of each other H, or a phenyl group, with the proviso that at least one of X¹, X² and X³ is N.

8. The compound according to any of claims 1 to 5, which is a compound of formula 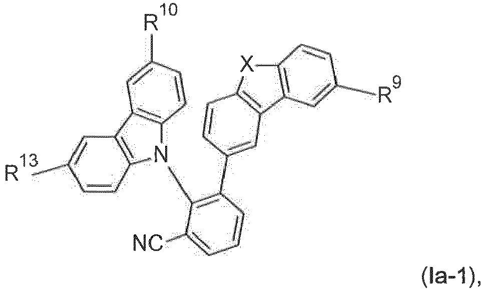 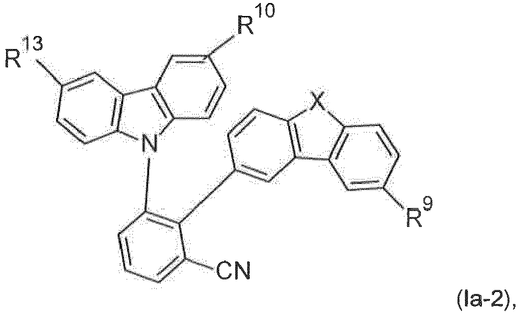 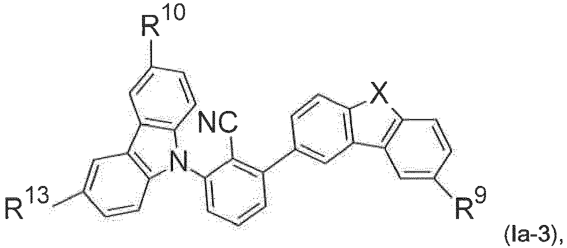 or 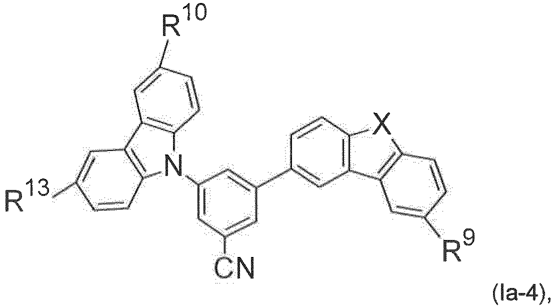 wherein
X is O, or S,
R⁹, R¹⁰ and R¹³ are independently of each other H, CN, a group of formula 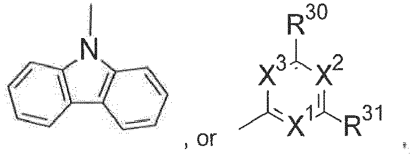
X¹, X² and X³ are independently of each other N, or CH,
R³⁰ and R³¹ are independently of each other H, or a phenyl group, with the proviso that at least one of X¹, X² and X³ is N, or
a compound of formula 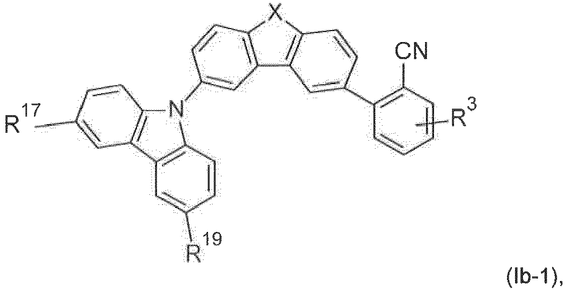 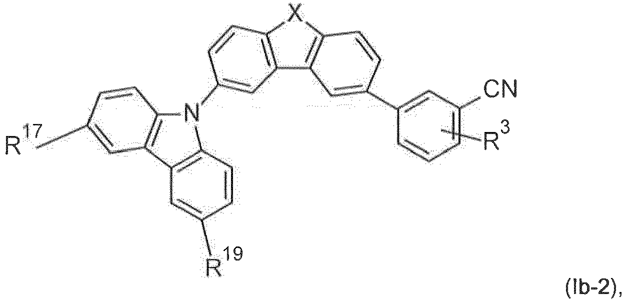 or 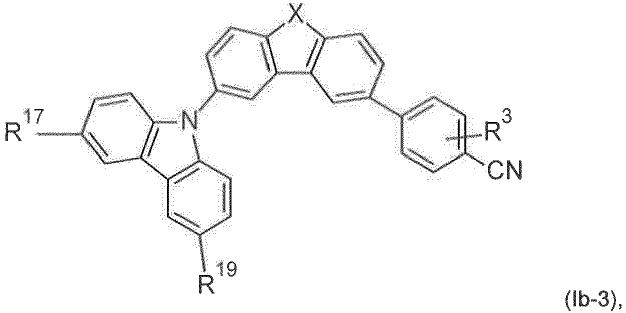 wherein
R³ is H, or CN,
R¹⁷ and R¹⁹ are independently of each other H, CN, a group of formula 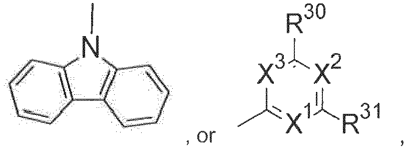
X¹, X² and X³ are independently of each other N, or CH,
R³⁰ and R³¹ are independently of each other H, or a phenyl group, with the proviso that at least one of X¹, X² and X³ is N.

9. The compound according to any of claims 1 to 8, wherein X is O.

10. An electronic device, comprising a compound according to any of claims 1 to 9,

11. The electronic device according to claim 10, which is an electroluminescent device.

12. An electron transport layer, hole blocking layer, or an emitting layer comprising a compound according to any of claims 1 to 9.

13. The emitting layer according to claim 12, comprising a compound according to any of claims 1 to 9 as host material in combination with a phosphorescent emitter.

14. An apparatus selected from the group consisting of stationary visual display units; mobile visual display units; illumination units; keyboards; items of clothing; furniture; wallpaper, comprising the organic electronic device according to claim 10, or 11, or the electron transport layer, hole blocking layer and/or the emitting layer according to claim 12.

15. Use of the compounds of formula I according to any of claims 1 to 9 for electrophotographic photoreceptors, photoelectric converters, organic solar cells, switching elements, organic light emitting field effect transistors, image sensors, dye lasers, electroluminescent devices, as transport material in hole blocking layer and/or emissive layer and/or as electron blocking layer.

## Patentansprüche

1. Verbindung der Formel 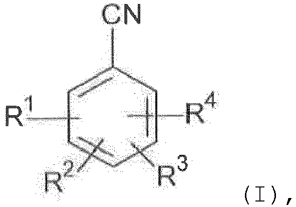 wobei
R¹ für eine Gruppe der Formel 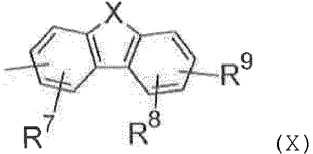 oder 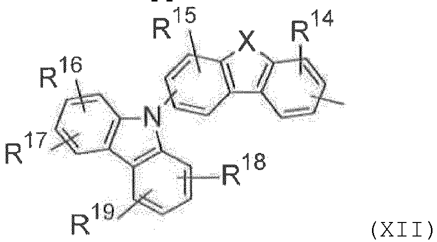 steht,
R², R³ und R⁴ unabhängig voneinander für H, CN, Si(R²⁴)(R²⁵)(R²⁶), P(O)(R²⁷)(R²⁸), N(R³⁰)(R³¹), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴, -COOR³⁵, eine C₃-C₁₈-Cycloalkylgruppe, die gegebenenfalls durch G substituiert sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, eine Gruppe der Formel (X), (XI), (XII) oder (XIII) stehen,
X für O oder S steht,
R⁷ bis R⁹ und R¹⁴ bis R¹⁹ unabhängig voneinander für H, CN, Si(R²⁴)(R²⁵)(R²⁶), P(O)(R²⁷)(R²⁸), N(R³⁰)(R³¹), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², -SR³³, -COR³⁴, -COOR³⁵, eine C₃-C₁₈-Cycloalkylgruppe, die gegebenenfalls durch G substituiert sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
R²⁴, R²⁵, R²⁶, R²⁷ und R²⁸ unabhängig voneinander für eine C₁-C₂₅-Alkylgruppe, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen,
R³² und R³³ unabhängig voneinander für eine C₆-C₁₈-Arylgruppe, eine C₆-C₁₈-Arylgruppe, die durch eine C₁-C₁₈-Alkylgruppe oder C₁-C₁₈-Alkoxygruppe substituiert ist, eine C₁-C₁₈-Alkylgruppe oder eine C₁-C₁₈-Alkylgruppe, die durch -O- unterbrochen ist, stehen,
R³⁴ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht,
R³⁵ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht,
D für -CO-, -COO-, -S-, -SO-, -SO₂-, -O-, -NR⁶⁵-, -SiR⁷⁰R⁷¹-, -POR⁷²-, -CR⁶³=CR⁶⁴- oder -C≡C- steht,
E für -OR⁶⁹, -SR⁶⁹, -NR⁶⁵R⁶⁶, -COR⁶⁸, -COOR⁶⁷, -CONR⁶⁵R⁶⁶, -CN oder NO₂ steht,
G für E oder eine C₁-C₁₈-Alkylgruppe, eine C₃-C₁₈-Cycloalkylgruppe, eine C₆-C₂₄-Arylgruppe, eine C₆-C₂₄-Arylgruppe, die durch -CN, C₁-C₁₈-Alkyl oder C₁-C₁₈-Alkyl, das durch O unterbrochen ist, substituiert ist, eine C₂-C₅₀-Heteroarylgruppe oder eine C₂-C₃₀-Heteroarylgruppe, die durch -CN, C₁-C₁₈-Alkyl oder C₁-C₁₈-Alkyl, das durch O unterbrochen ist, substituiert ist, steht,
R⁶³ und R⁶⁴ unabhängig voneinander für H, C₆-C₁₈-Aryl, ein C₆-C₁₈-Aryl, das durch C₁-C₁₈-Alkyl oder C₁-C₁₈-Alkoxy substituiert ist, C₁-C₁₈-Alkyl oder C₁-C₁₈-Alkyl, das durch -O- unterbrochen ist, stehen, R⁶⁵ und R⁶⁶ unabhängig voneinander für eine C₆-C₁₈-Arylgruppe, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, stehen oder
R⁶⁵ und R⁶⁶ zusammen einen fünf- oder sechsgliedrigen Ring bilden,
R⁶⁷ für eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht,
R⁶⁸ für H, eine C₆-C₁₈-Arylgruppe, eine C₆-C₁₈-Arylgruppe, die durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O-unterbrochen ist, steht,
R⁶⁹ für ein C₆-C₁₈-Aryl, ein C₆-C₁₈-Aryl, das durch C₁-C₁₈-Alkyl oder C₁-C₁₈-Alkoxy substituiert ist, eine C₁-C₁₈-Alkylgruppe oder eine C₁-C₁₈-Alkylgruppe, die durch -O- unterbrochen ist, steht,
R⁷⁰ und R⁷¹ unabhängig voneinander für eine C₁-C₁₈-Alkylgruppe, eine C₆-C₁₈-Arylgruppe oder eine C₆-C₁₈-Arylgruppe, die durch C₁-C₁₈-Alkyl substituiert ist, stehen und
R⁷² für eine C₁-C₁₈-Alkylgruppe, eine C₆-C₁₈-Arylgruppe oder eine C₆-C₁₈-Arylgruppe, die durch C₁-C₁₈-Alkyl substituiert ist, steht,
mit der Maßgabe, dass R² für eine Gruppe der Formel (XI), (XII) oder (XIII) steht, wenn R¹ für eine Gruppe der Formel (X), 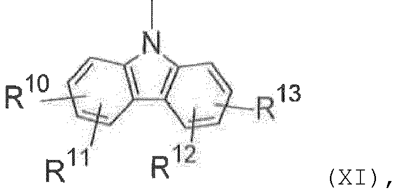 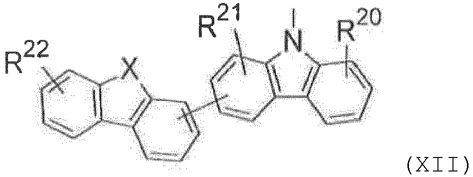 steht,
R¹⁰ bis R¹³ und R²⁰ bis R²³ unabhängig voneinander für H, CN, Si(R²⁴)(R²⁵)(R²⁶) , P(O)(R²⁷)(R²⁸), N(R³⁰)(R³¹), eine C₁-C₂₅-Alkylgruppe, die gegebenenfalls durch E substituiert und/oder durch D unterbrochen sein kann, eine C₂-C₂₅-Alkenylgruppe, eine C₂-C₂₅-Alkinylgruppe, -OR³², - SR³³, -COR³⁴, -COOR³⁵, eine C₃-C₁₈-Cycloalkylgruppe, die gegebenenfalls durch G substituiert sein kann, eine C₆-C₂₄-Arylgruppe, die gegebenenfalls durch G substituiert sein kann, oder eine C₂-C₃₀-Heteroarylgruppe, die gegebenenfalls durch G substituiert sein kann, stehen.

2. Verbindung nach Anspruch 1, bei der es sich um eine Verbindung der Formel 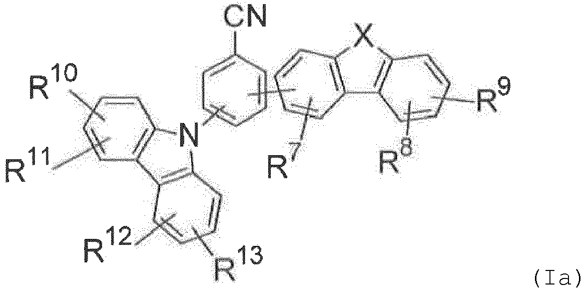 oder 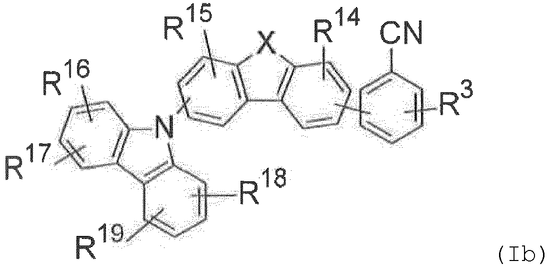 handelt, wobei X, R³ und R⁷ bis R¹⁹ wie in Anspruch 1 definiert sind.

3. Verbindung nach Anspruch 2, bei der es sich um eine Verbindung der Formel 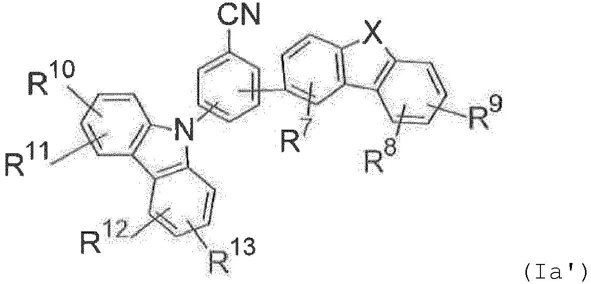 oder 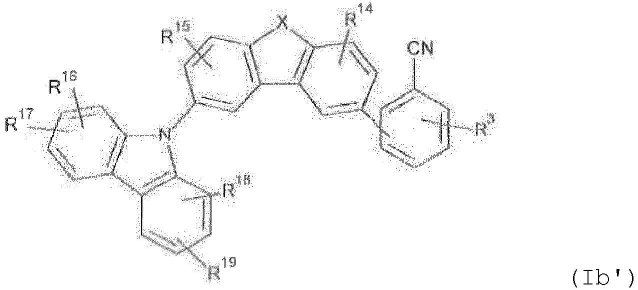 handelt, wobei X, R³ und R⁷ bis R¹⁹ wie in Anspruch 1 definiert sind.

4. Verbindung nach Anspruch 3, bei der es sich um eine Verbindung der Formel 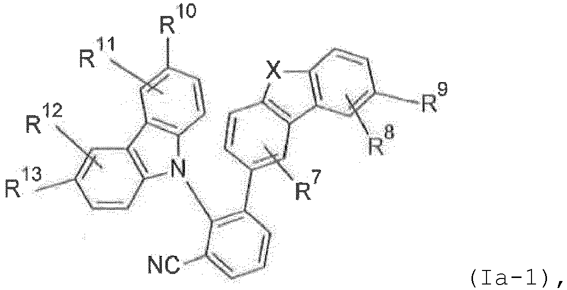 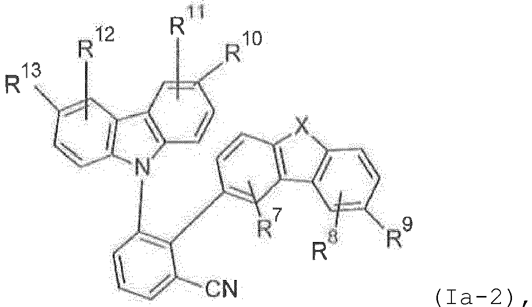 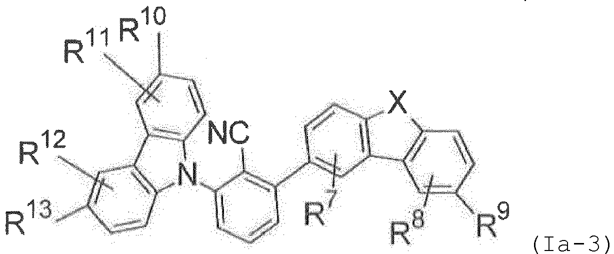 oder 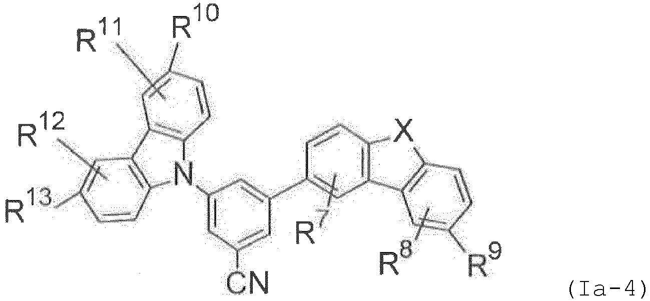 handelt, wobei X, R⁷ bis R¹³ wie in Anspruch 1 definiert sind.

5. Verbindung nach Anspruch 4, wobei
R⁷, R⁸, R¹¹ und R¹² unabhängig voneinander für H oder eine C₁-C₂₅-Alkylgruppe stehen,
R⁹, R¹⁰ und R¹³ unabhängig voneinander für H, CN, eine Gruppe der Formel 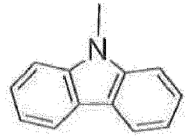 oder 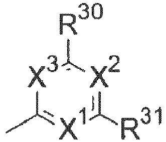 stehen,
X¹, X² und X³ unabhängig voneinander für N oder CH stehen,
R³⁰ und R³¹ unabhängig voneinander für H oder eine Phenylgruppe stehen, mit der Maßgabe, dass mindestens eine der Gruppen X¹, X² und X³ für N steht.

6. Verbindung nach Anspruch 3, bei der es sich um eine Verbindung der Formel 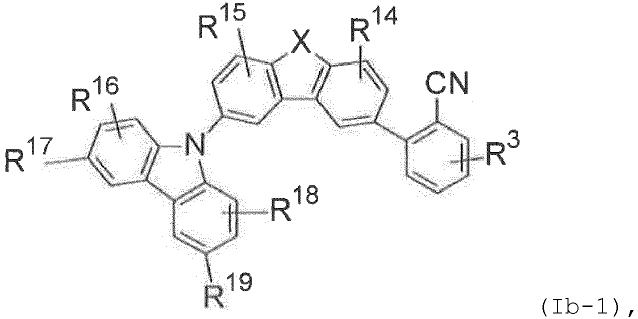 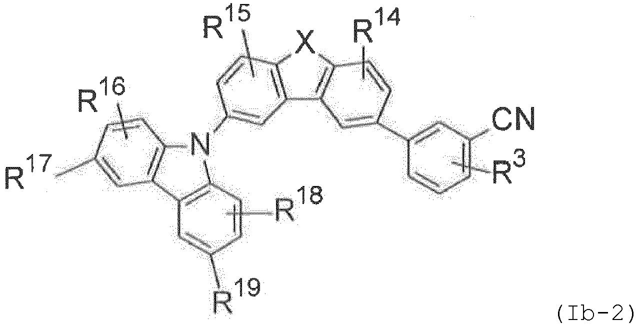 oder 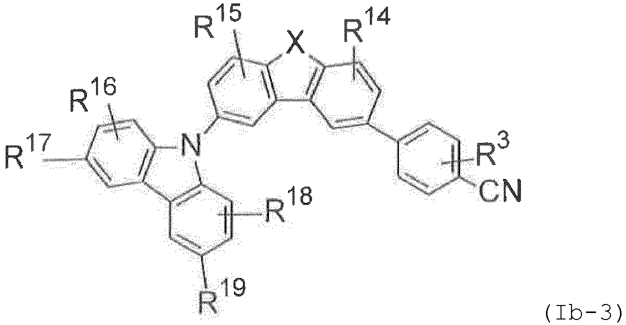 handelt, wobei R³ für H oder CN steht und X, R¹⁴ bis R¹⁹ wie in Anspruch 1 definiert sind.

7. Verbindung nach Anspruch 6, wobei
R¹⁴, R¹⁵, R¹⁶ und R¹⁸ unabhängig voneinander für H oder eine C₁-C₂₅-Alkylgruppe stehen,
R¹⁷ und R¹⁹ unabhängig voneinander für H, CN, eine Gruppe der Formel 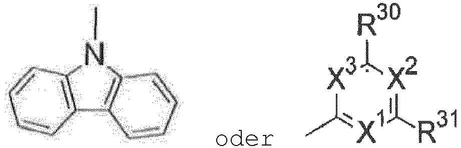 stehen,
X¹, X² und X³ unabhängig voneinander für N oder CH stehen,
R³⁰ und R³¹ unabhängig voneinander für H oder eine Phenylgruppe stehen, mit der Maßgabe, dass mindestens eine der Gruppen X¹, X² und X³ für N steht.

8. Verbindung nach einem der Ansprüche 1 bis 5, bei der es sich um eine Verbindung der Formel 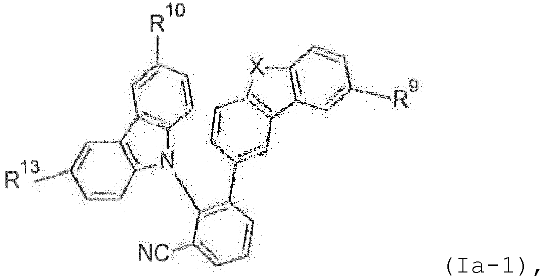 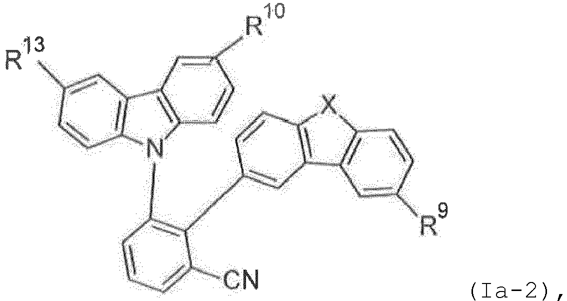 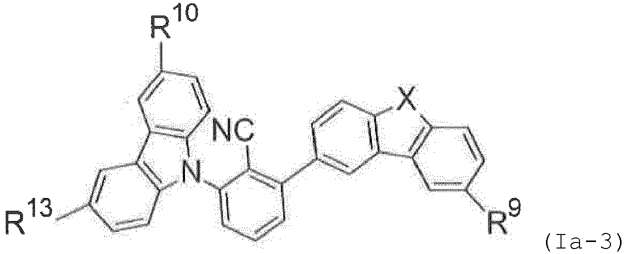 oder 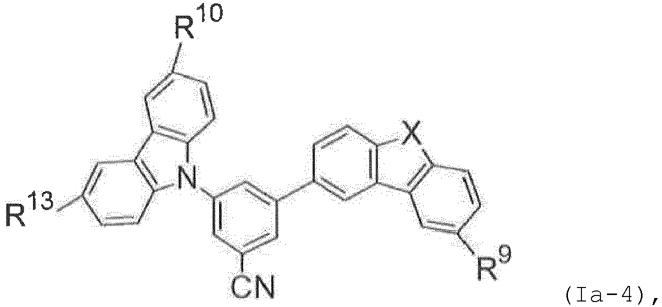 wobei
X für O oder S steht,
R⁹, R¹⁰ und R¹³ unabhängig voneinander für H, CN, eine Gruppe der Formel 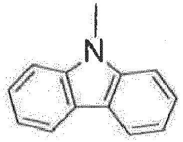 oder 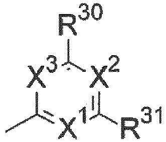 stehen,
X¹, X² und X³ unabhängig voneinander für N oder CH stehen,
R³⁰ und R³¹ unabhängig voneinander für H oder eine Phenylgruppe stehen, mit der Maßgabe, dass mindestens eine der Gruppen X¹, X² und X³ für N steht, oder
eine Verbindung der Formel 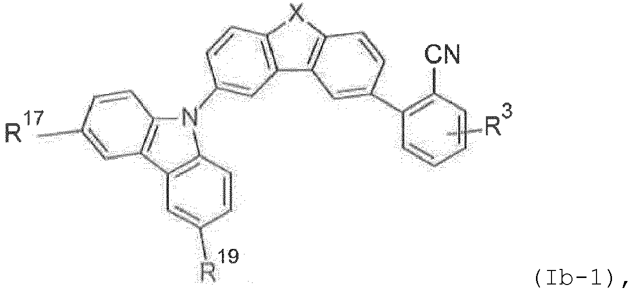 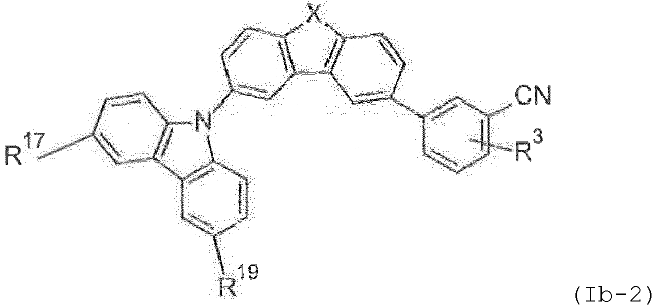 oder 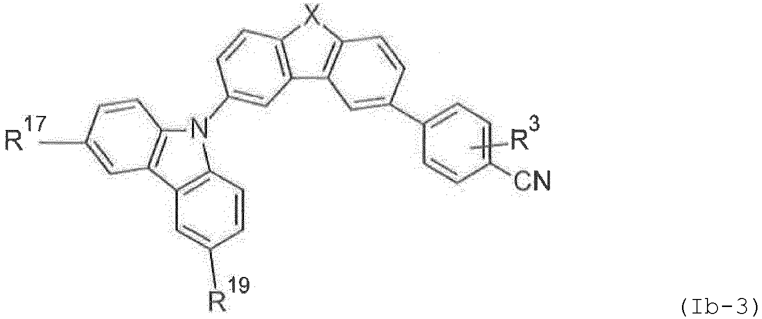
handelt, wobei R³ für H oder CN steht,
R¹⁷ und R¹⁹ unabhängig voneinander für H, CN, eine Gruppe der Formel 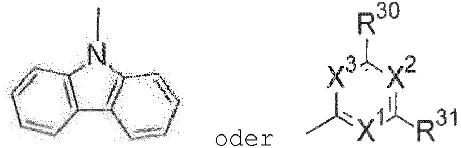 stehen,
X¹, X² und X³ unabhängig voneinander für N oder CH stehen,
R³⁰ und R³¹ unabhängig voneinander für H oder eine Phenylgruppe stehen, mit der Maßgabe, dass mindestens eine der Gruppen X¹, X² und X³ für N steht.

9. Verbindung nach einem der Ansprüche 1 bis 8, wobei X für O steht.

10. Elektronische Vorrichtung, umfassend eine Verbindung nach einem der Ansprüche 1 bis 9.

11. Elektronische Vorrichtung nach Anspruch 10, bei der es sich um eine Elektrolumineszenzvorrichtung handelt.

12. Elektronentransportschicht, Lochblockierungsschicht oder Emissionsschicht, umfassend eine Verbindung nach einem der Ansprüche 1 bis 9.

13. Emissionsschicht nach Anspruch 12, umfassend eine Verbindung nach einem der Ansprüche 1 bis 9 als Wirtsmaterial in Kombination mit einem phosphoreszierenden Emitter.

14. Apparatur, ausgewählt aus der Gruppe bestehend aus stationären Bildschirmen, mobilen Bildschirmen, Beleuchtungseinheiten, Tastaturen, Kleidungsstücken, Möbeln und Tapeten, umfassend die organische elektronische Vorrichtung nach Anspruch 10 oder 11 oder die Elektronentransportschicht, die Lochblockierungsschicht und/oder die Emissionsschicht nach Anspruch 12.

15. Verwendung der Verbindungen der Formel I nach einem der Ansprüche 1 bis 9 für elektrophotographische Photorezeptoren, photoelektrische Umwandler, organische Solarzellen, Schaltelemente, organische lichtemittierende Feldeffekttransistoren, Bildsensoren, Farbstofflaser und Elektrolumineszenzvorrichtungen, als Transportmaterial in einer Lochblockierungsschicht und/oder Emissionsschicht und/oder als Elektronenblockierungsschicht.

## Revendications

1. Composé de la formule 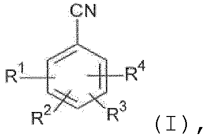 dans laquelle
R¹ représente un groupe de formule 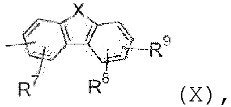 ou 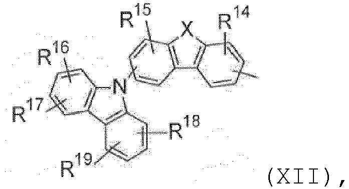
R², R³ et R⁴ représentent indépendamment les uns des autres H, CN, Si(R²⁴)(R²⁵)(R²⁶), P(O)(R²⁷)(R²⁸), N(R³⁰)(R³¹), un groupe alkyle en C₁-C₂₅, qui peut éventuellement être substitué par E, et/ou interrompu par D, un groupe alcényle en C₂-C₂₅, un groupe alcynyle en C₂-C₂₅, -OR³², -SR³³, -COR³⁴, -COOR³⁵, un groupe cycloalkyle en C₃-C₁₈, qui peut éventuellement être substitué par G ; un groupe aryle en C₆-C₂₄, qui peut éventuellement être substitué par G, ou un groupe hétéroaryle en C₂-C₃₀, qui peut éventuellement être substitué par G ; un groupe de formules (X), (XI), (XII), ou (XIII) ;
X représente O ou S,
R⁷ à R⁹ et R¹⁴ à R¹⁹ représentent indépendamment les uns des autres H, CN, Si(R²⁴)(R²⁵)(R²⁶), P(O)(R²⁷)(R²⁸), N(R³⁰)(R³¹), un groupe alkyle en C₁-C₂₅, qui peut éventuellement être substitué par E, et/ou interrompu par D, un groupe alcényle en C₂-C₂₅, un groupe alcynyle en C₂-C₂₅, -OR³², -SR³³, -COR³⁴, -COOR³⁵, un groupe cycloalkyle en C₃-C₁₈, qui peut éventuellement être substitué par G ; un groupe aryle en C₆-C₂₄, qui peut éventuellement être substitué par G, ou un groupe hétéroaryle en C₂-C₃₀, qui peut éventuellement être substitué par G ;
R²⁴, R²⁵, R²⁶, R²⁷ et R²⁸ représentent indépendamment les uns des autres un groupe alkyle en C₁-C₂₅, un groupe aryle en C₆-C₂₄, qui peut éventuellement être substitué par G, ou un groupe hétéroaryle en C₂-C₃₀, qui peut éventuellement être substitué par G ;
R³² et R³³ représentent indépendamment l'un de l'autre un groupe aryle en C₆-C₁₈ ; un groupe aryle en C₆-C₁₈, qui est substitué par un groupe alkyle en C₁-C₁₈, ou un groupe alcoxy en C₁-C₁₈ ; un groupe alkyle en C₁-C₁₈ ; ou un groupe alkyle en C₁-C₁₈, qui est interrompu par -O- ;
R³⁴ représente H ; un groupe aryle en C₆-C₁₈ ; un groupe aryle en C₆-C₁₈, qui est substitué par un alkyle en C₁-C₁₈, ou un alcoxy en C₁-C₁₈ ; un groupe alkyle en C₁-C₁₈ ; ou un groupe alkyle en C₁-C₁₈, qui est interrompu par -O- ;
R³⁵ représente un groupe aryle en C₆-C₁₈ ; un groupe aryle en C₆-C₁₈, qui est substitué par un alkyle en C₁-C₁₈ ou un alcoxy en C₁-C₁₈ ; un groupe alkyle en C₁-C₁₈ ; ou un groupe alkyle en C₁-C₁₈, qui est interrompu par -O- ;
D représente -CO-, -COO-, -S-, -SO-, -SO₂-, -O-, -NR⁶⁵-, -SiR⁷⁰R⁷¹, -POR⁷², -CR⁶³=CR⁶⁴-, ou -C≡C- ;
E représente -OR⁶⁹, -SR⁶⁹, -NR⁶⁵R⁶⁶, -COR⁶⁸, -COOR⁶⁷, -CONR⁶⁵R⁶⁶, -CN, ou NO₂ ;
G représente E, ou un groupe alkyle en C₁-C₁₈, un groupe cycloalkyle en C₃-C₁₈, un groupe aryle en C₆-C₂₄, un groupe aryle en C₆-C₂₄, qui est substitué par -CN, un alkyle en C₁-C₁₈, ou un alkyle en C₁-C₁₈ qui est interrompu par O ; un groupe hétéroaryle en C₂-C₅₀, ou un groupe hétéroaryle en C₂-C₃₀, qui est substitué par -CN, un alkyle en C₁-C₁₈, un alkyle en C₁-C₁₈ qui est interrompu par O ;
R⁶³ et R⁶⁴ représentent indépendamment l'un de l'autre H, un aryle en C₆-C₁₈ ; un aryle en C₆-C₁₈ qui est substitué par un alkyle en C₁-C₁₈, ou un alcoxy en C₁-C₁₈ ; un alkyle en C₁-C₁₈ ; ou un alkyle en C₁-C₁₈ qui est interrompu par -O- ;
R⁶⁵ et R⁶⁶ représentent indépendamment l'un de l'autre un groupe aryle en C₆-C₁₈ ; un aryle en C₆-C₁₈ qui est substitué par un alkyle en C₁-C₁₈, ou un alcoxy en C₁-C₁₈ ; un groupe alkyle en C₁-C₁₈ ; ou un groupe alkyle en C₁-C₁₈, qui est interrompu par -O- ; ou
R⁶⁵ et R⁶⁶ forment ensemble un cycle à cinq ou six chaînons ;
R⁶⁷ représente un groupe aryle en C₆-C₁₈ ; un groupe aryle en C₆-C₁₈, qui est substitué par un alkyle en C₁-C₁₈, ou un alcoxy en C₁-C₁₈ ; un groupe alkyle en C₁-C₁₈ ; ou un groupe alkyle en C₁-C₁₈, qui est interrompu par -O- ;
R⁶⁸ représente H ; un groupe aryle en C₆-C₁₈ ; un groupe aryle en C₆-C₁₈, qui est substitué par un alkyle en C₁-C₁₈, ou un alcoxy en C₁-C₁₈ ; un groupe alkyle en C₁-C₁₈ ; ou un groupe alkyle en C₁-C₁₈, qui est interrompu par -O- ;
R⁶⁹ représente un aryle en C₆-C₁₈ ; un aryle en C₆-C₁₈, qui est substitué par un alkyle en C₁-C₁₈, ou un alcoxy en C₁-C₁₈ ; un groupe alkyle en C₁-C₁₈ ; ou un groupe alkyle en C₁-C₁₈, qui est interrompu par -O- ;
R⁷⁰ et R⁷¹ représentent indépendamment l'un de l'autre un groupe alkyle en C₁-C₁₈, un groupe aryle en C₆-C₁₈, ou un groupe aryle en C₆-C₁₈, qui est substitué par un alkyle en C₁-C₁₈, et
R⁷² représente un groupe alkyle en C₁-C₁₈, un groupe aryle en C₆-C₁₈, ou un groupe aryle en C₆-C₁₈, qui est substitué par un alkyle en C₁-C₁₈ ;
à condition que R² représente un groupe de formule (XI), (XII), ou (XIII), si R¹ représente un groupe de formule (X) 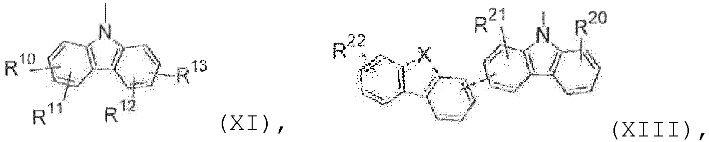
R¹⁰ à R¹³ et R²⁰ à R²³ représentent indépendamment les uns des autres H, CN, Si(R²⁴)(R²⁵)(R²⁶), P(O)(R²⁷)(R²⁸), N(R³⁰)(R³¹), un groupe alkyle en C₁-C₂₅, qui peut éventuellement être substitué par E, et/ou interrompu par D, un groupe alcényle en C₂-C₂₅, un groupe alcynyle en C₂-C₂₅, -OR³², -SR³³, -COR³⁴, -COOR³⁵, un groupe cycloalkyle en C₃-C₁₈, qui peut éventuellement être substitué par G ; un groupe aryle en C₆-C₂₄, qui peut éventuellement être substitué par G, ou un groupe hétéroaryle en C₂-C₃₀, qui peut éventuellement être substitué par G.

2. Composé selon la revendication 1, qui est un composé de formule 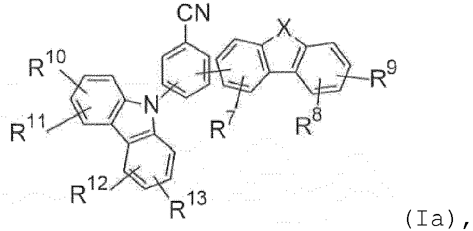 ou 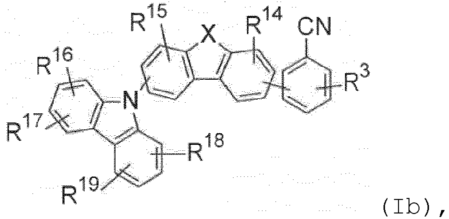 dans lesquelles X, R³ et R⁷ à R¹⁹ sont tels que définis dans la revendication 1.

3. Composé selon la revendication 2, qui est un composé de formule 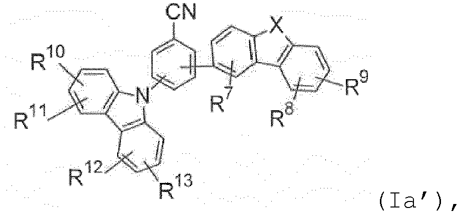 ou 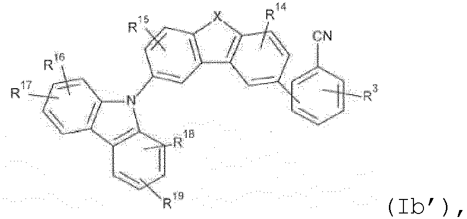 dans lesquelles X, R³ et R⁷ à R¹⁹ sont tels que définis dans la revendication 1.

4. Composé selon la revendication 3, qui est un composé de formule 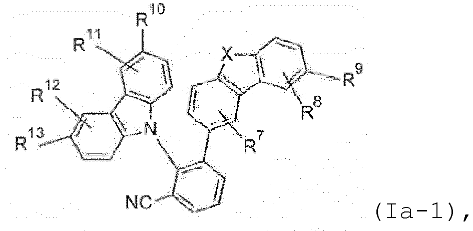 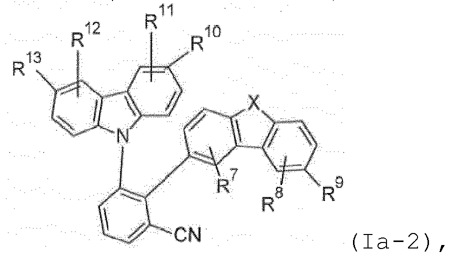 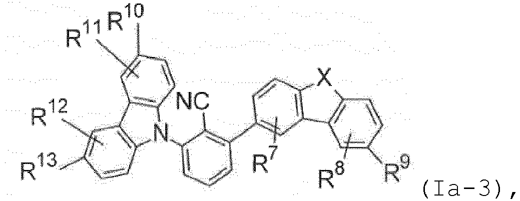 ou 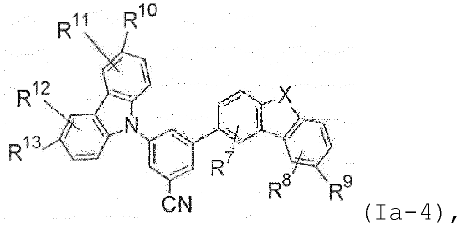 dans lesquelles X, R⁷ à R¹³ sont tels que définis dans la revendication 1.

5. Composé selon la revendication 4, dans lequel
R⁷, R⁸, R¹¹ et R¹² représentent indépendamment les uns des autres H, ou un groupe alkyle en C₁-C₂₅,
R⁹, R¹⁰ et R¹³ représentent indépendamment les uns des autres H, CN, un groupe de formule 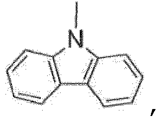 ou 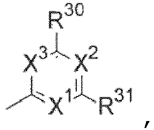
X¹, X² et X³ représentent indépendamment les uns des autres N, ou CH,
R³⁰ et R³¹ représentent indépendamment l'un de l'autre H, ou un groupe phényle, à condition qu'au moins un de X¹, X² et X³ représente N.

6. Composé selon la revendication 3, qui est un composé de formule 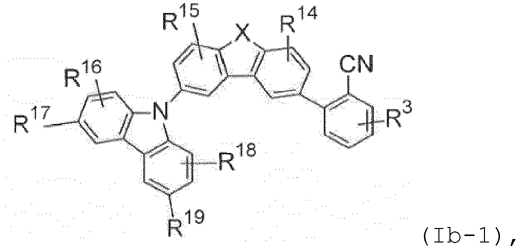 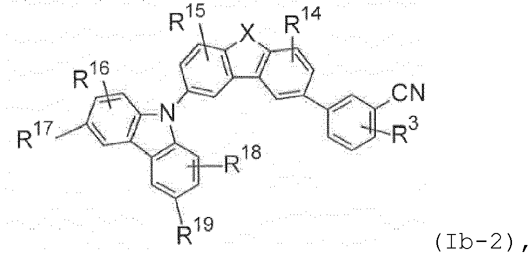 ou 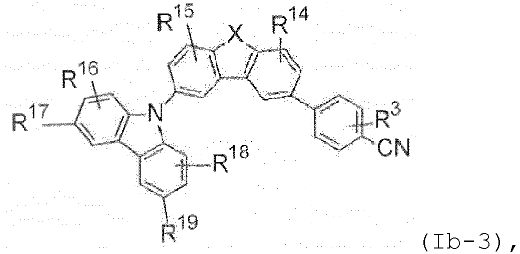 dans lesquelles R³ représente H, ou CN et X, R¹⁴ à R¹⁹ sont tels que définis dans la revendication 1.

7. Composé selon la revendication 6, dans lequel
R¹⁴, R¹⁵, R¹⁶ et R¹⁸ représentent indépendamment les uns des autres H, ou un groupe alkyle en C₁-C₂₅,
R¹⁷ et R¹⁹ représentent indépendamment l'un de l'autre H, CN, un groupe de formule 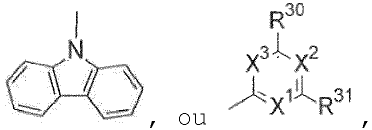
X¹, X² et X³ représentent indépendamment les uns des autres N, ou CH,
R³⁰ et R³¹ représentent indépendamment l'un de l'autre H, ou un groupe phényle, à condition qu'au moins un de X¹, X² et X³ représente N.

8. Composé de l'une quelconque des revendications 1 à 5, qui est un composé de formule 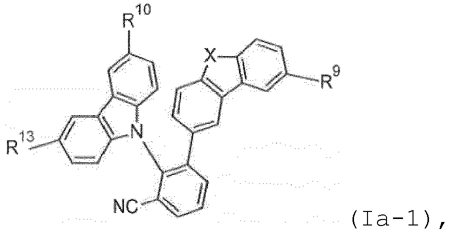 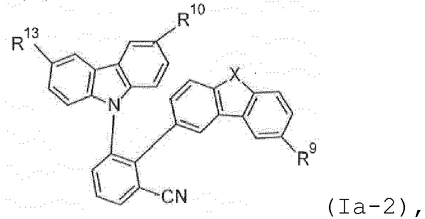 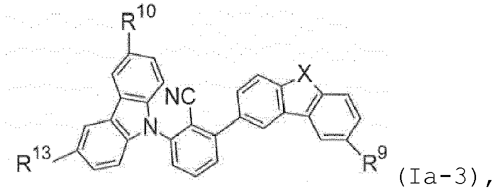 ou 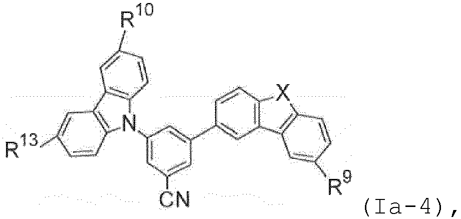 dans lesquelles
X représente O, ou S,
R⁹, R¹⁰ et R¹³ représentent indépendamment les uns des autres H, CN, un groupe de formule 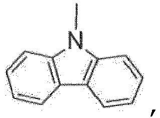 ou 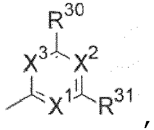
X¹, X² et X³ représentent indépendamment les uns des autres N, ou CH,
R³⁰ et R³¹ représentent indépendamment l'un de l'autre H, ou un groupe phényle, à condition qu'au moins un de X¹, X² et X³ représente N, ou
un composé de formule 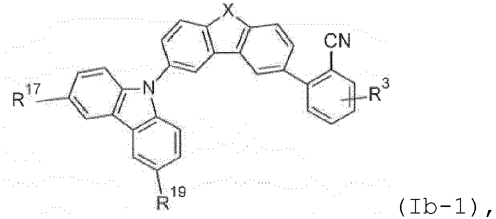 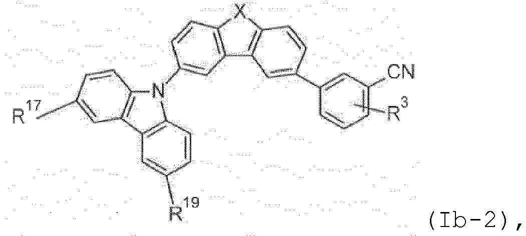 ou 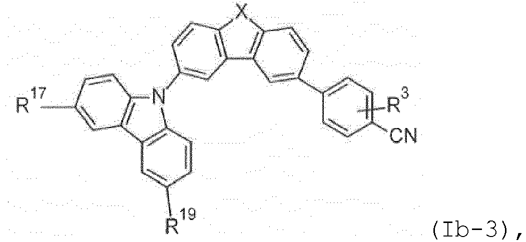 dans lesquelles
R³ représente H, ou CN,
R¹⁷ et R¹⁹ représentent indépendamment l'un de l'autre H, CN, un groupe de formule 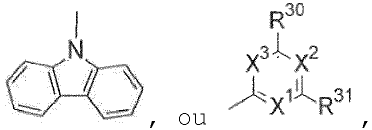
X¹, X² et X³ représentent indépendamment les uns des autres N, ou CH,
R³⁰ et R³¹ représentent indépendamment l'un de l'autre H, ou un groupe phényle, à condition qu'au moins un de X¹, X² et X³ représente N.

9. Composé de l'une quelconque des revendications 1 à 8, dans lequel X représente O.

10. Dispositif électronique, comprenant un composé selon l'une quelconque des revendications 1 à 9.

11. Dispositif électronique selon la revendication 10, qui est un dispositif électroluminescent.

12. Couche de transport d'électrons, couche de blocage de trous, ou couche d'émission comprenant un composé selon l'une quelconque des revendications 1 à 9.

13. Couche d'émission selon la revendication 12, comprenant un composé selon l'une quelconque des revendications 1 à 9 comme matériau hôte en combinaison avec un émetteur phosphorescent.

14. Appareil choisi dans le groupe constitué par les unités d'affichage visuel stationnaires ; les unités d'affichage visuel mobiles ; les unités d'éclairage ; les claviers ; les articles vestimentaires ; les meubles ; le papier peint, comprenant le dispositif électronique organique selon la revendication 10, ou 11, ou la couche de transport d'électrons, la couche de blocage de trous et/ou la couche d'émission selon la revendication 12.

15. Utilisation des composés de formule I selon l'une quelconque des revendications 1 à 9 pour des photorécepteurs électrophotographiques, des convertisseurs photoélectriques, des cellules solaires organiques, des éléments de commutation, des transistors à effet de champ organiques émettant de la lumière, des capteurs d'image, des lasers à colorant, des dispositifs électroluminescents, comme matériau de transport dans une couche de blocage de trous et/ou une couche d'émission et/ou comme couche de blocage d'électrons.
